# EUROPEAN PATENT APPLICATION

(11) **EP 4 744 582 A1**
(43) Date of publication of application: **20.05.2026**
(21) Application number: 24884811.1
(22) Date of filing: 30.10.2024
(51) Int. Cl.: A61B 5/021

(54) **BLOOD PRESSURE MEASUREMENT METHOD, WEARABLE DEVICE, AND STORAGE MEDIUM**

(30) Priority: 31.10.2023 CN 202311440599
(71) Applicant: Huawei Technologies Co., Ltd., Shenzhen, Guangdong 518129 (CN)
(72) Inventor: ZENG, Yan, Shenzhen, Guangdong 518129 (CN); LI, Hongbao, Shenzhen, Guangdong 518129 (CN)
(74) Representative: Gill Jennings & Every LLP
(86) International application number: PCT/CN2024/128469
(87) International publication number: WO 2025/092811

(57) **Abstract**

A blood pressure monitoring method, a wearable device, and a storage medium are provided. The method is applied to a wearable device (100), the wearable device includes a motion sensor (203G), and the method includes: The wearable device collects a first blood pressure measurement value; the wearable device obtains motion data collected by the motion sensor; the wearable device determines a first blood pressure compensation value when determining, based on the motion data, that a user is in a first posture; and the wearable device determines a first blood pressure monitoring value based on the first blood pressure measurement value and the first blood pressure compensation value. The first posture includes any one of the following: a standing posture, a sitting posture, or a lying posture. The wearable device may revise a blood pressure measurement result based on different user postures, thereby improving accuracy of measuring blood pressure by the wearable device.

## Description

This application claims priority to Chinese Patent Application No. 202311440599.6, filed with the China National Intellectual Property Administration on October 31, 2023 and entitled "BLOOD PRESSURE MONITORING METHOD, WEARABLE DEVICE, AND STORAGE MEDIUM", which is incorporated herein by reference in its entirety.

### TECHNICAL FIELD

This application relates to the terminal field, and in particular, to a blood pressure monitoring method, a wearable device, and a storage medium.

### BACKGROUND

With improvement of living standards, people's health attracts increasing attention. Hypertension is a common cardiovascular disease, and regularly measuring blood pressure is one of important means to ensure health of hypertensive patients. Ambulatory blood pressure measurement is a technology of continuously measuring blood pressure of a user 24 hours without affecting daily activities of the user, and a plurality of blood pressure measurement values may be obtained within 24 hours. Usually, measurement is performed once every 10 minutes to 15 minutes, and an average value of the plurality of blood pressure measurement values within 24 hours is used as a blood pressure value. Currently, there is a cuff-based blood pressure monitor for measuring ambulatory blood pressure of a user. However, to use the cuff-based blood pressure monitor, the user needs to carry the cuff-based blood pressure monitor 24 hours. It is inconvenient for the user to use the cuff-based blood pressure monitor.

To facilitate blood pressure measurement of the user, a wrist ambulatory blood pressure monitor is provided. The wrist ambulatory blood pressure monitor is comfortable to wear, and saves time and effort. However, the wrist ambulatory blood pressure monitor requires the user to maintain a standard sitting posture and keep a blood pressure measurement part flush with the heart, to ensure accuracy of a blood pressure measurement result. Usually, a posture in which the user measures blood pressure is not standard, and blood pressure measurement results of the user in different measurement postures are inaccurate. How to improve accuracy of the blood pressure measurement result of the wrist ambulatory blood pressure monitor needs to be further studied.

### SUMMARY

This application provides a blood pressure monitoring method, a wearable device, and a storage medium. The wearable device may revise a blood pressure measurement result based on different user postures, thereby improving accuracy of measuring blood pressure by the wearable device.

According to a first aspect, this application provides a blood pressure monitoring method. The method is applied to a wearable device, the wearable device includes a motion sensor, and the method includes: The wearable device collects a first blood pressure measurement value; the wearable device obtains motion data collected by the motion sensor; the wearable device determines a first blood pressure compensation value when determining, based on the motion data, that a user is in a first posture; and the wearable device determines a first blood pressure monitoring value based on the first blood pressure measurement value and the first blood pressure compensation value. The first posture includes any one of the following: a standing posture, a sitting posture, or a lying posture.

In some embodiments, the motion sensor may be an acceleration sensor and/or an angular velocity sensor. The motion data may be one or more types of data such as acceleration data, /or angular velocity data, a motion step count, a heart rate, and a motion trajectory.

According to the method provided in the first aspect, the wearable device may revise a blood pressure measurement result based on different user postures, thereby improving accuracy of measuring blood pressure by the wearable device.

With reference to the first aspect, in a possible implementation, that the wearable device determines the first blood pressure compensation value when determining, based on the motion data, that the user is in the first posture specifically includes: The wearable device determines the first blood pressure compensation value when determining, based on the motion data, that the user is in the first posture and the wearable device is worn on a left-hand wrist.

In another possible implementation, the method further includes: The wearable device determines a second blood pressure compensation value when determining, based on the motion data, that the user is in the first posture and the wearable device is worn on a right-hand wrist. The first blood pressure compensation value is different from the second blood pressure compensation value.

In some embodiments, the wearable device may determine a motion trajectory of the wearable device based on the motion data, and determine, based on the motion trajectory of the wearable device, whether the wearable device is worn on a left hand or a right hand.

In this way, when identifying a user posture, the wearable device can further identify whether the wearable device is worn on the left hand or the right hand, and determine different blood pressure compensation values based on whether the wearable device is worn on the left hand or the right hand. This can further improve accuracy of blood pressure measurement.

With reference to the first aspect, in a possible implementation, that the wearable device determines the first blood pressure compensation value when determining, based on the motion data, that the user is in the first posture specifically includes: The wearable device determines a first included angle between a positive direction of an X axis and a positive direction of a first direction of the wearable device, where the first included angle is greater than 0 degrees and less than 180 degrees, the first direction is parallel to gravitational acceleration G, the positive direction of the first direction is opposite to a positive direction of the gravitational acceleration G, and when the wearable device is worn on the left-hand wrist, the X axis is parallel to a forearm, and the positive direction of the X axis is a direction pointing to a user finger; and the wearable device determines the first blood pressure compensation value based on the first included angle when determining, based on the motion data, that the user is in the first posture.

When identifying the user posture, the wearable device further identifies an angle between a wrist wearing the wearable device and the positive direction of the first direction, and determines different blood pressure compensation values based on different angles. This can further improve accuracy of blood pressure measurement.

With reference to the first aspect, in a possible implementation, when the first posture is the lying posture, that the wearable device determines the first blood pressure compensation value when determining, based on the motion data, that the user is in the first posture specifically includes: The wearable device determines the first blood pressure compensation value when determining, based on the motion data, that the user is in the lying posture and a center of a palm corresponding to a wrist wearing the wearable device faces a ground.

With reference to the first aspect, in a possible implementation, the method further includes: The wearable device determines a third blood pressure compensation value when determining, based on the motion data, that the user is in the lying posture and the center of the palm corresponding to the wrist wearing the wearable device faces a sky. The third blood pressure compensation value is different from the first blood pressure compensation value.

With reference to the first aspect, in a possible implementation, the method further includes: The wearable device determines a fourth blood pressure compensation value when determining, based on the motion data, that the user is in the lying posture and the center of the palm corresponding to the wrist wearing the wearable device faces sideward. The fourth blood pressure compensation value is different from the first blood pressure compensation value and the third blood pressure compensation value.

In this way, when a first user posture is the lying posture, the wearable device may determine different blood pressure compensation values based on a placement posture of the palm corresponding to the wrist wearing the wearable device. This can further improve accuracy of blood pressure measurement.

With reference to the first aspect, in a possible implementation, when the first posture is the standing posture, the wearable device determines, based on the motion data, that the user is in the standing posture, specifically including: when the motion data meets a first condition, the wearable device determines that the user is in the standing posture. The first condition includes but is not limited to any one or more of the following: a plurality of groups of heart rate values within first duration before blood pressure measurement starts are greater than a first heart rate value; a change value of an included angle between the positive direction of the X axis and the first direction within second duration before the blood pressure measurement starts is greater than a first angle value; and an acceleration component of the gravitational acceleration G on a Z axis is close to a minimum value, and the Z axis is perpendicular to a plane on which a display of the wearable device is located.

This is not limited thereto. The wearable device may further determine the standing posture based on another condition. This is not limited in this application.

With reference to the first aspect, in a possible implementation, when the first posture is the sitting posture, the wearable device determines, based on the motion data, that the user is in the sitting posture, specifically including: when the motion data meets a second condition, the wearable device determines that the user is in the sitting posture. The second condition includes but is not limited to any one or more of the following: a plurality of groups of heart rate values within first duration before blood pressure measurement starts are greater than a second heart rate value and less than a first heart rate value, where the second heart rate value is less than the first heart rate value; a change value of an included angle between the positive direction of the X axis and the first direction within second duration before the blood pressure measurement starts is greater than a second angle value and less than a first angle value, and the second angle value is less than the first angle value; and an acceleration component of the gravitational acceleration G on a Z axis is close to a minimum value, and the Z axis is perpendicular to a plane on which a display of the wearable device is located.

This is not limited thereto. The wearable device may further determine the sitting posture based on another condition. This is not limited in this application.

With reference to the first aspect, in a possible implementation, when the first posture is the lying posture, the wearable device determines, based on the motion data, that the user is in the lying posture, specifically including: when the motion data meets a third condition, the wearable device determines that the user is in the sitting posture. The third condition includes but is not limited to any one or more of the following: a plurality of groups of heart rate values within first duration before blood pressure measurement starts are less than a second heart rate value; a motion trajectory of the wearable device satisfies a preset motion trajectory, and the preset motion trajectory is an up-down motion trajectory in a vertical direction; and acceleration components of the gravitational acceleration G on the X axis and a Y axis are close to a minimum value, and the Y axis is perpendicular to the X axis.

This is not limited thereto. The wearable device may further determine the lying posture based on another condition. This is not limited in this application.

With reference to the first aspect, in a possible implementation, before collecting, by the wearable device, the first blood pressure measurement value, the method further includes: The wearable device displays first prompt information when detecting that the wearable device switches from a non-worn state to a worn state, where the first prompt information is used to prompt the user to confirm whether the wearable device is worn by a local user; and the wearable device receives a first operation performed by the user on a first option in the first prompt information, and in response to the first operation, confirms that the wearable device is worn by the local user; and after determining the first blood pressure monitoring value, the method further includes: The wearable device stores the first blood pressure monitoring value in a first storage area, where the first storage area stores blood pressure measurement data of the local user.

With reference to the first aspect, in a possible implementation, the method further includes: The wearable device receives a second operation performed by the user on a second option in the first prompt information, and in response to the second operation, confirms that the wearable device is worn by a non-local user; and after determining the first blood pressure monitoring value, the method further includes: The wearable device stores the first blood pressure monitoring value in a second storage area, where the second storage area stores blood pressure measurement data of a non-local user, and the first storage area is different from the second storage area.

In this way, before starting to measure the blood pressure, the wearable device may prompt the user to choose whether the wearable device is worn by the local user. Therefore, blood pressure measurement data of different users can be prevented from being stored together, and accuracy of an analysis result of blood pressure measurement data of a single user is affected.

According to a second aspect, this application provides a wearable device. The wearable device includes a motion sensor, a memory, and a processor, the motion sensor, the memory, and the processor are coupled, the memory is configured to store a computer program, and when the processor executes and invokes the computer program, the wearable device is enabled to perform the blood pressure monitoring method according to any possible implementation of any one of the foregoing aspects.

According to a third aspect, this application provides a computer-readable storage medium, including instructions. When the instructions are run on a wearable device, the wearable device is enabled to perform the blood pressure monitoring method according to any possible implementation of any one of the foregoing aspects.

According to a fourth aspect, this application provides a chip system. The chip system includes one or more processors, and the processor is configured to invoke computer instructions, to perform the blood pressure monitoring method according to any possible implementation of any one of the foregoing aspects.

According to a fifth aspect, this application provides a computer program product including instructions. When the computer program product runs on a wearable device, the wearable device is enabled to perform the blood pressure monitoring method according to any possible implementation of any one of the foregoing aspects.

For descriptions of beneficial effects of the second aspect to the fifth aspect, refer to the descriptions of the beneficial effects of the first aspect. Details are not described herein again in this application.

### BRIEF DESCRIPTION OF DRAWINGS

FIG. 1 is a diagram of a standard measurement posture;
FIG. 2 is a diagram in which a user wears a wearable device 100;
FIG. 3 is a diagram of a composition structure of a wearable device 100;
FIG. 4A is an example diagram of a principle of an oscillometric method according to an embodiment of this application;
FIG. 4B is another example diagram of a principle of an oscillometric method according to an embodiment of this application;
FIG. 5A is a diagram of a hardware structure of a wearable device 100;
FIG. 5B is a diagram of a composition structure of an airbag, an air pump, and a pneumatic connection component according to an embodiment of this application;
FIG. 6A to FIG. 6F are diagrams of enabling a blood pressure measurement mode;
FIG. 6G to FIG. 6M are diagrams in which a wearable device 100 confirms a user identity;
FIG. 7A to FIG. 7C are diagrams in which a wearable device 100 prompts a user to measure blood pressure;
FIG. 8A is a diagram in which a user wears a wearable device 100 when standing;
FIG. 8B is another diagram in which a user wears a wearable device 100 when standing;
FIG. 8C is a schematic flowchart of a method for correcting a collected blood pressure measurement value by a wearable device 100;
FIG. 8D to FIG. 8F are a group of diagrams in which a wrist wearing a wearable device 100 is located at different placement locations when a user is in a standing posture;
FIG. 8G to FIG. 8I are another group of diagrams in which a wrist wearing a wearable device 100 is located at different placement locations when a user is in a standing posture;
FIG. 9A is a diagram in which a user wears a wearable device 100 when sitting;
FIG. 9B is another diagram in which a user wears a wearable device 100 when sitting;
FIG. 9C is a schematic flowchart of a method for correcting a collected blood pressure measurement value by a wearable device 100;
FIG. 9D to FIG. 9F are a group of diagrams in which a wrist wearing a wearable device 100 is located at different placement locations when a user is in a sitting posture;
FIG. 9G to FIG. 9I are another group of diagrams in which a wrist wearing a wearable device 100 is located at different placement locations when a user is in a sitting posture;
FIG. 10A is a diagram in which a user wears a wearable device 100 when lying;
FIG. 10B is another diagram in which a user wears a wearable device 100 when lying;
FIG. 10C is a schematic flowchart of a method for correcting a collected blood pressure measurement value by a wearable device 100;
FIG. 10D to FIG. 10F are a group of diagrams in which a wrist wearing a wearable device 100 is located at different placement locations when a user is in a lying posture;
FIG. 10G to FIG. 10I are diagrams of three placement postures of a palm;
FIG. 10J to FIG. 10L are another group of diagrams in which a wrist wearing a wearable device 100 is located at different placement locations when a user is in a lying posture;
FIG. 11A to FIG. 11C are a group of diagrams in which a wearable device 100 displays a blood pressure measurement result;
FIG. 11D to FIG. 11G are another group of diagrams in which a wearable device 100 displays blood pressure monitoring values in a specific time period;
FIG. 12 is a diagram of a method procedure of a blood pressure measurement method according to this application; and
FIG. 13 is a diagram of a blood pressure measurement apparatus according to this application.

### DESCRIPTION OF EMBODIMENTS

The technical solutions according to embodiments of this application are clearly and completely described in the following with reference to the accompanying drawings. In the descriptions of embodiments of this application, "/" indicates or, unless otherwise specified. For example, A/B may indicate A or B. In this specification, "and/or" describes only an association relationship between associated objects, and indicates that three relationships may exist. For example, A and/or B may indicate the following three cases: Only A exists, both A and B exist, and only B exists. In addition, in the descriptions of embodiments of this application, "a plurality of" means two or more than two.

The following terms "first" and "second" are merely intended for a purpose of description, and shall not be understood as an indication or implication of relative importance or implicit indication of a quantity of indicated technical features. Therefore, a feature limited by "first" and "second" may explicitly or implicitly include one or more features. In the descriptions of embodiments of this application, unless otherwise specified, "a plurality of" means two or more.

A term "user interface (user interface, UI)" in the following embodiments of this application is a medium interface for interaction and information exchange between an application or an operating system and a user, and implements conversion between an internal form of information and a form acceptable to the user. The user interface is usually represented in a form of a graphical user interface (graphical user interface, GUI), and is a user interface that is related to a computer operation and that is displayed in a graphic manner. The user interface may be a visual interface element, for example, text, an icon, a button, a menu, a tab, a text box, a dialog box, a status bar, a navigation bar, or a widget, that is displayed on a display of a wearable device.

To facilitate measurement of user blood pressure, a user may measure the user blood pressure by using a wrist ambulatory blood pressure monitor. The wrist ambulatory blood pressure monitor is worn on a wrist of the user, is easy to wear, and does not affect daily activities of the user. In addition, the wrist ambulatory blood pressure monitor facilitates measurement of ambulatory blood pressure of the user, to monitor the user blood pressure in real time.

With development of electronic technologies, functions of wearable devices are continuously enhanced. For example, a wearable device like a band or a watch may provide a blood pressure measurement function, to help a user measure blood pressure of the user anytime and anywhere, to learn of a physical condition of the user. The user may wear the wearable device on the wrist, so that not only the user blood pressure can be monitored in real time, but also other functions such as playing music, making/answering a call, sending a message, and viewing motion data can be implemented based on the wearable device.

When the user measures the blood pressure by using the wearable device worn on the wrist, the user needs to maintain a standard measurement posture. The standard measurement posture may be keeping a sitting posture and keeping the wrist wearing the wearable device flush with the heart, to ensure accuracy of a blood pressure measurement result.

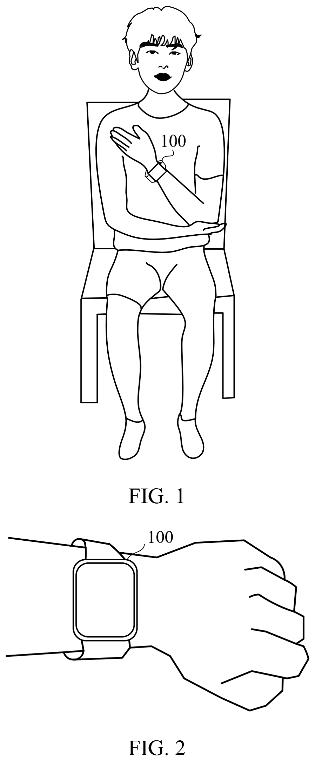

FIG. 1 is a diagram of a standard measurement posture.

As shown in FIG. 1, a wearable device 100 is worn on a wrist of a left hand of a user, and the user keeps a sitting posture and raises the wrist of the left hand upward to be flush with a location of the heart. In this way, a blood pressure measurement value collected by the wearable device 100 is close to a real value.

However, in a process in which the wearable device 100 measures an ambulatory blood pressure, the user cannot always keep the standard measurement posture. When the wearable device 100 measures the blood pressure, if the user does not keep the standard measurement posture, an error between the blood pressure measurement value collected by the wearable device 100 and the real value is large, and a measurement result is inaccurate.

To improve accuracy of the blood pressure measurement value collected by the wearable device 100, this application provides a blood pressure measurement method. In the method, the wearable device 100 may identify a user posture, and determine a blood pressure compensation value based on the user posture. Then, the wearable device 100 determines a final blood pressure monitoring value based on the blood pressure measurement value and the blood pressure compensation value that are collected by the wearable device 100.

A motion sensor includes but is not limited to an inertial sensor (Inertial Measurement Unit, IMU), configured to determine the posture. Specifically, after the wearable device 100 is in a worn state, the inertial sensor may collect motion data, determine the user posture, and determine the blood pressure compensation value based on the user posture.

The motion data includes but is not limited to acceleration data and/or angular velocity data.

The user posture includes but is not limited to a standing posture, a sitting posture, a lying posture, and the like.

In the method, when the wearable device 100 measures the blood pressure but the user does not keep the standard measurement posture, the blood pressure measurement value collected by the wearable device 100 may be verified based on the user posture, to improve accuracy of the blood pressure measurement value collected by the wearable device 100.

For how the wearable device 100 identifies the user posture and how to determine the blood pressure compensation value based on the user posture, refer to the following descriptions. Details are not described herein again in this application.

FIG. 2 is a diagram in which a user wears a wearable device 100.

As shown in FIG. 2, the user may wear the wearable device 100 on a wrist of the user.

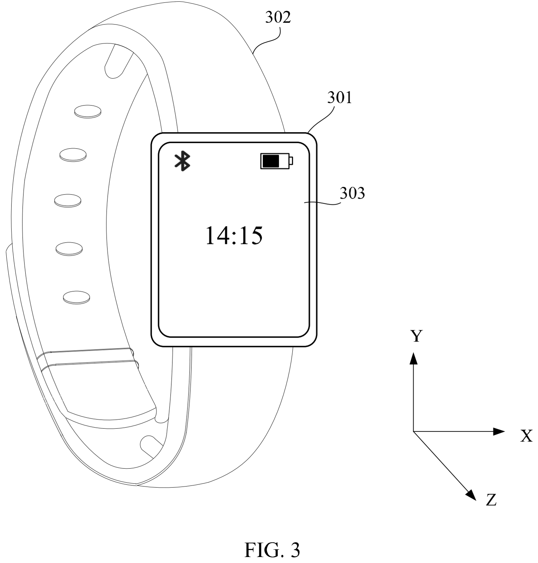

FIG. 3 is a diagram of a composition structure of a wearable device 100.

As shown in FIG. 3, the wearable device 100 may include a watch body 301 and a wearable component 302.

A direction that is perpendicular to a right edge and a left edge of the watch body 301 and that is away from the left edge of the watch body 301 is a positive direction of an X axis. A direction that is perpendicular to an upper edge and a lower edge of the watch body 301 and that is away from the lower edge of the watch body 301 is a positive direction of a Y axis. The X axis and the Y axis may determine an X-Y plane, and the X-Y plane is parallel to a plane on which a display in the watch body 301 is located. A direction that is perpendicular to the X-Y plane and that is away from the wearable component is a positive direction of a Z axis.

An inertial sensor is configured in the watch body 301, and the watch body 301 collects motion data by using the inertial sensor. The watch body 301 may include a display 303. The display 303 may be configured to display time, a battery level of the watch body 301, a Bluetooth identifier, a received message, motion data of the user, and other content. The display 303 may be configured to receive a tap operation of the user to turn on the display, enable or disable a sport mode, or the like. The display 303 may further record a moving step count and consumed energy of the user, and has basic functions such as an incoming call reminder and a message notification. In a possible implementation, the watch body 301 may establish a wireless communication connection to the wearable device 100 through Bluetooth. The watch body 301 may send the motion data of the user to the wearable device 100 to which the connection is established. In addition, when the wearable device 100 receives an incoming call or a message notification, the watch body 301 may receive an instruction from a mobile phone, to remind the user about the incoming call or the message notification.

The wearable component 302 is used for mounting the watch body 301. For example, the wearable component 302 may be a wristband strap, a watch strap, or another apparatus. The wearable component 302 is an apparatus that can attach the watch body 301 to the wrist of the user. The wearable device 100 is attached to the wrist of the user, so that an inertial sensor collects motion data of the wrist of the user, to monitor motion of the wrist of the user and determine the user posture.

When the wearable device 100 starts to measure blood pressure, the wearable device 100 may control the wearable component 302 to shrink and then expand, to measure user blood pressure.

In some embodiments, a process in which the wearable device 100 measures blood pressure may include: The wearable device 100 first inflates the wearable component 302 to temporarily obstruct a brachial arterial vessel; then records, during slow deflation, a barometric pressure value of the wearable component 302 and a pulse signal generated by a pulse; and finally determines the user blood pressure based on the barometric pressure value of the wearable component 302 and an amplitude or an envelope of the pulse signal. Blood flow causes lateral pressure to a blood vessel wall. A change in a magnitude of the lateral pressure causes slight vibration of the blood vessel wall. The pulse signal is a signal generated through the slight vibration of the blood vessel wall. Determining the user blood pressure based on the barometric pressure value of the wearable component 302 and the amplitude or the envelope of the pulse signal is also referred to as an oscillometric method.

In another embodiment, a process in which the wearable device 100 measures blood pressure may include: The wearable device 100 may gradually inflate the wearable component 302, so that the brachial arterial vessel changes from being gradually blocked to being completely blocked; record a barometric pressure value of the wearable component 302 and a pulse signal generated by a pulse; then determine user blood pressure based on the barometric pressure value of the wearable component 302 and an amplitude or an envelope of the pulse signal; and finally perform deflation. Blood flow causes lateral pressure to a blood vessel wall. A change in a magnitude of the lateral pressure causes slight vibration of the blood vessel wall. The pulse signal is a signal generated through the slight vibration of the blood vessel wall. Determining the user blood pressure based on the barometric pressure value of the wearable component 302 and the amplitude or the envelope of the pulse signal is also referred to as an oscillometric method.

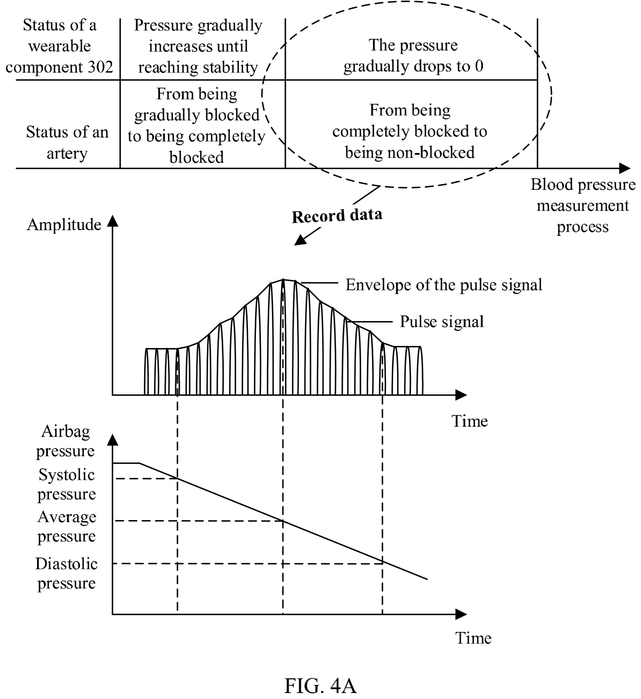

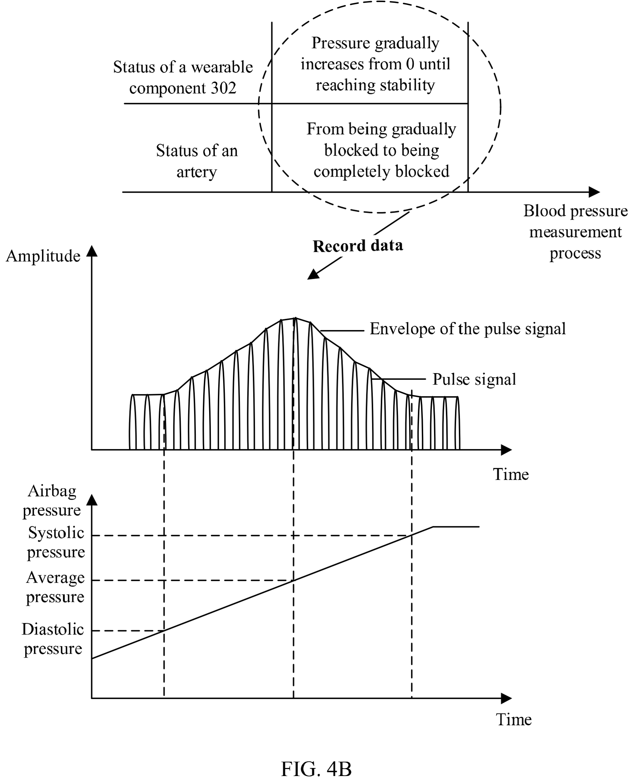

The following describes example principles of the oscillometric method based on content shown in FIG. 4A and FIG. 4B.

FIG. 4A is an example diagram of a principle of an oscillometric method according to an embodiment of this application.

As shown in FIG. 4A, in a process in which a wearable device 100 inflates a wearable component 302 to temporarily obstruct a brachial arterial vessel, a status of the wearable component 302 is that pressure gradually increases until reaching stability, and a status of an artery is from being gradually blocked to being completely blocked. Then, during slow deflation, the status of the wearable component 302 is that pressure gradually drops to 0, and the status of the artery is from being completely blocked to being non-blocked. When the status of the wearable component 302 is that the pressure gradually drops to 0, a barometric pressure value and a pulse signal of the wearable component 302 are recorded. When the barometric pressure value of the wearable component 302 is greater than or equal to systolic pressure, the artery is obstructed, and the pulse signal is a fine oscillation wave. When the barometric pressure value of the wearable component 302 gradually decreases and is less than the systolic pressure and greater than average pressure, the artery gradually becomes non-blocked, and an amplitude of the pulse signal continuously increases. When the barometric pressure value of the wearable component 302 is equal to the average pressure, the amplitude of the pulse signal reaches a maximum value. When the barometric pressure value of the wearable component 302 continues to gradually decrease and is greater than diastolic pressure and less than the average pressure, the amplitude of the pulse signal gradually decreases. When the barometric pressure value of the wearable component 302 is less than the diastolic pressure, the pulse signal is a fine oscillation wave. Therefore, the wearable device 100 may determine the systolic pressure and the diastolic pressure of the user based on a change in the amplitude of the pulse signal and the barometric pressure value of the wearable component 302. In a possible implementation, the barometric pressure value and the pulse signal of the wearable component 302 may be determined by a barometric pressure sensor built in the wearable device 100.

FIG. 4B is another example diagram of a principle of an oscillometric method according to an embodiment of this application.

As shown in FIG. 4B, in a process in which a wearable device 100 inflates a wearable component 302 to temporarily obstruct a brachial arterial vessel, a status of the wearable component 302 is that pressure gradually increases until reaching stability, and a status of an artery is from being gradually blocked to being completely blocked. When the status of the wearable component 302 is that the pressure gradually increases until reaching stability, a barometric pressure value and a pulse signal of the wearable component 302 are recorded. When the barometric pressure value of the wearable component 302 gradually increases and diastolic pressure is less than average pressure, the pulse signal is a fine oscillation wave. When the barometric pressure value of the wearable component 302 continues to gradually increase and is greater than the diastolic pressure and less than the average pressure, an amplitude of the pulse signal gradually increases. When the barometric pressure value of the wearable component 302 is equal to the average pressure, the amplitude of the pulse signal reaches a maximum value. When the barometric pressure value of the wearable component 302 gradually increases and is greater than the average pressure and less than systolic pressure, the artery is gradually blocked, and the amplitude of the pulse signal continuously decreases. When the barometric pressure value of the wearable component 302 is greater than or equal to the systolic pressure, the artery is blocked, and the pulse signal is a fine oscillation wave. Therefore, the wearable device 100 may determine the systolic pressure and the diastolic pressure of the user based on a change in the amplitude of the pulse signal and the barometric pressure value of the wearable component 302. In a possible implementation, the barometric pressure value and the pulse signal of the wearable component 302 may be determined by a barometric pressure sensor built in the wearable device 100.

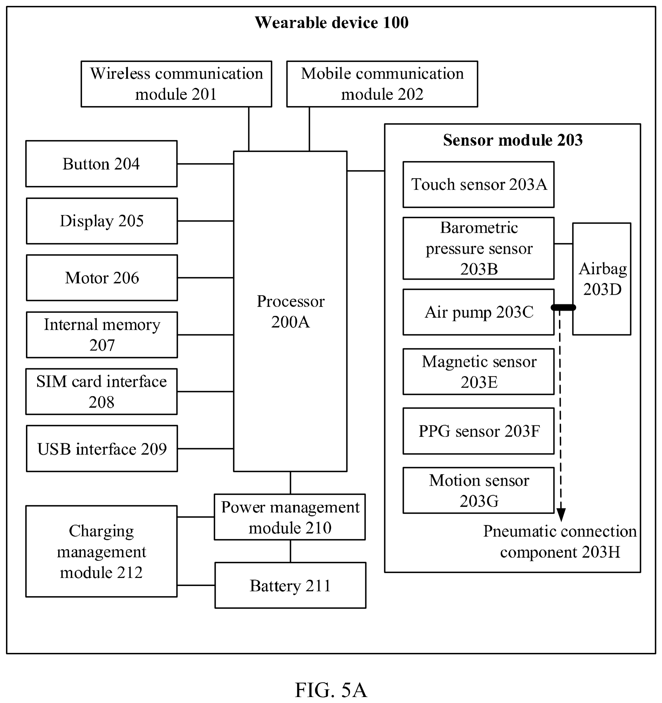

FIG. 5A is a diagram of a hardware structure of a wearable device 100.

As shown in FIG. 5A, the wearable device may be a band, a watch, or another wearable device; or the wearable device 100 may be a non-wearable device like a wall-mounted blood pressure monitor. A specific type of the wearable device is not particularly limited in this embodiment of this application. In this embodiment of this application, only an example in which the wearable device 100 is a watch is used for description.

The wearable device 100 may include a processor 200A, a wireless communication module 201, a mobile communication module 202, a sensor module 203, a button 204, a display 205, a motor 206, an internal memory 207, a SIM card interface 208, a USB interface 209, a power management module 210, a battery 211, and a charging management module 212. The sensor module 203 may include a touch sensor 203A, a barometric pressure sensor 203B, an air pump 203C, an airbag 203D, a magnetic sensor 203E, a photoplethysmography (photoplethysmography, PPG) sensor 203F, a motion sensor 203G, and a pneumatic connection component 203H. A function of the airbag 203D is similar to a function of a wearable component 302.

It can be understood that the structure shown in this embodiment of the present invention does not constitute a specific limitation on the wearable device. In some other embodiments of this application, the wearable device may include more or fewer components than those shown in the figure, or some components may be combined, or some components may be split, or the components may be arranged differently. The components shown in the figure may be implemented by hardware, software, or a combination of software and hardware.

The processor 200A may include one or more processing units. For example, the processor 200A may include an application processor (application processor, AP), a modem processor, a graphics processing unit (graphics processing unit, GPU), an image signal processor (image signal processor, ISP), a controller, a video codec, a digital signal processor (digital signal processor, DSP), a baseband processor, and/or a neural-network processing unit (neural-network processing unit, NPU). Different processing units may be independent components, or may be integrated into one or more processors.

In some embodiments, the processor 200A may include one or more interfaces. The interface may include an inter-integrated circuit (inter-integrated circuit, I2C) interface, an inter-integrated circuit sound (inter-integrated circuit sound, I2S) interface, a pulse code modulation (pulse code modulation, PCM) interface, a universal asynchronous receiver/transmitter (universal asynchronous receiver/transmitter, UART) interface, a mobile industry processor interface (mobile industry processor interface, MIPI), a general-purpose input/output (general-purpose input/output, GPIO) interface, a subscriber identity module (subscriber identity module, SIM) interface, a universal serial bus (universal serial bus, USB) interface, and/or the like.

In some embodiments, the processor 200A may alternatively be a micro processing unit (microcontroller unit, MCU).

The I2C interface is a bidirectional synchronous serial bus, and includes a serial data line (serial data line, SDA) and a serial clock line (serial clock line, SCL). In some embodiments, the processor 200A may include a plurality of groups of I2C buses. The processor 200A may be coupled to the touch sensor 203A, the power management module 210, and the like separately through different 12C bus interfaces. For example, the processor 200A may be coupled to the touch sensor 203A through the I2C interface, so that the processor 200A communicates with the touch sensor 203A through the I2C bus interface, to implement a touch function of the wearable device.

The I2S interface may be configured to perform audio communication. The PCM interface may also be used to perform audio communication, and sample, quantize, and code an analog signal. The UART interface is a universal serial data bus, and is configured to perform asynchronous communication. The bus may be a two-way communication bus. The bus converts to-be-transmitted data between serial communication and parallel communication. In some embodiments, the UART interface is usually configured to connect the processor 200A to the wireless communication module 201. For example, the processor 200A communicates with a Bluetooth module in the wireless communication module 201 through the UART interface, to implement a Bluetooth function.

The MIPI interface may be configured to connect the processor 200A to a peripheral component like the display 205. The MIPI interface includes a camera serial interface (camera serial interface, CSI), a display serial interface (display serial interface, DSI), and the like. The processor 200A communicates with the display 205 through the DSI interface, to implement a display function of the wearable device.

The GPIO interface may be configured by software. The GPIO interface may be configured as a control signal or a data signal. The USB interface 209 is an interface that conforms to a USB standard specification, and may be specifically a mini USB interface, a micro USB interface, a USB Type-C interface, or the like. The USB interface 209 may be configured to connect to a charger to charge the wearable device, or may be configured to transmit data between the wearable device and a peripheral device.

It can be understood that an interface connection relationship between the modules in this embodiment of the present invention is merely an example for description, and does not constitute a limitation on a structure of the wearable device. In some other embodiments of this application, the wearable device may alternatively use an interface connection mode different from that in the foregoing embodiment, or use a combination of a plurality of interface connection modes.

The charging management module 212 is configured to receive a charging input from a charger. The charger may be a wireless charger or a wired charger. In some embodiments of wired charging, the charging management module 212 may receive a charging input from a wired charger through the USB interface 209. In some embodiments of wireless charging, the charging management module 212 may receive a wireless charging input through a wireless charging coil of the wearable device. When charging the battery 211, the charging management module 212 may further supply power to the wearable device through the power management module 210.

The power management module 210 is configured to connect to the battery 211, the charging management module 212, and the processor 200A. The power management module 210 receives an input from the battery 211 and/or the charging management module 212, and supplies power to the processor 200A, the internal memory 207, the display 205, the wireless communication module 201, and the like. The power management module 210 may be further configured to monitor parameters such as a battery capacity, a quantity of battery cycles, and a battery health status (electric leakage or impedance). In some other embodiments, the power management module 210 may alternatively be disposed in the processor 200A. In some other embodiments, the power management module 210 and the charging management module 212 may alternatively be disposed in a same component.

A wireless communication function of the wearable device may be implemented by the mobile communication module 202, the wireless communication module 201, the modem processor, the baseband processor, and the like.

The mobile communication module 202 may provide a solution applied to the wearable device for wireless communication such as 2G/3G/4G/5G. The mobile communication module 202 may include at least one filter, a switch, a power amplifier, a low noise amplifier (low noise amplifier, LNA), and the like. The mobile communication module 202 may receive an electromagnetic wave through the antenna, perform processing such as filtering or amplification on the received electromagnetic wave, and transmit a processed electromagnetic wave to the modem processor for demodulation. In some embodiments, at least some functional modules of the mobile communication module 202 may be disposed in the processor 200A. In some embodiments, at least some functional modules of the mobile communication module 202 may be disposed in a same component as at least some modules of the processor 200A.

The wireless communication module 201 may provide a solution applied to the wearable device for wireless communication such as a wireless local area network (wireless local area network, WLAN) (for example, a wireless fidelity (wireless fidelity, Wi-Fi) network), Bluetooth (Bluetooth, BT), a global navigation satellite system (global navigation satellite system, GNSS), frequency modulation (frequency modulation, FM), a near field communication (near field communication, NFC) technology, or an infrared (infrared, IR) technology. The wireless communication module 201 may be one or more components integrating at least one communication processing module. The wireless communication module 201 receives an electromagnetic wave through the antenna, performs frequency modulation and filtering on an electromagnetic wave signal, and sends a processed signal to the processor 200A. The wireless communication module 201 may further receive a to-be-sent signal from the processor 200A, perform frequency modulation and amplification on the signal, and convert a processed signal into an electromagnetic wave for radiation through the antenna.

The button 204 includes a power button, a volume button, and the like. The button 204 may be a mechanical button or a touch button. The wearable device may receive an input on the button, and generate a button signal input related to a user setting and function control of the wearable device.

The display 205 is configured to display an image, a video, or the like. The display 205 includes a display panel. The display panel may be a liquid crystal display (liquid crystal display, LCD), an organic light-emitting diode (organic light-emitting diode, OLED), an active-matrix organic light-emitting diode (active-matrix organic light-emitting diode, AMOLED), a flexible light-emitting diode (flex light-emitting diode, FLED), a quantum dot light-emitting diode (quantum dot light-emitting diode, QLED), or the like. In some embodiments, the wearable device may include one or N displays 205, where N is a positive integer greater than 1.

The motor 206 may generate a vibration prompt. The motor 206 may be configured to produce an incoming call vibration prompt or a touch vibration feedback. For example, touch operations performed on different applications (for example, photographing and audio playback) may correspond to different vibration feedback effects. The motor 206 may also correspond to different vibration feedback effects for touch operations performed on different areas of the display 205.

The internal memory 207 may include one or more random access memories (random access memory, RAM) and one or more non-volatile memories (non-volatile memory, NVM).

The random access memory may include a static random access memory (static random access memory, SRAM), a dynamic random access memory (dynamic random access memory, DRAM), a synchronous dynamic random access memory (synchronous dynamic random access memory, SDRAM), a double data rate synchronous dynamic random access memory (double data rate synchronous dynamic random access memory, DDR SDRAM, for example, a 5th generation DDR SDRAM is usually referred to as a DDR5 SDRAM), and the like.

The non-volatile memory may include a magnetic disk storage device and a flash memory (flash memory). According to an operating principle, the flash memory may be classified into NOR FLASH, NAND FLASH, 3D NAND FLASH, and the like; according to potential orders of cells, the flash memory may be classified into a single-level cell (single-level cell, SLC), a multi-level cell (multi-level cell, MLC), a triple-level cell (triple-level cell, TLC), a quad-level cell (quad-level cell, QLC), and the like; and according to storage specifications, the flash memory may be classified into a universal flash storage (English: universal flash storage, UFS), an embedded multi media card (embedded multi media Card, eMMC), and the like. The processor 200A may directly perform a read or write operation on the random access memory. The random access memory may be configured to store executable programs (for example, machine instructions) of an operating system or another running program, and may be further configured to store data of a user and an application, and the like. The non-volatile memory may also store an executable program, data of the user and an application, and the like, which may be pre-loaded to the random access memory for the processor 200A to directly perform a read or write operation.

The SIM card interface 208 is used for connecting a SIM card. The SIM card may be inserted into the SIM card interface 208 or removed from the SIM card interface 208, to implement contact with or separation from the wearable device. The wearable device may support one or N SIM card interfaces, where N is a positive integer greater than 1. The SIM card interface 208 may support a nano-SIM card, a micro-SIM card, a SIM card, and the like. A plurality of cards may be inserted in a same SIM card interface 208 at the same time. The plurality of cards may be of a same type or different types. The SIM card interface 208 is also compatible with different types of SIM cards. The SIM card interface 208 is also compatible with an external memory card. The wearable device interacts with a network through the SIM card, to implement functions such as calling and data communication. In some embodiments, an eSIM, namely, an embedded SIM card, is used for the wearable device. The eSIM card may be embedded into the wearable device, and cannot be separated from the wearable device.

In some embodiments, the wearable device 100 may alternatively not include the SIM card interface 208.

The touch sensor 203A is also referred to as a "touch device". The touch sensor 203A may be disposed in the display 205. The touch sensor 203A and the display 205 constitute a touchscreen, which is also referred to as a "touch screen". The touch sensor 203A is configured to detect a touch operation performed on or near the touch sensor. The touch sensor may transfer the detected touch operation to the application processor to determine a type of the touch event. The display 205 may provide a visual output related to the touch operation. In some other embodiments, the touch sensor 203A may alternatively be disposed on a surface of the wearable device at a location different from a location of the display 205.

The barometric pressure sensor 203B is configured to measure barometric pressure. In some embodiments of this application, the wearable device measures barometric pressure in the airbag 203D through the barometric pressure sensor 203B. In some embodiments of this application, some components of the barometric pressure sensor 203B are located in the airbag 203D, and are configured to sense barometric pressure in the airbag 203D.

The air pump 203C is configured to perform inflation and deflation. In some embodiments of this application, the wearable device inflates the airbag 203D through the air pump 203C, where the air pump 203C is connected to the airbag 203D through the pneumatic connection component 203H. The airbag 203D is configured to press against a blood vessel of the user.

The magnetic sensor 203E includes a Hall effect sensor. In some embodiments of this application, the wearable device may determine, through the magnetic sensor 203F, whether the airbag 203D on the wearable device is removed. For example, the airbag 203D or a watch strap connected to the airbag 203D may be equipped with a magnet. The wearable device may determine, through the magnetic sensor, a magnetic flux generated by the airbag 203D or the magnet on the airbag 203D, to determine whether the airbag 203D on the wearable device is removed.

The PPG sensor 203F is configured to obtain health data of the user based on a PPG signal collected by the PPG sensor 203F. The health data of the user includes but is not limited to a heart rate, blood oxygen, a respiratory rate, blood oxygen saturation (SaO2), and the like.

The motion sensor 203G includes an inertial sensor, and the motion sensor 203G may be configured to collect motion data, and determine a user posture based on the motion data.

It should be noted that the pneumatic connection component 203H may be an independent component, or the pneumatic connection component 203H may be a pneumatic line formed by combining other hardware modules, or the pneumatic connection component 203H may be a part of another component, for example, a part of the air pump 203C, or a part of the airbag 203D.

It should be noted that the sensor module 203 may further include an acceleration sensor, an infrared sensor, and the like.

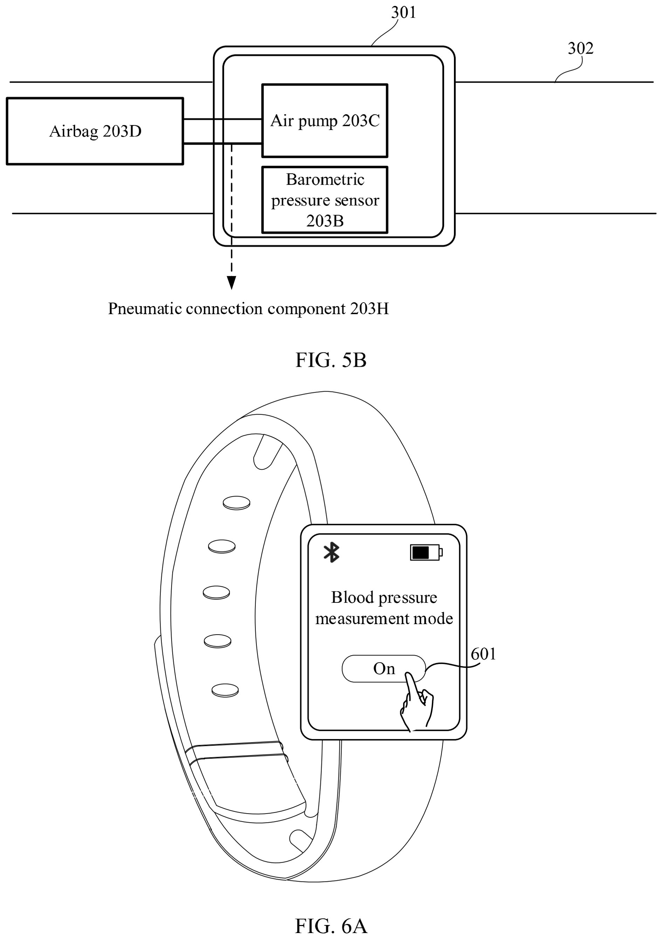

As shown in FIG. 5B, when the wearable device 100 is a watch, the airbag 203D is attached to a side, close to a body, of the wearable component 302. The air pump 203C is connected to the airbag 203D through the pneumatic connection component 203H. The airbag 203D may be attached only to one side of the wearable component 302, and the side of the wearable component 302 may be located above an artery location on a wrist of the user, for example, above a radial artery location.

The air pump 203C may be located in a watch body of the smartwatch. The airbag 203D may be connected to a buckle of a watch strap, and the airbag 203D is connected to a watch face through an air vent cover. Correspondingly, the airbag 203D may be separated from the watch strap, or may be separated from the watch face.

The following describes how a wearable device 100 identifies a user posture and blood pressure measurement values in different user postures.

In some embodiments, after receiving a user operation, the wearable device 100 may start to measure blood pressure and obtain a blood pressure measurement value.

In another embodiment, the wearable device 100 may alternatively automatically measure blood pressure and obtain a blood pressure measurement value. In this way, the wearable device 100 may obtain ambulatory blood pressure of a user through measurement, to monitor user blood pressure in real time.

**In this application, that the wearable device 100 measures the user blood pressure includes but is not limited to the following steps: enabling a blood pressure measurement mode, starting to measure the blood pressure, and displaying a blood pressure measurement result.**

### Enable the blood pressure measurement mode

FIG. 6A to FIG. 6F are diagrams of enabling a blood pressure measurement mode.

In some embodiments, the user may enable the blood pressure measurement mode in the wearable device 100.

For example, as shown in FIG. 6A, the wearable device 100 receives a user, and in response to the user operation, displays a user interface shown in FIG. 6A. The user interface may include an option 601, and the option 601 is used to enable the blood pressure measurement mode of the wearable device 100.

As shown in FIG. 6A, the wearable device 100 may receive an input operation (for example, a tap) performed by the user on the option 601, and in response to the input operation performed by the user, the wearable device 100 may enable the blood pressure measurement mode.

In a possible implementation, that the wearable device 100 enables the blood pressure measurement mode may mean that the wearable device 100 may periodically/aperiodically automatically start to measure the user blood pressure, and the user does not need to actively intervene in measuring the user blood pressure.

In another possible implementation, that the wearable device 100 enables the blood pressure measurement mode may alternatively mean that the wearable device 100 may start to measure the user blood pressure a single time.

In another embodiment, the user may alternatively enable the blood pressure measurement mode in an electronic device 200 that establishes a connection to the wearable device 100.

For example, the wearable device 100 may establish a Bluetooth connection to the electronic device 200.

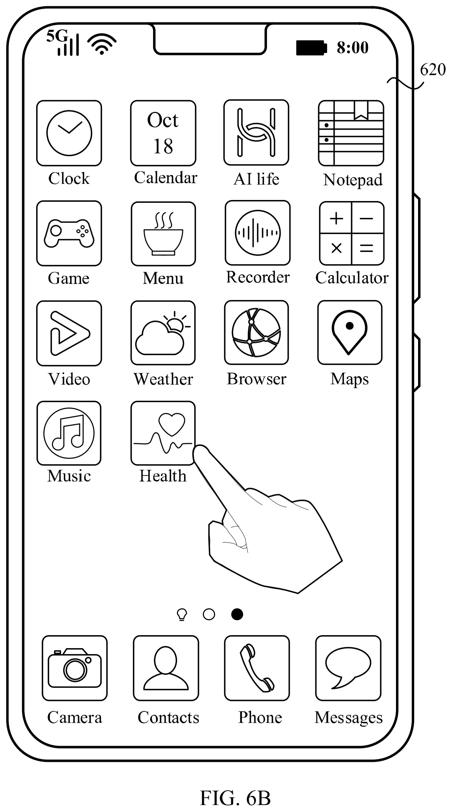

For example, as shown in FIG. 6B, the electronic device 200 displays a user interface 620, and the user interface 620 may be a home screen of the electronic device 200. The user interface 620 shows application icons of a plurality of applications.

In some embodiments, the user may enable the blood pressure measurement mode in a Health application of the electronic device 200.

In addition to the Health application, the user may enable the blood pressure measurement mode in another application. The Health application is only used as an example for description in this application, but shall not constitute a limitation.

For example, the user may further enable the blood pressure measurement mode in an AI life application.

As shown in FIG. 6B, the user interface 620 may include a Health application icon. In response to an operation performed on the Health application icon, the electronic device may open the Health application.

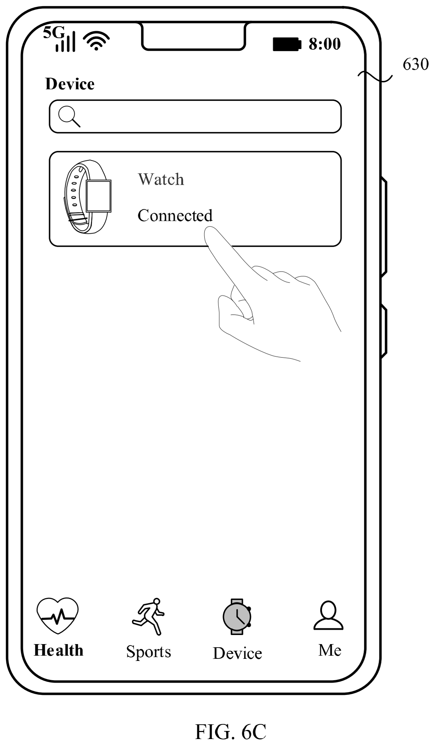

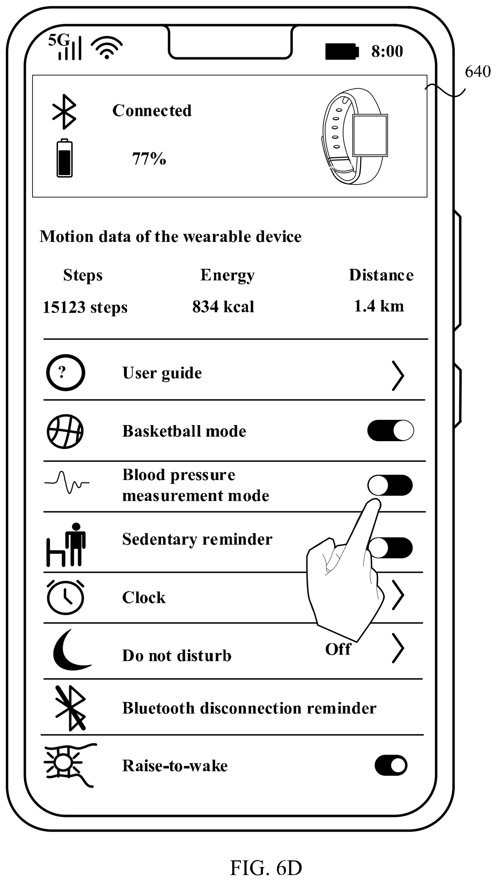

As shown in FIG. 6C, when the Health application is opened, the electronic device may display a user interface 630. The user interface 630 may include a "Device" option. The "Device" option may be a "Device" option corresponding to the wearable device 100. A device icon and a device name of the wearable device 100 and a connection state between the wearable device 100 and the electronic device 200 may be displayed on the "Device" option. Content displayed on the "Device" option is not limited in this embodiment of this application. In response to an operation performed on the "Device" option, the electronic device may display a user interface 640 shown in FIG. 6D. The user interface 640 may be a user interface used to manage the wearable device 100 in the Health application.

The user interface 640 may include a device state, motion data, and a professional sports mode.

The device state may be used to indicate the connection state between the wearable device 100 and the electronic device and a battery level of the wearable device 100. For example, when it is detected that the electronic device establishes a communication connection relationship with the wearable device 100 in a Bluetooth connection manner, the device state may indicate that a connection manner is a Bluetooth connection and the connection state is "Connected". Further, the electronic device may obtain battery level information of the wearable device 100. The device state may indicate a current battery level of the wearable device 100, for example, 77%. Content indicated by the device state may further include more content. This is not limited in this embodiment of this application.

The motion data may include a moving step count, consumed energy, and a moving distance of the user that are recorded by the wearable device 100. Data in the motion data is one-day data that is of the user and that is recorded by the wearable device 100 in an operating state. The data may include a total moving step count, total consumed energy, and a total moving distance of the user in daily activities such as walking, playing basketball, and running.

The professional sports mode may be used to enable or disable the blood pressure measurement mode and a running mode. The professional sports mode may include an enabling button for the blood pressure measurement mode and an enabling button used to enable a running mode. In response to a user operation, for example, a touch operation, performed on the enabling button for the blood pressure measurement mode, the electronic device 200 may send, to the wearable device 100, an instruction for enabling the blood pressure measurement mode. Then, the electronic device 200 may display, in the user interface 640, a disabling button used to disable the blood pressure measurement mode. In response to a user operation, for example, a touch operation, performed on the disabling button for disabling the blood pressure measurement mode, the electronic device may send, to the wearable device 100, an instruction for disabling the blood pressure measurement mode, and display the user interface 640 shown in FIG. 6D.

In addition to the foregoing manner of enabling the blood pressure measurement mode of the wearable device 100, the blood pressure measurement mode of the wearable device 100 may be enabled in another manner. This is not limited in this application.

In response to enabling the blood pressure measurement mode, the wearable device 100 may enable the blood pressure measurement mode after a countdown of 3 seconds after vibration. When the blood pressure measurement mode is enabled, the user may be prompted that the blood pressure measurement mode is enabled.

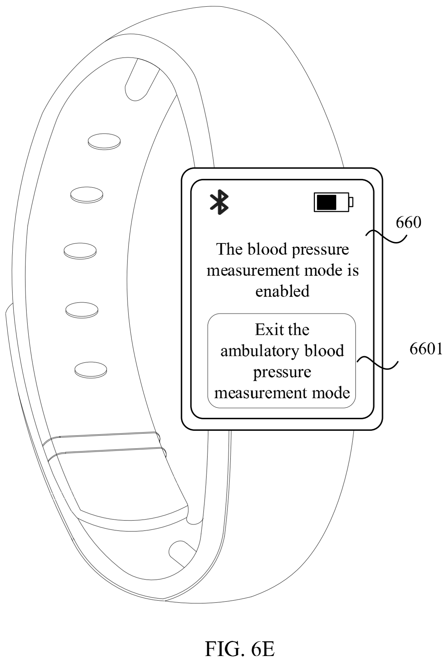

In some embodiments, after the wearable device 100 enables the blood pressure measurement mode, the wearable device 100 may display a user interface 660 shown in FIG. 6E. The user interface 660 may include prompt information "The blood pressure measurement mode is enabled". The prompt information is used to prompt the user that the wearable device 100 has enabled the blood pressure measurement mode. The user interface 660 further includes an option 6601 of "Exit the blood pressure measurement mode". The user may enable the wearable device 100 to disable the blood pressure measurement mode by using the option 6601 of "Exit the blood pressure measurement mode".

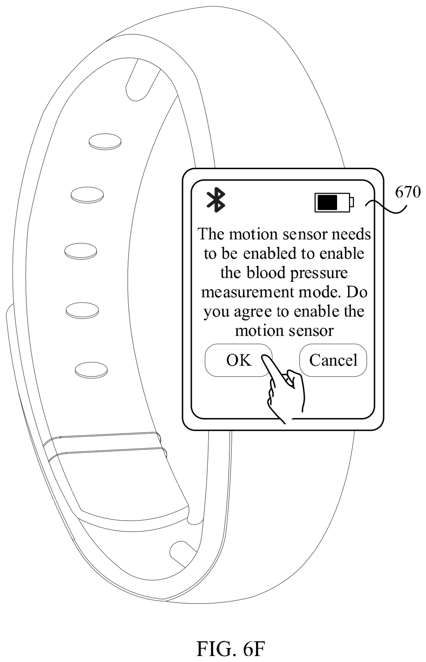

In some embodiments, after the wearable device 100 enables the blood pressure measurement mode, the wearable device 100 may detect whether a motion sensor in the wearable device 100 is enabled, to ensure accuracy of a blood pressure monitoring result. When the wearable device 100 detects that the motion sensor in the wearable device 100 is not enabled, the wearable device 100 may display a user interface 670 shown in FIG. 6F. The user interface 670 includes prompt information "The motion sensor needs to be enabled to enable the blood pressure measurement mode. Do you agree to enable the motion sensor", and the prompt information is used to prompt the user to enable the motion sensor in the wearable device 100. The user interface 670 further includes an "OK" option and a "Cancel" option. The user may enable the motion sensor in the wearable device 100 by using the "OK" option, or the user may not enable the motion sensor in the wearable device 100 by using the "Cancel" option.

For example, as shown in FIG. 6F, the wearable device 100 may receive an input operation (for example, a tap) performed by the user on the "OK" option in the user interface 670, and in response to the input operation performed by the user, the wearable device 100 may enable the motion sensor in the wearable device 100.

In another embodiment, after the wearable device 100 enables the blood pressure measurement mode, when the wearable device 100 detects that the motion sensor in the wearable device 100 is enabled, the wearable device 100 may not display the prompt information shown in FIG. 6F.

In some embodiments, the wearable device 100 may collect statistics on a blood pressure measurement value of a same user in a period of time, analyze the blood pressure measurement value of the user in the period of time, and provide a targeted opinion on the blood pressure measurement value of the same user.

After the wearable device 100 enables the blood pressure measurement mode, and before the wearable device 100 starts to measure the user blood pressure, the wearable device 100 needs to confirm a user identity. In this way, blood pressure measurement values of different users may be separately stored, to avoid a problem that the wearable device 100 subsequently provides an inaccurate targeted opinion on the blood pressure measurement value of the same user because the blood pressure measurement values of the different users are mixed together.

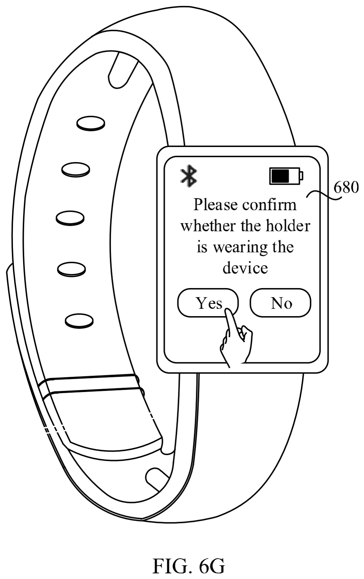

FIG. 6G to FIG. 6M are diagrams in which a wearable device 100 confirms a user identity.

Optionally, when the user wears the wearable device 100 a first time, the wearable device 100 may confirm the user identity. That the user wears the wearable device 100 the first time may mean that after being removed from a wrist by the user, the wearable device 100 is worn on the wrist of the user again, or is worn on the wrist of the user after a specific period of time.

For example, after the wearable device 100 enables the blood pressure measurement mode, the wearable device 100 may display a user interface 680 shown in FIG. 6G. The user interface 680 includes a prompt message "Please confirm whether the holder is wearing the device", and the prompt information is used to prompt the user to confirm an identity of a user who wears the wearable device 100. The holder may be an owner of the wearable device 100, and the owner of the wearable device 100 is a user who wears the wearable device 100 a long period of time. The user interface 680 further includes a "Yes" option and a "No" option. The "Yes" option is used to confirm that the wearable device 100 is worn by the holder currently. The "No" option is used to confirm that the wearable device 100 is not worn by the holder currently.

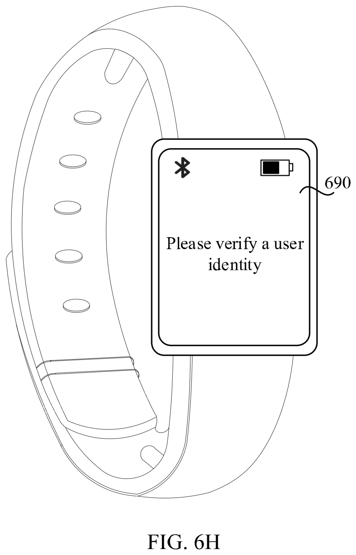

In a possible implementation, the wearable device 100 may receive an input operation (for example, a tap) performed by the user on the "Yes" option in the user interface 680, and in response to the input operation performed by the user, the wearable device 100 may display a user interface 690 shown in FIG. 6H. The user interface 690 includes prompt information "Please verify a user identity", and the prompt information is used to prompt the user to verify whether a current user is the holder, to avoid a misoperation.

An identity verification manner includes but is not limited to facial recognition, fingerprint recognition, voiceprint recognition, and the like. Identity verification may alternatively be performed in another manner. This is not limited in this application.

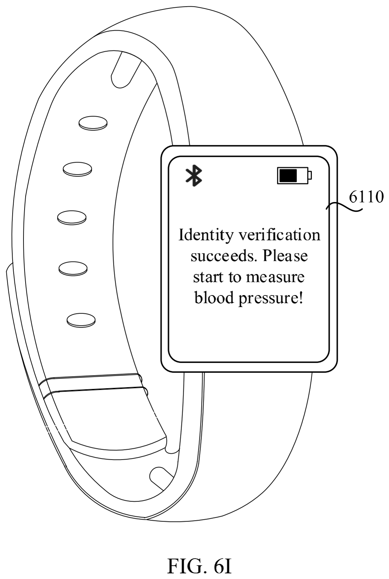

When identity verification succeeds, that is, it is determined that the owner of the wearable device 100 is wearing the device, the wearable device 100 may display a user interface 6110 shown in FIG. 6I. The user interface 6110 includes prompt information "Identity verification succeeds. Please start to measure blood pressure!", and the prompt information indicates, to the user, that the owner of the wearable device 100 is wearing the device.

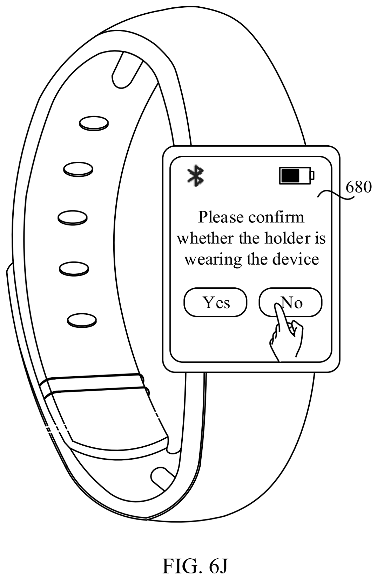

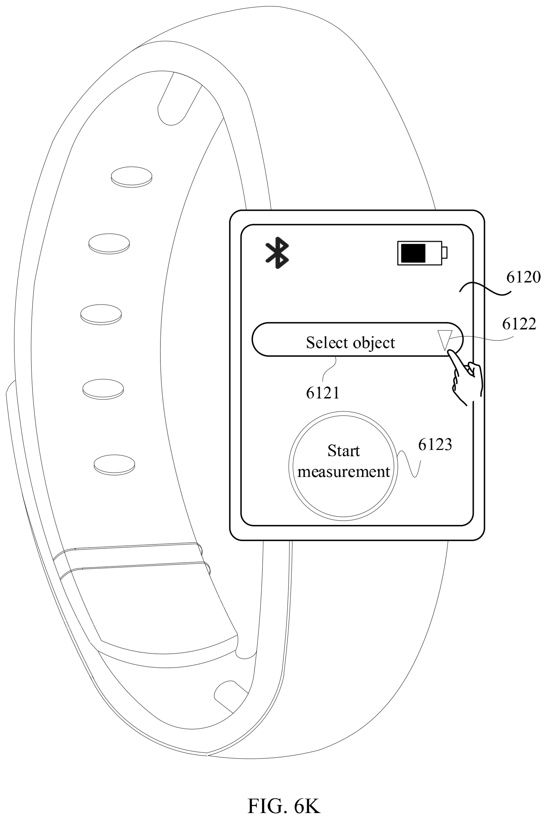

In another possible implementation, as shown in FIG. 6J, the wearable device 100 may receive an input operation (for example, a tap) performed by the user on a "No" option in the user interface 680, and in response to the input operation performed by the user, the wearable device 100 may display a user interface 6120 shown in FIG. 6K. The user interface 690 includes a selection bar 6121, and the selection bar 6121 is used by the user to select a user name.

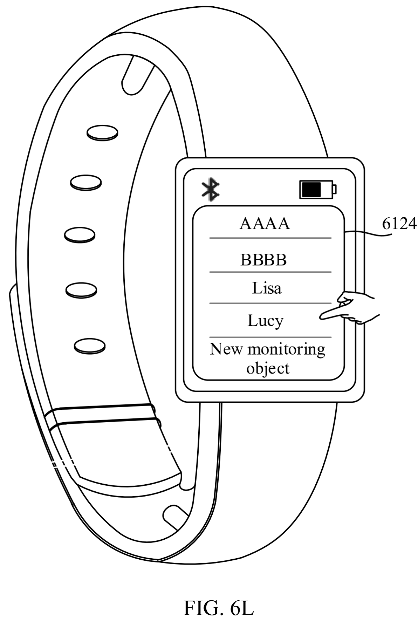

As shown in FIG. 6K, the wearable device 100 may receive an input operation (for example, a tap) performed by the user on an option 6122 in the selection bar 6121, and in response to the input operation performed by the user, the wearable device 100 may display a selection bar 6124 shown in FIG. 6L. Options of a plurality of monitoring objects are shown in the selection bar 6124. For example, the plurality of monitoring objects include but are not limited to a monitoring object "AAAA", a monitoring object "BBBB", a monitoring object "Lisa", and a monitoring object "Lucy". The user may select any monitoring object, and then start to measure the blood pressure; and bind current monitoring data and a currently selected monitoring object for storage, to avoid mixing blood pressure measurement results of different users.

Optionally, if the selection bar 6124 does not include an option of a user who needs to be detected currently, the wearable device 100 may receive an input operation performed by the user on an option of "New monitoring object" in the selection bar 6123, and add the option of the user who needs to be detected currently.

In this way, monitoring data of different monitoring objects may be stored separately, to facilitate subsequent view of the monitoring data of the different monitoring objects in specific duration.

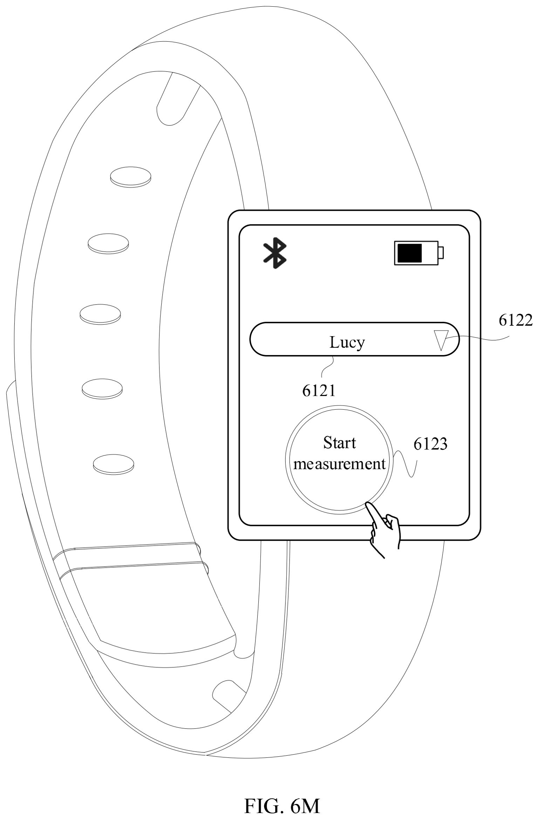

For example, as shown in FIG. 6L, the wearable device 100 may receive an input operation (for example, a tap) performed by the user on the option of the monitoring object "Lucy" in the selection bar 6124, and in response to the input operation performed by the user, the wearable device 100 may determine that a current monitoring object is "Lucy", and the wearable device 100 may display, in the selection bar 6121, a "Lucy" identifier shown in FIG. 6M.

Then, the wearable device 100 may receive an input operation (for example, a tap) performed by the user on a "Start measurement" option, and in response to the input operation performed by the user, may start to measure the blood pressure.

Then, the electronic device 200 may bind a blood pressure measurement value of the monitoring object "Lucy" and the monitoring object "Lucy" for storage. Specifically, the electronic device 200 may find a storage area of the monitoring object "Lucy", and store the blood pressure measurement value of the monitoring object "Lucy" in the storage area of the monitoring object "Lucy". It should be noted that storage areas of different monitoring objects are different and are isolated from each other, to avoid mixing blood pressure measurement results of different users.

In another embodiment, as shown in FIG. 6J, after the wearable device 100 receives an input operation (for example, a tap) performed by the user on the "No" option in the user interface 680, the wearable device 100 may start to measure the user blood pressure, but does not store a blood pressure measurement value of the current user and a blood pressure measurement value of a previous user together, or the wearable device 100 may not store a blood pressure measurement value of the current user.

### Start to measure the blood pressure

**That the wearable device 100 measures the blood pressure includes three stages: a blood pressure measurement reminder stage, identifying the user posture and determining a blood pressure compensation value based on the user posture, and obtaining a blood pressure monitoring value based on the blood pressure compensation value and the blood pressure measurement value.**

### I. Blood pressure measurement reminder stage

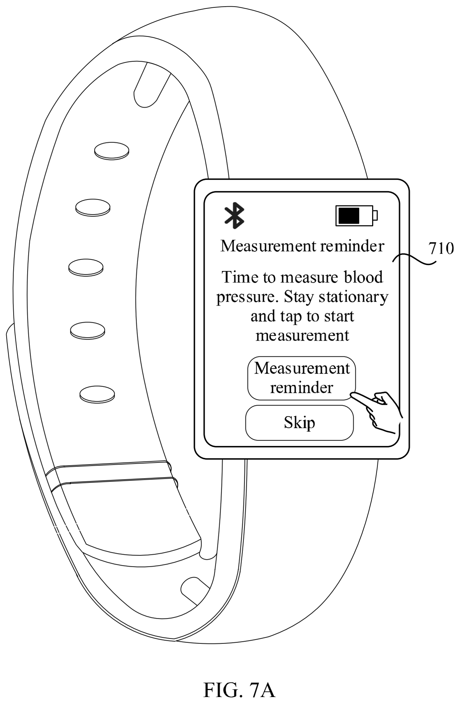

**FIG. 7A to FIG. 7C** **are diagrams in which a wearable device 100 prompts a user to measure blood pressure.**

For example, as shown in FIG. 7A, when the wearable device 100 starts to measure the blood pressure, to ensure accuracy of a blood pressure measurement result, the wearable device 100 may display a user interface 710 shown in FIG. 7A. The user interface 710 shows prompt information "Time to measure blood pressure. Stay stationary and tap to start measurement". The prompt information is used to prompt the user to remain in a stationary state during blood pressure measurement, to avoid an inaccurate blood pressure measurement result caused by motion. The user interface 710 further includes a "Measurement reminder" option and a "Skip" option. The user may view precautions for blood pressure measurement by using the "Measurement reminder" option. Alternatively, the user may directly start to measure the blood pressure without viewing the precautions for blood pressure measurement by using the "Skip" option.

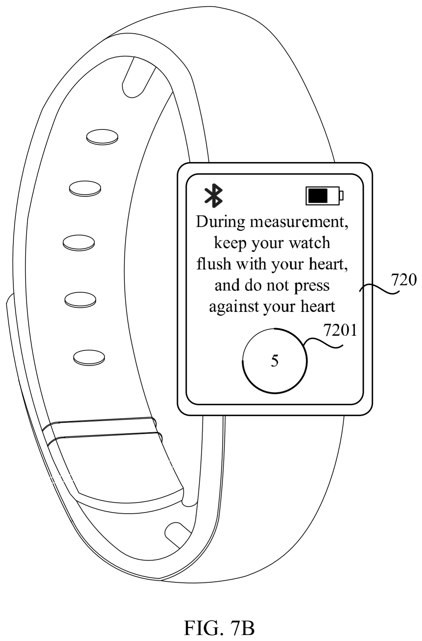

For example, as shown in FIG. 7A, the wearable device 100 may receive an input operation (for example, a tap) performed by the user on the "Measurement reminder" option in the user interface 710, and in response to the input operation performed by the user, the wearable device 100 may display a user interface 720 shown in FIG. 7B. The user interface 720 includes prompt information: "During measurement, keep your watch flush with your heart, and do not press against your heart", and the prompt information is used to prompt the user with a correct measurement posture. The user interface 720 includes a "Timer" option 7201. The "Timer" option 7201 is used to prompt the user to raise the watch to a location flush with the heart within preset time.

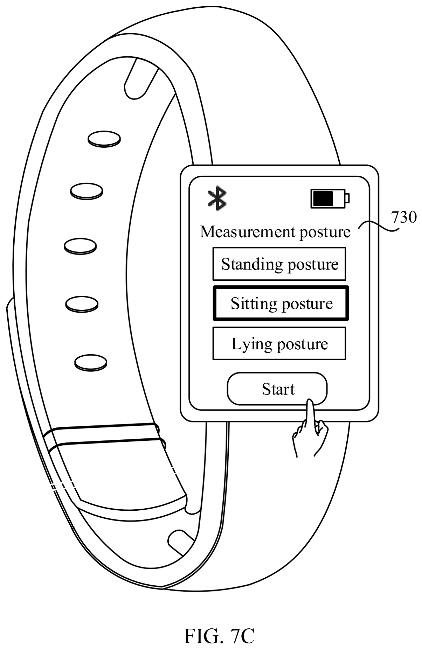

In some embodiments, the wearable device 100 may determine the user posture based on the motion data collected by the motion sensor, and display a user interface 730 shown in FIG. 7C. The user interface 730 includes a plurality of options such as a "Standing posture" option, a "Sitting posture" option, and a "Lying posture" option. The plurality of options are used by the user to confirm a current user posture. For example, when the wearable device 100 identifies that the user is in a sitting posture, the wearable device 100 may deepen the "Sitting posture" option for display, to prompt the user that the wearable device 100 identifies that the user is in the sitting posture. Optionally, in addition to deepening display, the wearable device 100 may further prompt, in another manner, the user that the wearable device 100 identifies that the user is in the sitting posture. This is not limited in this application.

For example, as shown in FIG. 7B, after the user confirms that the user is in the sitting posture, the wearable device 100 may receive an input operation (for example, a tap) performed by the user on a "Start" option in the user interface 730, and in response to the input operation performed by the user, the wearable device 100 may start to measure the user blood pressure.

In some embodiments, if the user posture that is identified by the wearable device 100 and that is displayed in the user interface 730 is incorrect, for example, a standing posture is identified as the sitting posture, the user may reselect the "Standing posture" option, and then tap the "Start" option to start to measure the user blood pressure. This can prevent the wearable device 100 from identifying an incorrect user posture.

In some embodiments, the wearable device 100 may alternatively not display the prompt information shown in FIG. 7A to FIG. 7C, but directly start to measure the user blood pressure, to avoid frequently disturbing the user. For example, at night, the wearable device 100 may not display the prompt information shown in FIG. 7A to FIG. 7C, and the wearable device 100 may automatically measure the user blood pressure without displaying the prompt information, to avoid disturbing rest of the user.

### II. Identify the user posture

**Different user postures exert different impact on the blood pressure measurement value collected by the wearable device 100. Therefore, the wearable device 100 needs to identify the user posture, and correct, based on different user postures, the blood pressure measurement value collected by the wearable device 100, to improve accuracy of a blood pressure value collected by the wearable device 100.**

**The following describes how the wearable device 100 identifies the user posture.**

**The user posture includes but is not limited to a standing posture, a sitting posture, a lying posture, and the like.**

### 1. Identify the standing posture of the user.

### (1) The wearable device 100 is worn on a left-hand wrist of the user.

The wearable device 100 may determine the standing posture of the user based on but not limited to any one or more of the following manners.

**Manner 1:** The wearable device 100 may determine the standing posture of the user based on a heart rate and/or the motion data collected by the wearable device 100.

In some embodiments, the wearable device 100 may collect a heart rate of the user by using a pre-configured PPG sensor. When the user naturally walks or runs, the heart rate of the user is high. If the heart rate is greater than a first heart rate value, it may be determined that the user posture is the standing posture.

Optionally, the wearable device 100 may determine the user posture based on a plurality of groups of heart rate values within first duration before blood pressure measurement starts. If the plurality of groups of heart rate values within the first duration before the blood pressure measurement starts are greater than the first heart rate value, it may be determined that the user posture is the standing posture.

In some embodiments, the wearable device 100 may alternatively collect motion data, for example, information such as a step count, of the user by using the pre-configured motion sensor. When the user naturally walks or runs, the step count of the user increases accumulatively. If a change value of a step count within first duration before blood pressure measurement starts is greater than a first value, it may be determined that the user posture is the standing posture.

**Manner 2:** The wearable device 100 may determine the standing posture of the user based on an acceleration component of gravitational acceleration G on a Z axis.

When the user stands, the Z axis is always perpendicular to the gravitational acceleration G, and the standing posture of the user may be determined based on the acceleration component of the gravitational acceleration G on the Z axis.

Specifically, when the wearable device 100 determines that the acceleration component of the gravitational acceleration G on the Z axis is close to a minimum value, the standing posture of the user may be determined.

**Manner 3:** The wearable device 100 may obtain a motion posture of the wearable device 100, and determine the standing posture of the user based on the motion posture of the wearable device 100.

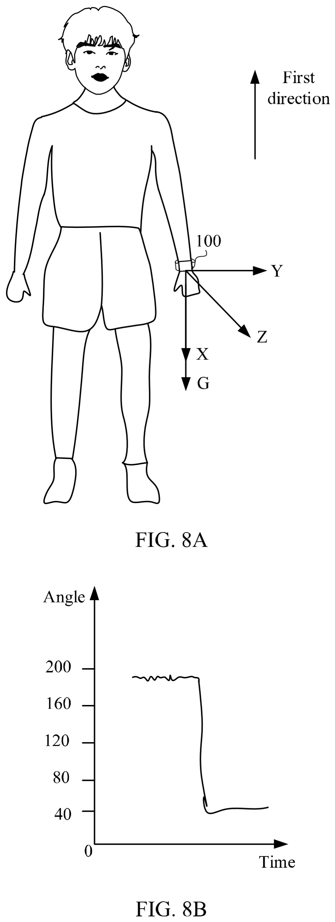

For example, FIG. 8A is a diagram in which a user wears a wearable device 100 when standing.

As shown in FIG. 8A, a direction that is along a forearm of the user and that is parallel to a user finger is a positive direction of an X axis. A direction that is perpendicular to the positive direction of the X axis and that is away from a user body is a positive direction of a Y axis. The X axis and the Y axis may determine an X-Y plane. A direction that is perpendicular to the X-Y plane and that is away from a wearable component is a positive direction of a Z axis.

A positive direction of gravitational acceleration G is always perpendicular to the ground. A direction that is parallel to the positive direction of the gravitational acceleration G and that is opposite to the positive direction of the gravitational acceleration G is a first direction.

The wearable device 100 may determine the standing posture of the user based on a change in an included angle between the X axis and the first direction.

In some embodiments, before the wearable device 100 starts to measure the blood pressure, the wearable device 100 may display the prompt information shown in FIG. 7B, to prompt the user to make the wearable device 100 flush with the heart. In this case, in a process in which the user raises the wearable device 100 to be flush with the heart, the change in the included angle between the X axis and the first direction may be an angle change shown in FIG. 8B.

As shown in FIG. 8B, when the user naturally walks or runs, the forearm is naturally vertical. As shown in FIG. 8B. The included angle between the positive direction of the X axis and the first direction changes approximately at 180 degrees. In a process in which the user raises the wearable device 100 to be flush with the heart, the included angle between the positive direction of the X axis and the first direction decreases sharply, for example, decreases from 180 degrees to approximately 40 degrees. After the user makes the wearable device 100 be flush with the heart, the included angle between the positive direction of the X axis and the first direction is stable at approximately 40 degrees.

The motion posture of the wearable device 100 may be a change value of the included angle between the positive direction of the X axis and the first direction. The standing posture of the user may be determined based on the change value of the included angle between the positive direction of the X axis and the first direction. Specifically, if it is detected, before the blood pressure measurement starts, that a change value of an included angle between the positive direction of the X axis and the first direction within second duration is greater than a first angle value, the standing posture of the user may be determined. For example, the first angle value may be 100 degrees. The first angle value may alternatively be another value. This is not limited in this application either.

In addition to the foregoing three manners, the wearable device 100 may determine the standing posture of the user in another manner. This is not limited in this application either.

After determining the standing posture of the user, the wearable device 100 may determine a blood pressure compensation value corresponding to the standing posture of the user based on the standing posture of the user, and correct, based on the blood pressure compensation value corresponding to the standing posture of the user, the blood pressure measurement value collected by the wearable device 100.

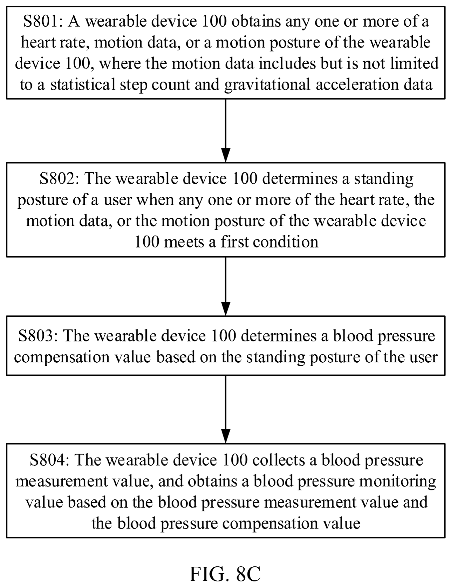

FIG. 8C is a schematic flowchart of a method for correcting a collected blood pressure measurement value by a wearable device 100.

S801: The wearable device 100 obtains any one or more of the heart rate, the motion data, or the motion posture of the wearable device 100, where the motion data includes but is not limited to a statistical step count and gravitational acceleration data.

S802: The wearable device 100 determines the standing posture of the user when any one or more of the heart rate, the motion data, or the motion posture of the wearable device 100 meets a first condition.

The heart rate may be collected by the PPG sensor pre-configured in the wearable device 100, or the heart rate may be collected by the electronic device 200 and then sent to the wearable device 100. This is not limited in this application.

The motion data and the motion posture of the wearable device 100 may be collected by an inertial sensor pre-configured in the wearable device 100.

The wearable device 100 determines the standing posture of the user when any one or more of the heart rate, the motion data, or the motion posture of the wearable device 100 meets the first condition.

The first condition may include but is not limited to any one or more of the following:
1. The plurality of groups of heart rate values within the first duration before the blood pressure measurement starts are greater than the first heart rate value.
2. It is detected, before the blood pressure measurement starts, that the change value of the included angle between the positive direction of the X axis and the first direction within the second duration is greater than the first angle value.
3. The acceleration component of the gravitational acceleration G on the Z axis is close to the minimum value.

For how the wearable device 100 determines the standing posture of the user, refer to the descriptions in Manner 1 to Manner 3. Details are not described herein again in this application.

For how the wearable device 100 determines the standing posture of the user, refer to the descriptions in Manner 1 to Manner 3. Details are not described herein again in this application.

S803: The wearable device 100 determines the blood pressure compensation value based on the standing posture of the user.

It should be noted that different blood pressure compensation values are determined based on different user postures. For example, the standing posture, the sitting posture, and the lying posture correspond to different blood pressure compensation values.

After determining the standing posture of the user, the wearable device 100 may determine the blood pressure compensation value corresponding to the standing posture of the user based on the standing posture of the user.

In a possible implementation, the wearable device 100 locally stores blood pressure compensation values corresponding to a plurality of different user postures. The wearable device 100 may locally determine the blood pressure compensation value corresponding to the standing posture of the user based on the standing posture of the user.

In another possible implementation, the wearable device 100 may send the standing posture of the user to the electronic device 200 that establishes a communication connection to the wearable device 100. The electronic device 200 locally stores the blood pressure compensation values corresponding to the plurality of different user postures. The electronic device 200 may locally determine the blood pressure compensation value corresponding to the standing posture of the user based on the standing posture of the user. Then, the electronic device 200 sends the blood pressure compensation value corresponding to the standing posture of the user to the wearable device 100.

In another possible implementation, the wearable device 100 may send the standing posture of the user to a server. The server locally stores the blood pressure compensation values corresponding to the plurality of different user postures. The server may locally determine the blood pressure compensation value corresponding to the standing posture of the user based on the standing posture of the user. Then, the server sends the blood pressure compensation value corresponding to the standing posture of the user to the wearable device 100.

Optionally, the blood pressure compensation values corresponding to the different user postures may be periodically/aperiodically updated.

S804: The wearable device 100 collects the blood pressure measurement value, and obtains the blood pressure monitoring value based on the blood pressure measurement value and the blood pressure compensation value.

The wearable device 100 may collect the blood pressure measurement value, and obtain the blood pressure monitoring value based on the blood pressure measurement value and the blood pressure compensation value. The blood pressure monitoring value is a blood pressure value finally obtained through measurement.

In this way, when the user blood pressure is measured, impact of different user postures on the blood pressure measurement result can be eliminated, to improve accuracy of the blood pressure measurement result.

In some embodiments, when the wearable device 100 identifies the standing posture of the user, placing the wrist wearing the wearable device 100 at different locations also affects the blood pressure measurement value collected by the wearable device 100.

Based on this, to further improve accuracy of the blood pressure measurement value collected by the wearable device 100, the wearable device 100 further needs to determine a location of the wrist wearing the wearable device 100, determine the blood pressure compensation value based on the location of the wrist wearing the wearable device 100, and then obtain a final blood pressure monitoring value based on the blood pressure measurement value collected by the wearable device 100 and the blood pressure compensation value. In other words, in the standing posture of the user, the blood pressure compensation value varies with the location of the wrist wearing the wearable device 100.

In a possible implementation, the blood pressure compensation value may be determined based on the included angle between the positive direction of the X axis of the wearable device 100 and the first direction.

In a possible implementation, in the standing posture, the wearable device 100 locally stores blood pressure compensation values corresponding to a plurality of angles between the positive direction of the X axis and the first direction. The wearable device 100 may locally determine a blood pressure compensation value corresponding to a first angle based on the angle between the positive direction of the X axis and the first direction.

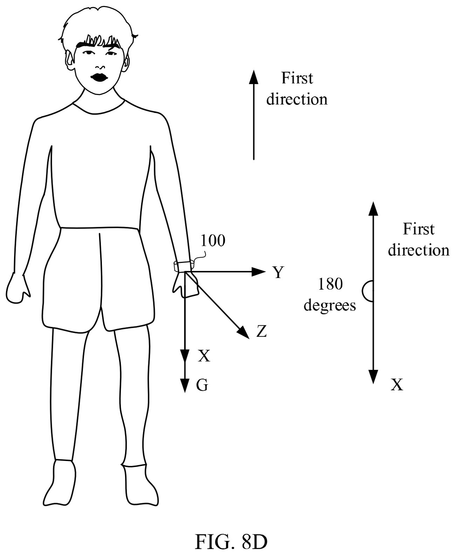

FIG. 8D to FIG. 8F are a group of diagrams in which a wrist wearing a wearable device 100 is located at different placement locations when a user is in a standing posture.

FIG. 8D is a diagram in which an arm is placed vertically pointing to a ground when a user is in a standing posture.

As shown in FIG. 8D, the positive direction of the X axis is opposite to the first direction, and the included angle between the positive direction of the X axis and the first direction is close to 180 degrees.

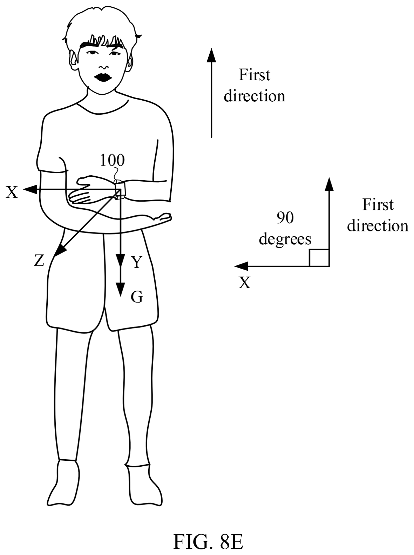

FIG. 8E is a diagram of horizontally placing an arm when a user is in a standing posture.

As shown in FIG. 8E, the included angle between the positive direction of the X axis and the first direction is close to 90 degrees.

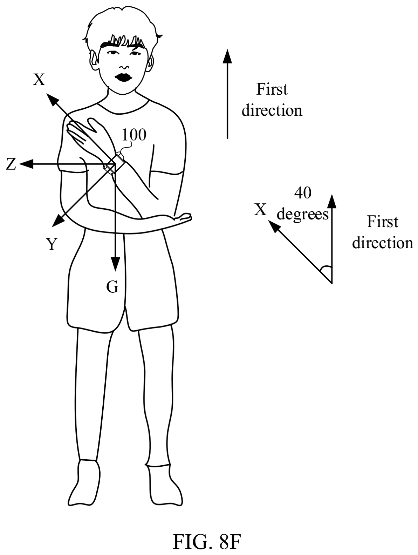

FIG. 8F is a diagram in which a wrist wearing a wearable device 100 is placed at a location of a heart when a user is in a standing posture.

As shown in FIG. 8F, the included angle between the positive direction of the X axis and the first direction is close to 40 degrees.

**Table 1**

| Included angle between a positive direction of an X axis and a first direction | Blood pressure compensation value |
|---|---|
| 180 degrees to 140 degrees | A |
| 139 degrees to 90 degrees | B |
| 89 degrees to 40 degrees | C |
| 39 degrees to 0 degrees | D |

Table 1 shows the blood pressure compensation value corresponding to the angle between the positive direction of the X axis and the first direction. As shown in Table 1, when the included angle between the positive direction of the X axis and the first direction is between 180 degrees and 140 degrees, the blood pressure compensation value is A. When the included angle between the positive direction of the X axis and the first direction is between 139 degrees and 90 degrees, the blood pressure compensation value is B. When the included angle between the positive direction of the X axis and the first direction is between 89 degrees and 40 degrees, the blood pressure compensation value is C. When the included angle between the positive direction of the X axis and the first direction is between 39 degrees and 0 degrees, the blood pressure compensation value is D.

The blood pressure compensation value A, the blood pressure compensation value B, the blood pressure compensation value C, and the blood pressure compensation value D are different from each other.

For example, when the wearable device 100 determines that the included angle between the positive direction of the X axis and the first direction is 160 degrees, the wearable device 100 may determine that the blood pressure compensation value is A.

For another example, when the wearable device 100 determines that the included angle between the positive direction of the X axis and the first direction is 40 degrees, the wearable device 100 may determine that the blood pressure compensation value is C.

Table 1 merely describes the blood pressure compensation value corresponding to the angle between the positive direction of the X axis and the first direction as an example. Alternatively, each angle may correspond to one blood pressure compensation value. This is not limited in this application either.

In another possible implementation, the wearable device 100 may send the first angle between the positive direction of the X axis and the first direction in the standing posture of the user to the electronic device 200 that establishes a communication connection to the wearable device 100. The electronic device 200 locally stores blood pressure compensation values corresponding to a plurality of angles between the positive direction of the X axis and the first direction in the standing posture of the user. The electronic device 200 may locally determine the blood pressure compensation value based on the first angle between the positive direction of the X axis and the first direction in the standing posture of the user. Then, the electronic device 200 sends the determined blood pressure compensation value to the wearable device 100.

In another possible implementation, the wearable device 100 may send the first angle between the positive direction of the X axis and the first direction in the standing posture of the user to the server. The server locally stores the blood pressure compensation values corresponding to the plurality of angles between the positive direction of the X axis and the first direction in the standing posture of the user. The server may locally determine the blood pressure compensation value based on the first angle between the positive direction of the X axis and the first direction in the standing posture of the user. Then, the server sends the determined blood pressure compensation value to the wearable device 100.

In another embodiment, the wearable device 100 may determine a distance between the wearable device 100 and the heart in a vertical direction based on the included angle between the X axis of the wearable device 100 and the first direction, and then determine the blood pressure compensation value based on the distance between the wearable device 100 and the heart in the vertical direction.

In a possible implementation, the distance between the wearable device 100 and the heart in the vertical direction may be determined by the wearable device 100 based on the included angle between the positive direction of the X axis of the wearable device 100 and the first direction; or may be determined, by the electronic device 2000 that establishes a communication connection to the wearable device 100, based on the included angle between the positive direction of the X axis of the wearable device 100 and the first direction; may also be determined by the server based on the included angle between the positive direction of the X axis of the wearable device 100 and the first direction.

**Table 2**

| Included angle between a positive direction of an X axis and a first direction | Distance between a wearable device 100 and a heart in a vertical direction | Blood pressure compensation value |
|---|---|---|
| 180 degrees to 140 degrees | a | A |
| 139 degrees to 90 degrees | b | B |
| 89 degrees to 40 degrees | c | C |
| 39 degrees to 0 degrees | d | D |

Table 2 shows the blood pressure compensation value corresponding to the angle between the positive direction of the X axis and the first direction. As shown in Table 2, when the included angle between the positive direction of the X axis and the first direction is between 180 degrees and 140 degrees, the distance between the wearable device 100 and the heart in the vertical direction is a, and the blood pressure compensation value is A. When the included angle between the positive direction of the X axis and the first direction is between 139 degrees and 90 degrees, the distance between the wearable device 100 and the heart in the vertical direction is b, and the blood pressure compensation value is B. When the included angle between the positive direction of the X axis and the first direction is between 89 degrees and 40 degrees, the distance between the wearable device 100 and the heart in the vertical direction is c, and the blood pressure compensation value is C. When the included angle between the positive direction of the X axis and the first direction is between 39 degrees and 0 degrees, the distance between the wearable device 100 and the heart in the vertical direction is d, and the blood pressure compensation value is D.

The blood pressure compensation value A, the blood pressure compensation value B, the blood pressure compensation value C, and the blood pressure compensation value D are different from each other.

The blood pressure compensation value corresponding to the distance between the wearable device 100 and the heart in the vertical direction in Table 2 may be stored in the wearable device 100, or may be stored in the electronic device 200, or may be stored in the server. This is not limited in this application.

### (2) The wearable device 100 is worn on a right-hand wrist of the user.

For a specific implementation of how to identify the standing posture of the user when the wearable device 100 is worn on the right-hand wrist of the user, refer to descriptions of a specific implementation of how to identify the standing posture of the user when the wearable device 100 is worn on the left-hand wrist of the user. Details are not described herein again in this application.

A difference lies in that the positive direction of the X axis, the positive direction of the Y axis, and the positive direction of the Z axis when the wearable device 100 is worn on the right-hand wrist of the user are different from the positive direction of the X axis, the positive direction of the Y axis, and the positive direction of the Z axis when the wearable device 100 is worn on the left-hand wrist of the user.

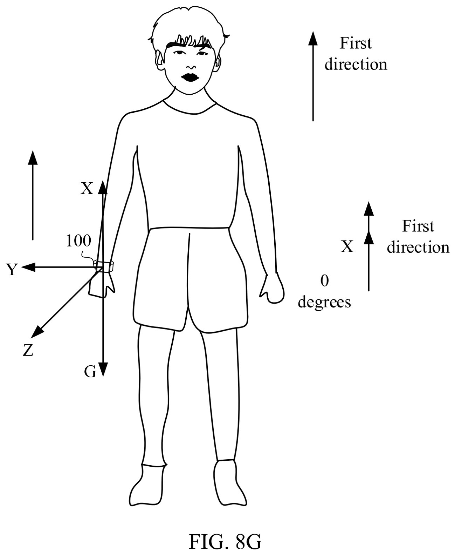

FIG. 8G to FIG. 8I are another group of diagrams in which a wrist wearing a wearable device 100 is located at different placement locations when a user is in a standing posture.

FIG. 8G is a diagram in which an arm is placed vertically pointing to a ground when a user is in a standing posture.

As shown in FIG. 8G, the positive direction of the X axis is the same as the first direction, and the included angle between the positive direction of the X axis and the first direction is close to 0 degrees.

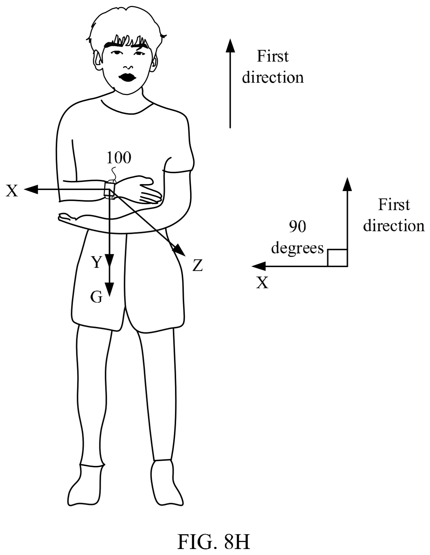

FIG. 8H is a diagram of horizontally placing an arm when a user is in a standing posture.

As shown in FIG. 8H, the included angle between the positive direction of the X axis and the first direction is close to 90 degrees.

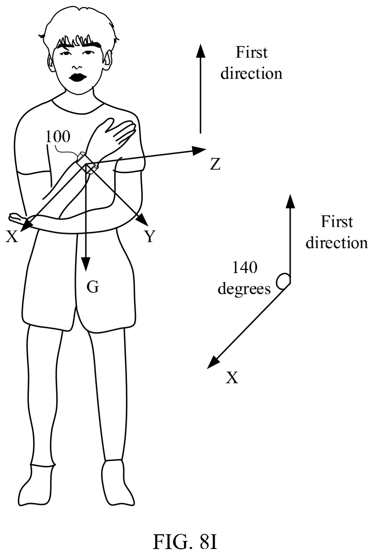

FIG. 8I is a diagram in which a wrist wearing a wearable device 100 is placed at a location of a heart when a user is in a standing posture.

As shown in FIG. 8I, the included angle between the positive direction of the X axis and the first direction is close to 140 degrees.

**Table 3**

| Included angle between a positive direction of an X axis and a first direction | Blood pressure compensation value |
|---|---|
| 180 degrees to 140 degrees | E |
| 139 degrees to 90 degrees | F |
| 89 degrees to 40 degrees | G |
| 39 degrees to 0 degrees | H |

Table 3 shows the blood pressure compensation value corresponding to the angle between the positive direction of the X axis and the first direction. As shown in Table 3, when the included angle between the positive direction of the X axis and the first direction is between 180 degrees and 140 degrees, the blood pressure compensation value is E. When the included angle between the positive direction of the X axis and the first direction is between 139 degrees and 90 degrees, the blood pressure compensation value is F. When the included angle between the positive direction of the X axis and the first direction is between 89 degrees and 40 degrees, the blood pressure compensation value is G. When the included angle between the positive direction of the X axis and the first direction is between 39 degrees and 0 degrees, the blood pressure compensation value is H.

The blood pressure compensation value A, the blood pressure compensation value B, the blood pressure compensation value C, and the blood pressure compensation value D are different from each other.

For example, when the wearable device 100 determines that the included angle between the positive direction of the X axis and the first direction is 160 degrees, the wearable device 100 may determine that the blood pressure compensation value is E.

For another example, when the wearable device 100 determines that the included angle between the positive direction of the X axis and the first direction is 40 degrees, the wearable device 100 may determine that the blood pressure compensation value is G.

Table 3 merely describes the blood pressure compensation value corresponding to the angle between the positive direction of the X axis and the first direction as an example. Alternatively, each angle may correspond to one blood pressure compensation value. This is not limited in this application either.

In another possible implementation, the wearable device 100 may send the first angle between the positive direction of the X axis and the first direction in the standing posture of the user to the electronic device 200 that establishes a communication connection to the wearable device 100. The electronic device 200 locally stores blood pressure compensation values corresponding to a plurality of angles between the positive direction of the X axis and the first direction in the standing posture of the user. The electronic device 200 may locally determine the blood pressure compensation value based on the first angle between the positive direction of the X axis and the first direction in the standing posture of the user. Then, the electronic device 200 sends the determined blood pressure compensation value to the wearable device 100.

In another possible implementation, the wearable device 100 may send the first angle between the positive direction of the X axis and the first direction in the standing posture of the user to the server. The server locally stores the blood pressure compensation values corresponding to the plurality of angles between the positive direction of the X axis and the first direction in the standing posture of the user. The server may locally determine the blood pressure compensation value based on the first angle between the positive direction of the X axis and the first direction in the standing posture of the user. Then, the server sends the determined blood pressure compensation value to the wearable device 100.

In another embodiment, the wearable device 100 may determine a distance between the wearable device 100 and the heart in a vertical direction based on the included angle between the X axis of the wearable device 100 and the first direction, and then determine the blood pressure compensation value based on the distance between the wearable device 100 and the heart in the vertical direction.

In a possible implementation, the distance between the wearable device 100 and the heart in the vertical direction may be determined by the wearable device 100 based on the included angle between the positive direction of the X axis of the wearable device 100 and the first direction; or may be determined, by the electronic device 2000 that establishes a communication connection to the wearable device 100, based on the included angle between the positive direction of the X axis of the wearable device 100 and the first direction; may also be determined by the server based on the included angle between the positive direction of the X axis of the wearable device 100 and the first direction.

**Table 4**

| Included angle between a positive direction of an X axis and a first direction | Distance between a wearable device 100 and a heart in a vertical direction | Blood pressure compensation value |
|---|---|---|
| 180 degrees to 140 degrees | e | E |
| 139 degrees to 90 degrees | f | F |
| 89 degrees to 40 degrees | g | G |
| 39 degrees to 0 degrees | h | H |

Table 4 shows the blood pressure compensation value corresponding to the angle between the positive direction of the X axis and the first direction. As shown in Table 4, when the included angle between the positive direction of the X axis and the first direction is between 180 degrees and 140 degrees, the distance between the wearable device 100 and the heart in the vertical direction is e, and the blood pressure compensation value is E. When the included angle between the positive direction of the X axis and the first direction is between 139 degrees and 90 degrees, the distance between the wearable device 100 and the heart in the vertical direction is f, and the blood pressure compensation value is F. When the included angle between the positive direction of the X axis and the first direction is between 89 degrees and 40 degrees, the distance between the wearable device 100 and the heart in the vertical direction is g, and the blood pressure compensation value is G. When the included angle between the positive direction of the X axis and the first direction is between 39 degrees and 0 degrees, the distance between the wearable device 100 and the heart in the vertical direction is h, and the blood pressure compensation value is H.

The distance e, the distance f, the distance g, and the distance h are different from each other.

The blood pressure compensation value E, the blood pressure compensation value F, the blood pressure compensation value G, and the blood pressure compensation value H are also different from each other.

The blood pressure compensation value corresponding to the distance between the wearable device 100 and the heart in the vertical direction in Table 4 may be stored in the wearable device 100, or may be stored in the electronic device 200, or may be stored in the server. This is not limited in this application.

In the method, the wearable device 100 may identify the standing posture of the user, determine the blood pressure compensation value based on the standing posture of the user, and correct, based on the blood pressure compensation value, the blood pressure measurement value collected by the wearable device 100.

In some embodiments, after identifying the standing posture of the user, the wearable device 100 may determine the blood pressure compensation value based on the angle between the positive direction of the X axis of the wearable device 100 and the first direction. Because the wearable device 100 is worn at different locations, angles between the positive direction of the X axis and the first direction are different, and blood pressure compensation values are also different. In this way, this can further improve accuracy of the blood pressure measurement value collected by the wearable device 100 in the standing posture of the user.

In some embodiments, the wearable device 100 may further identify whether the wearable device 100 is worn on a left hand or a right hand, and then determine the blood pressure compensation value based on the angle between the positive direction of the X axis of the wearable device 100 and the first direction. Because the wearable device 100 is worn on the left hand or the right hand, when the wearable device 100 is located at a same location, angles between the positive direction of the X axis and the first direction are different. Therefore, whether the wearable device 100 is worn on the left hand or on the right hand needs to be distinguished. After determining whether the wearable device 100 is worn on the left hand or the right hand, the wearable device 100 determines the blood pressure compensation value based on the angle between the positive direction of the X axis of the wearable device 100 and the first direction. In this way, this can further improve accuracy of the blood pressure measurement value collected by the wearable device 100 in the standing posture of the user.

Optionally, the wearable device 100 may determine, based on a motion trajectory of the wearable device 100, whether the wearable device 100 is worn on the left hand or the right hand. A motion trajectory of the wearable device 100 worn on the left hand is different from a motion trajectory of the wearable device 100 worn on the right hand.

### 2. Identify the sitting posture of the user.

### (1) The wearable device 100 is worn on the left-hand wrist of the user.

The wearable device 100 may determine the sitting posture of the user based on but not limited to any one or more of the following manners.

**Manner 1:** The wearable device 100 may determine the standing posture of the user based on a heart rate and/or the motion data collected by the wearable device 100.

In some embodiments, the wearable device 100 may collect a heart rate of the user by using a pre-configured PPG sensor. When the user naturally walks or runs, the heart rate of the user is high. If the heart rate is greater than a second heart rate value and less than a first heart rate value, it indicates that a motion amount of the user is small, and the heart rate is stable. In this case, the wearable device 100 may determine that the user posture is the sitting posture. The first heart rate value is greater than the second heart rate value.

Optionally, the wearable device 100 may determine the user posture based on a plurality of groups of heart rate values within first duration before blood pressure measurement starts. If the plurality of groups of heart rate values within the first duration before the blood pressure measurement starts are greater than the second heart rate value and less than the first heart rate value, it may be determined that the user posture is the sitting posture.

In some embodiments, the wearable device 100 may alternatively collect motion data, for example, information such as a step count, of the user by using the pre-configured motion sensor. If a change value of a step count within the first duration before the blood pressure measurement starts is greater than a second value and less than a first value or is less than a first value, it indicates that a motion step count of the user is small, and it may be determined that the user posture is the sitting posture. The first value is greater than the second value.

Manner 2: The wearable device 100 may determine the sitting posture of the user based on an acceleration component of gravitational acceleration G on a Z axis.

When the user sits, the Z axis is always perpendicular to the gravitational acceleration G, and the sitting posture of the user may be determined based on the acceleration component of the gravitational acceleration G on the Z axis.

Specifically, when the wearable device 100 determines that the acceleration component of the gravitational acceleration G on the Z axis is close to a minimum value, the sitting posture of the user may be determined.

**Manner 3:** The wearable device 100 may obtain a motion posture of the wearable device 100, and determine the standing posture of the user based on the motion posture of the wearable device 100.

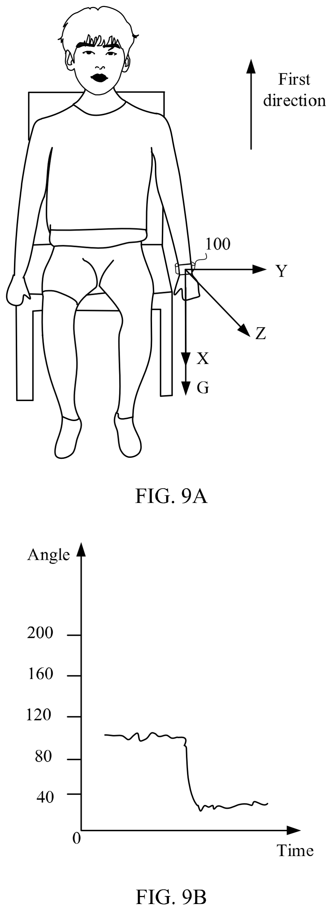

For example, FIG. 9A is a diagram in which a user wears a wearable device 100 when sitting.

As shown in FIG. 9A, a direction that is along a forearm of the user and that is parallel to a user finger is a positive direction of an X axis. A direction that is perpendicular to the positive direction of the X axis and that points to the outside is a positive direction of a Y axis. The X axis and the Y axis may determine an X-Y plane. A direction that is perpendicular to the X-Y plane and that is away from a wearable component is a positive direction of a Z axis.

A positive direction of gravitational acceleration G is always perpendicular to the ground. A direction that is parallel to the positive direction of the gravitational acceleration G and that is opposite to the positive direction of the gravitational acceleration G is a first direction.

The wearable device 100 may determine the standing posture of the user based on a change in an included angle between the X axis and the first direction.

In some embodiments, before the wearable device 100 starts to measure the blood pressure, the wearable device 100 may display the prompt information shown in FIG. 7B, to prompt the user to make the wearable device 100 flush with the heart. In this case, in a process in which the user raises the wearable device 100 to be flush with the heart, the change in the included angle between the X axis and the first direction may be an angle change shown in FIG. 8B.

As shown in FIG. 9B, when the user sits, the forearm of the user is usually placed on a leg or a desktop, and the included angle between the positive direction of the X axis of the wearable device 100 and the first direction is approximately 100 degrees. In a process in which the user raises the wearable device 100 to be flush with the heart, the included angle between the positive direction of the X axis and the first direction decreases sharply, for example, decreases from 100 degrees to approximately 40 degrees. After the user makes the wearable device 100 be flush with the heart, the included angle between the positive direction of the X axis and the first direction is stable at approximately 40 degrees.

The motion posture of the wearable device 100 may be a change value of the included angle between the positive direction of the X axis and the first direction. The standing posture of the user may be determined based on the change value of the included angle between the positive direction of the X axis and the first direction. Specifically, if it is detected, before the blood pressure measurement starts, that a change value of an included angle between the positive direction of the X axis and the first direction within second duration is greater than a second angle value and less than a first angle value, the standing posture of the user may be determined. The first angle value is greater than the second angle value. For example, the first angle value may be 100 degrees, and the second angle value may be 50 degrees. The first angle value and the second angle value may alternatively be other values. This is not limited in this application either.

In addition to the foregoing three manners, the wearable device 100 may determine the standing posture of the user in another manner. This is not limited in this application either.

After determining the sitting posture of the user, the wearable device 100 may determine a blood pressure compensation value corresponding to the sitting posture of the user based on the sitting posture of the user, and correct, based on the blood pressure compensation value corresponding to the sitting posture of the user, the blood pressure measurement value collected by the wearable device 100.

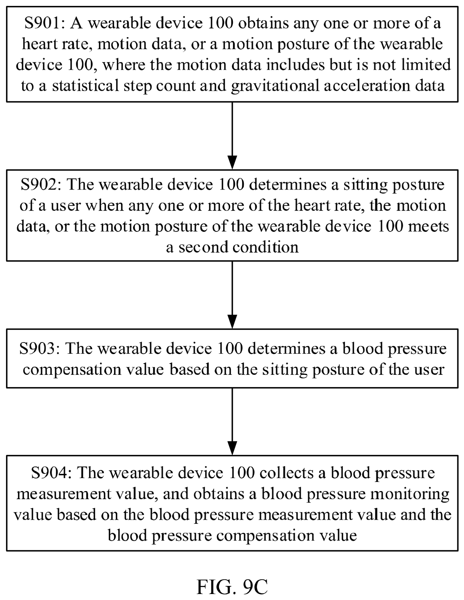

FIG. 9C is a schematic flowchart of a method for correcting a collected blood pressure measurement value by a wearable device 100.

S901: The wearable device 100 obtains any one or more of the heart rate, the motion data, or the motion posture of the wearable device 100, where the motion data includes but is not limited to a statistical step count and gravitational acceleration data.

S902: The wearable device 100 determines the sitting posture of the user when any one or more of the heart rate, the motion data, or the motion posture of the wearable device 100 meets a second condition.

The heart rate may be collected by the PPG sensor pre-configured in the wearable device 100, or the heart rate may be collected by the electronic device 200 and then sent to the wearable device 100. This is not limited in this application.

The motion data and the motion posture of the wearable device 100 may be collected by an inertial sensor pre-configured in the wearable device 100.

The wearable device 100 determines the sitting posture of the user when any one or more of the heart rate, the motion data, or the motion posture of the wearable device 100 meets the second condition.

The second condition may include but is not limited to any one or more of the following:
1. The plurality of groups of heart rate values within the first duration before the blood pressure measurement starts are greater than a second heart rate value and less than a first heart rate value.
2. It is detected, before the blood pressure measurement starts, that the change value of the included angle between the positive direction of the X axis and the first direction within the second duration is greater than the second angle value and less than the first angle value.
3. The acceleration component of the gravitational acceleration G on the Z axis is close to the minimum value.

For how the wearable device 100 determines the sitting posture of the user, refer to the descriptions in Manner 1 to Manner 3. Details are not described herein again in this application.

S903: The wearable device 100 determines the blood pressure compensation value based on the sitting posture of the user.

It should be noted that different blood pressure compensation values are determined based on different user postures. For example, the standing posture, the sitting posture, and the lying posture correspond to different blood pressure compensation values.

After determining the sitting posture of the user, the wearable device 100 may determine the blood pressure compensation value corresponding to the sitting posture of the user based on the sitting posture of the user.

In a possible implementation, the wearable device 100 locally stores blood pressure compensation values corresponding to a plurality of different user postures. The wearable device 100 may locally determine the blood pressure compensation value corresponding to the sitting posture of the user based on the sitting posture of the user.

In another possible implementation, the wearable device 100 may send the sitting posture of the user to the electronic device 200 that establishes a communication connection to the wearable device 100. The electronic device 200 locally stores the blood pressure compensation values corresponding to the plurality of different user postures. The electronic device 200 may locally determine the blood pressure compensation value corresponding to the sitting posture of the user based on the sitting posture of the user. Then, the electronic device 200 sends the blood pressure compensation value corresponding to the sitting posture of the user to the wearable device 100.

In another possible implementation, the wearable device 100 may send the sitting posture of the user to a server. The server locally stores the blood pressure compensation values corresponding to the plurality of different user postures. The server may locally determine the blood pressure compensation value corresponding to the sitting posture of the user based on the sitting posture of the user. Then, the server sends the blood pressure compensation value corresponding to the sitting posture of the user to the wearable device 100.

Optionally, the blood pressure compensation values corresponding to the different user postures may be periodically/aperiodically updated.

S904: The wearable device 100 collects the blood pressure measurement value, and obtains the blood pressure monitoring value based on the blood pressure measurement value and the blood pressure compensation value.

The wearable device 100 may collect the blood pressure measurement value, and obtain the blood pressure monitoring value based on the blood pressure measurement value and the blood pressure compensation value. The blood pressure monitoring value is a blood pressure value finally obtained through measurement.

In this way, when the user blood pressure is measured, impact of different user postures on the blood pressure measurement result can be eliminated, to improve accuracy of the blood pressure measurement result.

In some embodiments, when the wearable device 100 identifies the sitting posture of the user, placing the wrist wearing the wearable device 100 at different locations also affects the blood pressure measurement value collected by the wearable device 100.

Based on this, to further improve accuracy of the blood pressure measurement value collected by the wearable device 100, the wearable device 100 further needs to determine a location of the wrist wearing the wearable device 100, determine the blood pressure compensation value based on the location of the wrist wearing the wearable device 100, and then obtain a final blood pressure monitoring value based on the blood pressure measurement value collected by the wearable device 100 and the blood pressure compensation value. In other words, in the sitting posture of the user, the blood pressure compensation value varies with the location of the wrist wearing the wearable device 100.

In a possible implementation, the blood pressure compensation value may be determined based on the included angle between the positive direction of the X axis of the wearable device 100 and the first direction.

In a possible implementation, in the sitting posture, the wearable device 100 locally stores blood pressure compensation values corresponding to a plurality of angles between the positive direction of the X axis and the first direction. The wearable device 100 may locally determine a blood pressure compensation value corresponding to a first angle based on the angle between the positive direction of the X axis and the first direction.

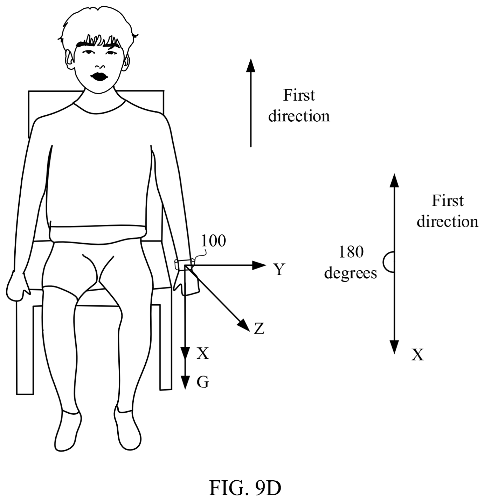

FIG. 9D to FIG. 9F are a group of diagrams in which a wrist wearing a wearable device 100 is located at different placement locations when a user is in a sitting posture.

FIG. 9D is a diagram in which an arm is placed vertically pointing to a ground when a user is in a sitting posture.

As shown in FIG. 9D, the positive direction of the X axis is opposite to the first direction, and the included angle between the positive direction of the X axis and the first direction is close to 180 degrees.

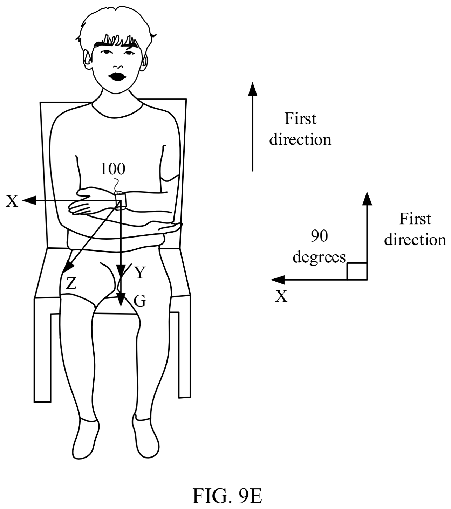

FIG. 9E is a diagram of horizontally placing an arm when a user is in a sitting posture.

As shown in FIG. 9E, the included angle between the positive direction of the X axis and the first direction is close to 90 degrees.

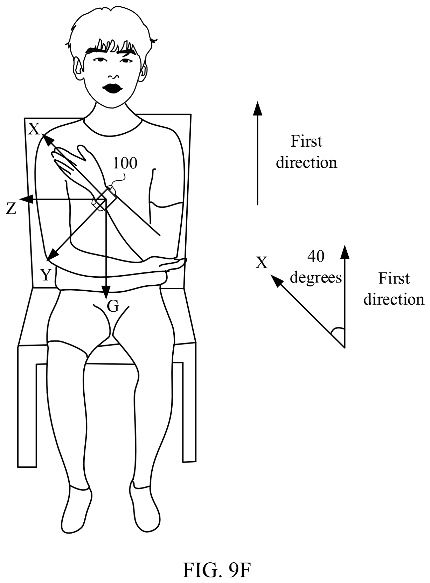

FIG. 9F is a diagram in which a wrist wearing a wearable device 100 is placed at a location of a heart when a user is in a sitting posture.

As shown in FIG. 9F, the included angle between the positive direction of the X axis and the first direction is close to 40 degrees.

**Table 5**

| Included angle between a positive direction of an X axis and a first direction | Blood pressure compensation value |
|---|---|
| 180 degrees to 140 degrees | I |
| 139 degrees to 90 degrees | J |
| 89 degrees to 40 degrees | K |
| 39 degrees to 0 degrees | L |

Table 5 shows the blood pressure compensation value corresponding to the angle between the positive direction of the X axis and the first direction. As shown in Table 5, when the included angle between the positive direction of the X axis and the first direction is between 180 degrees and 140 degrees, the blood pressure compensation value is I. When the included angle between the positive direction of the X axis and the first direction is between 139 degrees and 90 degrees, the blood pressure compensation value is H. When the included angle between the positive direction of the X axis and the first direction is between 89 degrees and 40 degrees, the blood pressure compensation value is K. When the included angle between the positive direction of the X axis and the first direction is between 39 degrees and 0 degrees, the blood pressure compensation value is L.

The blood pressure compensation value I, the blood pressure compensation value J, the blood pressure compensation value K, and the blood pressure compensation value L are different from each other.

Optionally, the blood pressure compensation value I is different from the blood pressure compensation value A, the blood pressure compensation value J is different from the blood pressure compensation value B, the blood pressure compensation value K is different from the blood pressure compensation value C, and the blood pressure compensation value L is different from the blood pressure compensation value D. That is, in different user postures, blood pressure compensation values of the wearable device 100 at a same location are also different.

For example, when the wearable device 100 determines that the included angle between the positive direction of the X axis and the first direction is 160 degrees, the wearable device 100 may determine that the blood pressure compensation value is I.

For another example, when the wearable device 100 determines that the included angle between the positive direction of the X axis and the first direction is 40 degrees, the wearable device 100 may determine that the blood pressure compensation value is K.

Table 5 merely describes the blood pressure compensation value corresponding to the angle between the positive direction of the X axis and the first direction as an example. Alternatively, each angle may correspond to one blood pressure compensation value. This is not limited in this application either.

In another possible implementation, the wearable device 100 may send the first angle between the positive direction of the X axis and the first direction in the sitting posture of the user to the electronic device 200 that establishes a communication connection to the wearable device 100. The electronic device 200 locally stores blood pressure compensation values corresponding to a plurality of angles between the positive direction of the X axis and the first direction in the sitting posture of the user. The electronic device 200 may locally determine the blood pressure compensation value based on the first angle between the positive direction of the X axis and the first direction in the sitting posture of the user. Then, the electronic device 200 sends the determined blood pressure compensation value to the wearable device 100.

In another possible implementation, the wearable device 100 may send the first angle between the positive direction of the X axis and the first direction in the sitting posture of the user to the server. The server locally stores the blood pressure compensation values corresponding to the plurality of angles between the positive direction of the X axis and the first direction in the sitting posture of the user. The server may locally determine the blood pressure compensation value based on the first angle between the positive direction of the X axis and the first direction in the sitting posture of the user. Then, the server sends the determined blood pressure compensation value to the wearable device 100.

In another embodiment, the wearable device 100 may determine a distance between the wearable device 100 and the heart in a vertical direction based on the included angle between the X axis of the wearable device 100 and the first direction, and then determine the blood pressure compensation value based on the distance between the wearable device 100 and the heart in the vertical direction.

In a possible implementation, the distance between the wearable device 100 and the heart in the vertical direction may be determined by the wearable device 100 based on the included angle between the positive direction of the X axis of the wearable device 100 and the first direction; or may be determined, by the electronic device 2000 that establishes a communication connection to the wearable device 100, based on the included angle between the positive direction of the X axis of the wearable device 100 and the first direction; may also be determined by the server based on the included angle between the positive direction of the X axis of the wearable device 100 and the first direction.

**Table 6**

| Included angle between a positive direction of an X axis and a first direction | Distance between a wearable device 100 and a heart in a vertical direction | Blood pressure compensation value |
|---|---|---|
| 180 degrees to 140 degrees | i | I |
| 139 degrees to 90 degrees | j | J |
| 89 degrees to 40 degrees | k | K |
| 39 degrees to 0 degrees | 1 | L |

Table 6 shows the blood pressure compensation value corresponding to the angle between the positive direction of the X axis and the first direction. As shown in Table 6, when the included angle between the positive direction of the X axis and the first direction is between 180 degrees and 140 degrees, the distance between the wearable device 100 and the heart in the vertical direction is i, and the blood pressure compensation value is I. When the included angle between the positive direction of the X axis and the first direction is between 139 degrees and 90 degrees, the distance between the wearable device 100 and the heart in the vertical direction is j, and the blood pressure compensation value is J. When the included angle between the positive direction of the X axis and the first direction is between 89 degrees and 40 degrees, the distance between the wearable device 100 and the heart in the vertical direction is c, and the blood pressure compensation value is C. When the included angle between the positive direction of the X axis and the first direction is between 39 degrees and 0 degrees, the distance between the wearable device 100 and the heart in the vertical direction is k, and the blood pressure compensation value is L.

The blood pressure compensation value I, the blood pressure compensation value J, the blood pressure compensation value K, and the blood pressure compensation value L are different from each other.

The blood pressure compensation value corresponding to the distance between the wearable device 100 and the heart in the vertical direction in Table 6 may be stored in the wearable device 100, or may be stored in the electronic device 200, or may be stored in the server. This is not limited in this application.

### (2) The wearable device 100 is worn on a right-hand wrist of the user.

For a specific implementation of how to identify the sitting posture of the user when the wearable device 100 is worn on the right-hand wrist of the user, refer to descriptions of a specific implementation of how to identify the sitting posture of the user when the wearable device 100 is worn on the left-hand wrist of the user. Details are not described herein again in this application.

A difference lies in that the positive direction of the X axis, the positive direction of the Y axis, and the positive direction of the Z axis when the wearable device 100 is worn on the right-hand wrist of the user are different from the positive direction of the X axis, the positive direction of the Y axis, and the positive direction of the Z axis when the wearable device 100 is worn on the left-hand wrist of the user.

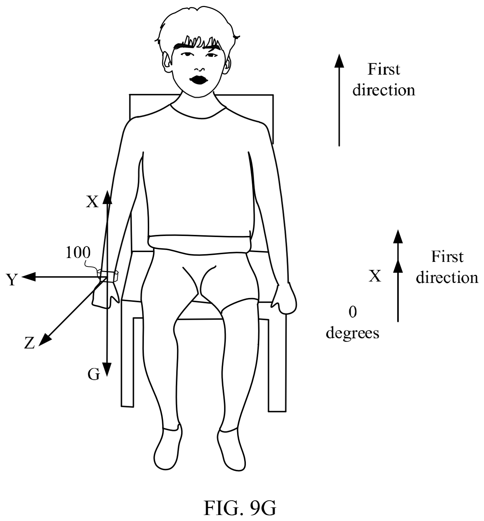

FIG. 9G to FIG. 9I are another group of diagrams in which a wrist wearing a wearable device 100 is located at different placement locations when a user is in a sitting posture.

FIG. 9G is a diagram in which an arm is placed vertically pointing to a ground when a user is in a sitting posture.

As shown in FIG. 9G, the positive direction of the X axis is the same as the first direction, and the included angle between the positive direction of the X axis and the first direction is close to 0 degrees.

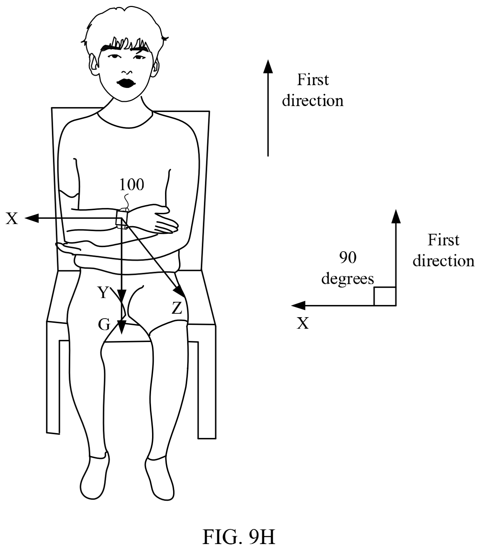

FIG. 9H is a diagram of horizontally placing an arm when a user is in a sitting posture.

As shown in FIG. 9H, the included angle between the positive direction of the X axis and the first direction is close to 90 degrees.

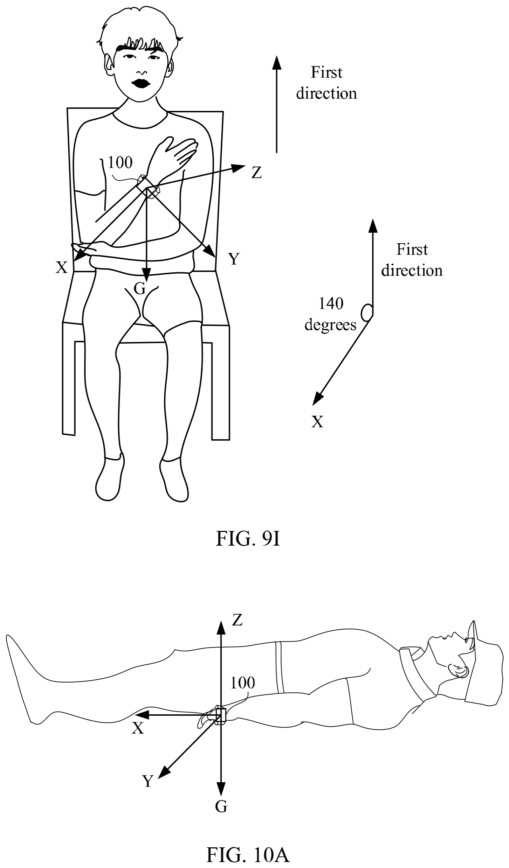

FIG. 9I is a diagram in which a wrist wearing a wearable device 100 is placed at a location of a heart when a user is in a sitting posture.

As shown in FIG. 9I, the included angle between the positive direction of the X axis and the first direction is close to 140 degrees.

**Table 7**

| Included angle between a positive direction of an X axis and a first direction | Blood pressure compensation value |
|---|---|
| 180 degrees to 140 degrees | M |
| 139 degrees to 90 degrees | N |
| 89 degrees to 40 degrees | O |
| 39 degrees to 0 degrees | P |

Table 7 shows the blood pressure compensation value corresponding to the angle between the positive direction of the X axis and the first direction. As shown in Table 7, when the included angle between the positive direction of the X axis and the first direction is between 180 degrees and 140 degrees, the blood pressure compensation value is M. When the included angle between the positive direction of the X axis and the first direction is between 139 degrees and 90 degrees, the blood pressure compensation value is N. When the included angle between the positive direction of the X axis and the first direction is between 89 degrees and 40 degrees, the blood pressure compensation value is O. When the included angle between the positive direction of the X axis and the first direction is between 39 degrees and 0 degrees, the blood pressure compensation value is P.

The blood pressure compensation value M, the blood pressure compensation value N, the blood pressure compensation value O, and the blood pressure compensation value P are different from each other.

For example, when the wearable device 100 determines that the included angle between the positive direction of the X axis and the first direction is 160 degrees, the wearable device 100 may determine that the blood pressure compensation value is M.

For another example, when the wearable device 100 determines that the included angle between the positive direction of the X axis and the first direction is 40 degrees, the wearable device 100 may determine that the blood pressure compensation value is O.

Table 7 merely describes the blood pressure compensation value corresponding to the angle between the positive direction of the X axis and the first direction as an example. Alternatively, each angle may correspond to one blood pressure compensation value. This is not limited in this application either.

In another possible implementation, the wearable device 100 may send the first angle between the positive direction of the X axis and the first direction in the sitting posture of the user to the electronic device 200 that establishes a communication connection to the wearable device 100. The electronic device 200 locally stores blood pressure compensation values corresponding to a plurality of angles between the positive direction of the X axis and the first direction in the sitting posture of the user. The electronic device 200 may locally determine the blood pressure compensation value based on the first angle between the positive direction of the X axis and the first direction in the sitting posture of the user. Then, the electronic device 200 sends the determined blood pressure compensation value to the wearable device 100.

In another possible implementation, the wearable device 100 may send the first angle between the positive direction of the X axis and the first direction in the sitting posture of the user to the server. The server locally stores the blood pressure compensation values corresponding to the plurality of angles between the positive direction of the X axis and the first direction in the sitting posture of the user. The server may locally determine the blood pressure compensation value based on the first angle between the positive direction of the X axis and the first direction in the sitting posture of the user. Then, the server sends the determined blood pressure compensation value to the wearable device 100.

In another embodiment, the wearable device 100 may determine a distance between the wearable device 100 and the heart in a vertical direction based on the included angle between the X axis of the wearable device 100 and the first direction, and then determine the blood pressure compensation value based on the distance between the wearable device 100 and the heart in the vertical direction.

In a possible implementation, the distance between the wearable device 100 and the heart in the vertical direction may be determined by the wearable device 100 based on the included angle between the positive direction of the X axis of the wearable device 100 and the first direction; or may be determined, by the electronic device 2000 that establishes a communication connection to the wearable device 100, based on the included angle between the positive direction of the X axis of the wearable device 100 and the first direction; may also be determined by the server based on the included angle between the positive direction of the X axis of the wearable device 100 and the first direction.

**Table 8**

| Included angle between a positive direction of an X axis and a first direction | Distance between a wearable device 100 and a heart in a vertical direction | Blood pressure compensation value |
|---|---|---|
| 180 degrees to 140 degrees | m | M |
| 139 degrees to 90 degrees | n | N |
| 89 degrees to 40 degrees | o | O |
| 39 degrees to 0 degrees | p | P |

Table 8 shows the blood pressure compensation value corresponding to the angle between the positive direction of the X axis and the first direction. As shown in Table 8, when the included angle between the positive direction of the X axis and the first direction is between 180 degrees and 140 degrees, the distance between the wearable device 100 and the heart in the vertical direction is m, and the blood pressure compensation value is M. When the included angle between the positive direction of the X axis and the first direction is between 139 degrees and 90 degrees, the distance between the wearable device 100 and the heart in the vertical direction is n, and the blood pressure compensation value is N. When the included angle between the positive direction of the X axis and the first direction is between 89 degrees and 40 degrees, the distance between the wearable device 100 and the heart in the vertical direction is o, and the blood pressure compensation value is O. When the included angle between the positive direction of the X axis and the first direction is between 39 degrees and 0 degrees, the distance between the wearable device 100 and the heart in the vertical direction is p, and the blood pressure compensation value is P.

The distance m, the distance n, the distance o, and the distance p are different from each other.

The blood pressure compensation value M, the blood pressure compensation value N, the blood pressure compensation value O, and the blood pressure compensation value P are different from each other.

The blood pressure compensation value corresponding to the distance between the wearable device 100 and the heart in the vertical direction in Table 8 may be stored in the wearable device 100, or may be stored in the electronic device 200, or may be stored in the server. This is not limited in this application.

In the method, the wearable device 100 may identify the sitting posture of the user, determine the blood pressure compensation value based on the sitting posture of the user, and correct, based on the blood pressure compensation value, the blood pressure measurement value collected by the wearable device 100.

In some embodiments, after identifying the sitting posture of the user, the wearable device 100 may determine the blood pressure compensation value based on the angle between the positive direction of the X axis of the wearable device 100 and the first direction. Because the wearable device 100 is worn at different locations, angles between the positive direction of the X axis and the first direction are different, and blood pressure compensation values are also different. In this way, this can further improve accuracy of the blood pressure measurement value collected by the wearable device 100 in the sitting posture of the user.

In some embodiments, the wearable device 100 may further identify whether the wearable device 100 is worn on a left hand or a right hand, and then determine the blood pressure compensation value based on the angle between the positive direction of the X axis of the wearable device 100 and the first direction. Because the wearable device 100 is worn on the left hand or the right hand, when the wearable device 100 is located at a same location, angles between the positive direction of the X axis and the first direction are different. Therefore, whether the wearable device 100 is worn on the left hand or on the right hand needs to be distinguished. After determining whether the wearable device 100 is worn on the left hand or the right hand, the wearable device 100 determines the blood pressure compensation value based on the angle between the positive direction of the X axis of the wearable device 100 and the first direction. In this way, this can further improve accuracy of the blood pressure measurement value collected by the wearable device 100 in the sitting posture of the user.

### 3. Identify the lying posture of the user

### (1) The wearable device 100 is worn on the left-hand wrist of the user.

The wearable device 100 may determine the lying posture of the user based on but not limited to any one or more of the following manners.

**Manner 1:** The wearable device 100 may determine the lying posture of the user based on a heart rate and/or the motion data collected by the wearable device 100.

In some embodiments, the wearable device 100 may collect a heart rate of the user by using a pre-configured PPG sensor. When the user is in the lying posture, the heart rate of the user is low. If the heart rate is less than a second heart rate value, it may be determined that the user posture is the lying posture. For example, after the user falls asleep at night, if the heart rate of the user is low and stable, it may be determined that the user posture is the lying posture.

Optionally, the wearable device 100 may determine the user posture based on a plurality of groups of heart rate values within first duration before blood pressure measurement starts. If the plurality of groups of heart rate values within the first duration before the blood pressure measurement starts is less than the second heart rate value, it may be determined that the user posture is the lying posture.

In some embodiments, the wearable device 100 may alternatively collect motion data, for example, information such as a step count, of the user by using the pre-configured motion sensor. If a change value of a step count within the first duration before the blood pressure measurement starts is less than a second value, it indicates that the user basically has no motion, and it may be determined that the user posture is the lying posture. For example, after the user falls asleep at night, the motion data of the user basically does not change, and it may be determined that the user posture is the lying posture.

**Manner 2:** The wearable device 100 may determine the lying posture of the user based on acceleration components of gravitational acceleration G on an X axis and a Y axis.

In some embodiments, as shown in FIG. 10A, when the user lies, the X axis and the Y axis are always perpendicular to the gravitational acceleration G, and the lying posture of the user may be determined based on the acceleration components of the gravitational acceleration G on the X axis and the Y axis.

Specifically, when the wearable device 100 determines that the acceleration components of the gravitational acceleration G on the X axis and the Y axis are close to a minimum value, the lying posture of the user may be determined.

**Manner 3:** The lying posture of the user may be determined between a first moment at night and a second moment in the morning.

For example, the first moment may be 10 p.m., and the second moment may be 6 a.m. From 10 p.m. to 6 a.m., it may be determined that the user is in a sleep state, and it may be determined that the user posture is the lying posture.

In addition to a time period from 10 p.m. to 6 a.m., a time period in which it is determined that the user is in the sleep state may be another time period. This is not limited in this application.

**Manner 4:** The lying posture of the user is determined based on the motion posture of the wearable device 100.

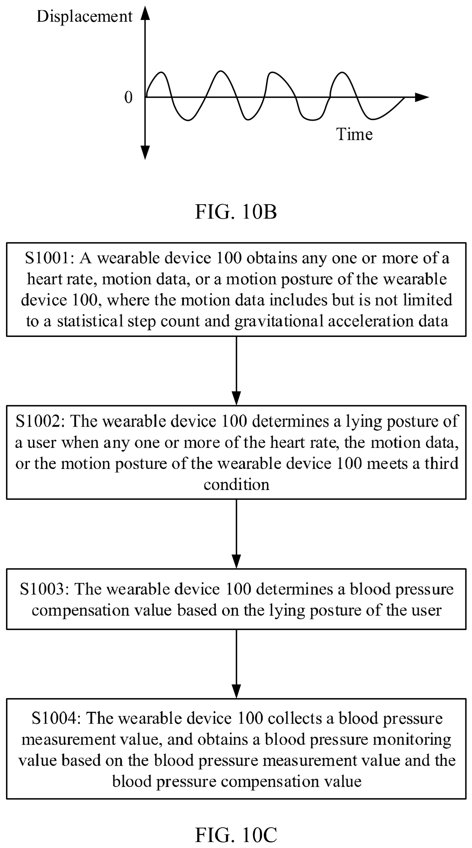

In some embodiments, when the user sleeps, if the user places a hand on an abdomen, a wrist moves up and down regularly with breathing of the abdomen. A displacement trajectory of the wearable device 100 may be a displacement trajectory shown in FIG. 10B. The lying posture of the user may be determined based on the displacement trajectory of the wearable device 100.

In addition to the foregoing four manners, the wearable device 100 may determine the lying posture of the user in another manner. This is not limited in this application either.

After determining the lying posture of the user, the wearable device 100 may determine a blood pressure compensation value corresponding to the lying posture of the user based on the lying posture of the user, and correct, based on the blood pressure compensation value corresponding to the lying posture of the user, the blood pressure measurement value collected by the wearable device 100.

FIG. 10C is a schematic flowchart of a method for correcting a collected blood pressure measurement value by a wearable device 100.

S1001: The wearable device 100 obtains any one or more of the heart rate, the motion data, or the motion posture of the wearable device 100, where the motion data includes but is not limited to a statistical step count and gravitational acceleration data.

S1002: The wearable device 100 determines the lying posture of the user when any one or more of the heart rate, the motion data, or the motion posture of the wearable device 100 meets a third condition.

The first condition, the second condition, and the third condition are different from each other.

The heart rate may be collected by the PPG sensor pre-configured in the wearable device 100, or the heart rate may be collected by the electronic device 200 and then sent to the wearable device 100. This is not limited in this application.

The motion data and the motion posture of the wearable device 100 may be collected by an inertial sensor pre-configured in the wearable device 100.

The wearable device 100 determines the lying posture of the user when any one or more of the heart rate, the motion data, or the motion posture of the wearable device 100 meets the third condition.

The third condition may include but is not limited to any one or more of the following:
1. The plurality of groups of heart rate values within the first duration before the blood pressure measurement starts are less than the second heart rate value;
2. The acceleration components of the gravitational acceleration G on the X axis and Y axis are close to the minimum value.
3. A time is between the first moment at night and the second moment in the morning.
4. The displacement trajectory of the wearable device 100 satisfies a preset trajectory.

For how the wearable device 100 determines the lying posture of the user, refer to the descriptions in Manner 1 to Manner 4. Details are not described herein again in this application.

S1003: The wearable device 100 determines the blood pressure compensation value based on the lying posture of the user.

It should be noted that different blood pressure compensation values are determined based on different user postures. For example, the standing posture, the sitting posture, and the lying posture correspond to different blood pressure compensation values.

After determining the lying posture of the user, the wearable device 100 may determine the blood pressure compensation value corresponding to the lying posture of the user based on the lying posture of the user.

In a possible implementation, the wearable device 100 locally stores blood pressure compensation values corresponding to a plurality of different user postures. The wearable device 100 may locally determine the blood pressure compensation value corresponding to the lying posture of the user based on the lying posture of the user.

In another possible implementation, the wearable device 100 may send the lying posture of the user to the electronic device 200 that establishes a communication connection to the wearable device 100. The electronic device 200 locally stores the blood pressure compensation values corresponding to the plurality of different user postures. The electronic device 200 may locally determine the blood pressure compensation value corresponding to the lying posture of the user based on the lying posture of the user. Then, the electronic device 200 sends the blood pressure compensation value corresponding to the lying posture of the user to the wearable device 100.

In another possible implementation, the wearable device 100 may send the lying posture of the user to a server. The server locally stores the blood pressure compensation values corresponding to the plurality of different user postures. The server may locally determine the blood pressure compensation value corresponding to the lying posture of the user based on the lying posture of the user. Then, the server sends the blood pressure compensation value corresponding to the lying posture of the user to the wearable device 100.

Optionally, the blood pressure compensation values corresponding to the different user postures may be periodically/aperiodically updated.

S 1004: The wearable device 100 collects the blood pressure measurement value, and obtains the blood pressure monitoring value based on the blood pressure measurement value and the blood pressure compensation value.

The wearable device 100 may collect the blood pressure measurement value, and obtain the blood pressure monitoring value based on the blood pressure measurement value and the blood pressure compensation value. The blood pressure monitoring value is a blood pressure value finally obtained through measurement.

In this way, when the user blood pressure is measured, impact of different user postures on the blood pressure measurement result can be eliminated, to improve accuracy of the blood pressure measurement result.

In some embodiments, when the wearable device 100 identifies the lying posture of the user, placing the wrist wearing the wearable device 100 at different locations also affects the blood pressure measurement value collected by the wearable device 100.

Based on this, to further improve accuracy of the blood pressure measurement value collected by the wearable device 100, the wearable device 100 further needs to determine a location of the wrist wearing the wearable device 100, determine the blood pressure compensation value based on the location of the wrist wearing the wearable device 100, and then obtain a final blood pressure monitoring value based on the blood pressure measurement value collected by the wearable device 100 and the blood pressure compensation value. In other words, in the lying posture of the user, the blood pressure compensation value varies with the location of the wrist wearing the wearable device 100.

In a possible implementation, the blood pressure compensation value may be determined based on the included angle between the positive direction of the X axis of the wearable device 100 and the first direction.

In a possible implementation, in the lying posture, the wearable device 100 locally stores blood pressure compensation values corresponding to a plurality of angles between the positive direction of the X axis and the first direction. The wearable device 100 may locally determine a blood pressure compensation value corresponding to a first angle based on the angle between the positive direction of the X axis and the first direction.

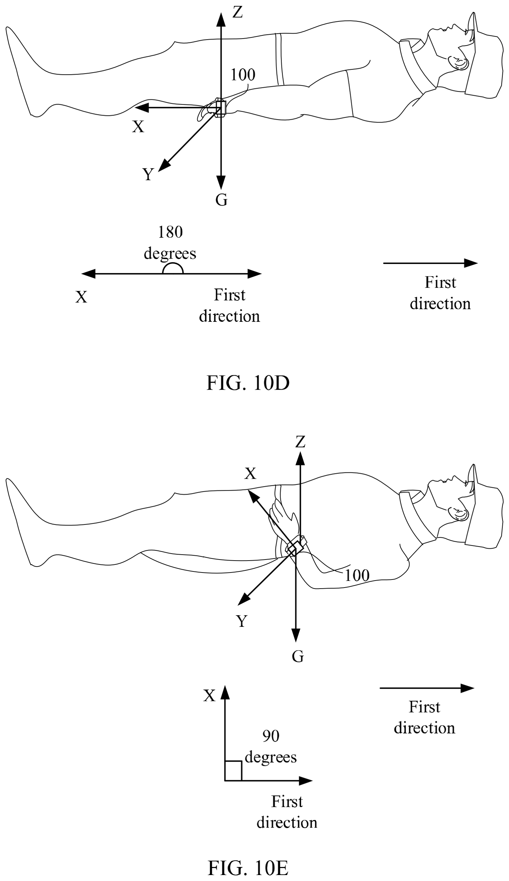

FIG. 10D to FIG. 10F are a group of diagrams in which a wrist wearing a wearable device 100 is located at different placement locations when a user is in a lying posture.

FIG. 10D is a diagram of horizontally placing an arm on a bed when a user is in a lying posture.

As shown in FIG. 10D, a direction that is along a forearm of the user and that is parallel to a user finger is a positive direction of an X axis. A direction that is perpendicular to the positive direction of the X axis and that points to the outside is a positive direction of a Y axis. The X axis and the Y axis may determine an X-Y plane. A direction that is perpendicular to the X-Y plane and that is away from a wearable component is a positive direction of a Z axis. A positive direction of gravitational acceleration G is always perpendicular to the ground. A direction that is perpendicular to the gravitational acceleration G and that points to the head is used as the first direction.

As shown in FIG. 10D, when the user is in the lying posture and the arm is horizontally placed, the positive direction of the X axis is opposite to the first direction, and the included angle between the positive direction of the X axis and the first direction is close to 180 degrees.

FIG. 10E is a diagram in which a wrist wearing a wearable device 100 is placed on an abdomen when a user is in a lying posture.

As shown in FIG. 10E, when the wrist wearing the wearable device 100 is placed on the abdomen when the user is in the lying posture, the included angle between the positive direction of the X axis and the first direction is close to 90 degrees.

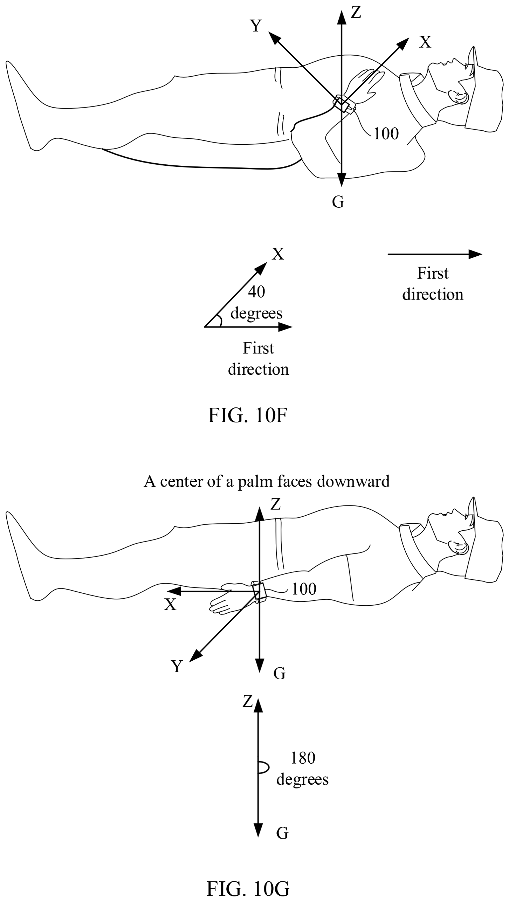

FIG. 10F is a diagram in which a wrist wearing a wearable device 100 is placed at a location of a heart when a user is in a lying posture.

As shown in FIG. 10F, when the wrist wearing the wearable device 100 is placed at the location of the heart when the user is in the lying posture, the included angle between the positive direction of the X axis and the first direction is close to 40 degrees.

**Table 9**

| Included angle between a positive direction of an X axis and a first direction | Blood pressure compensation value |
|---|---|
| 180 degrees to 140 degrees | M |
| 139 degrees to 90 degrees | N |
| 89 degrees to 40 degrees | O |
| 39 degrees to 0 degrees | P |

Table 9 shows the blood pressure compensation value corresponding to the angle between the positive direction of the X axis and the first direction. As shown in Table 9, when the included angle between the positive direction of the X axis and the first direction is between 180 degrees and 140 degrees, the blood pressure compensation value is M. When the included angle between the positive direction of the X axis and the first direction is between 139 degrees and 90 degrees, the blood pressure compensation value is N. When the included angle between the positive direction of the X axis and the first direction is between 89 degrees and 40 degrees, the blood pressure compensation value is P. When the included angle between the positive direction of the X axis and the first direction is between 39 degrees and 0 degrees, the blood pressure compensation value is Q.

The blood pressure compensation value M, the blood pressure compensation value N, the blood pressure compensation value O, and the blood pressure compensation value P are different from each other.

Optionally, the blood pressure compensation value M is different from the blood pressure compensation value A, the blood pressure compensation value N is different from the blood pressure compensation value B, the blood pressure compensation value O is different from the blood pressure compensation value C, and the blood pressure compensation value P is different from the blood pressure compensation value D. That is, in different user postures, blood pressure compensation values of the wearable device 100 at a same location are also different.

For example, when the wearable device 100 determines that the included angle between the positive direction of the X axis and the first direction is 160 degrees, the wearable device 100 may determine that the blood pressure compensation value is M.

For another example, when the wearable device 100 determines that the included angle between the positive direction of the X axis and the first direction is 40 degrees, the wearable device 100 may determine that the blood pressure compensation value is O.

Table 9 merely describes the blood pressure compensation value corresponding to the angle between the positive direction of the X axis and the first direction as an example. Alternatively, each angle may correspond to one blood pressure compensation value. This is not limited in this application either.

In another possible implementation, the wearable device 100 may send the first angle between the positive direction of the X axis and the first direction in the lying posture of the user to the electronic device 200 that establishes a communication connection to the wearable device 100. The electronic device 200 locally stores blood pressure compensation values corresponding to a plurality of angles between the positive direction of the X axis and the first direction in the lying posture of the user. The electronic device 200 may locally determine the blood pressure compensation value based on the first angle between the positive direction of the X axis and the first direction in the lying posture of the user. Then, the electronic device 200 sends the determined blood pressure compensation value to the wearable device 100.

In another possible implementation, the wearable device 100 may send the first angle between the positive direction of the X axis and the first direction in the lying posture of the user to the server. The server locally stores the blood pressure compensation values corresponding to the plurality of angles between the positive direction of the X axis and the first direction in the lying posture of the user. The server may locally determine the blood pressure compensation value based on the first angle between the positive direction of the X axis and the first direction in the lying posture of the user. Then, the server sends the determined blood pressure compensation value to the wearable device 100.

In another embodiment, the wearable device 100 may determine a distance between the wearable device 100 and the heart in a vertical direction based on the included angle between the X axis of the wearable device 100 and the first direction, and then determine the blood pressure compensation value based on the distance between the wearable device 100 and the heart in the vertical direction.

In a possible implementation, the distance between the wearable device 100 and the heart in the vertical direction may be determined by the wearable device 100 based on the included angle between the positive direction of the X axis of the wearable device 100 and the first direction; or may be determined, by the electronic device 2000 that establishes a communication connection to the wearable device 100, based on the included angle between the positive direction of the X axis of the wearable device 100 and the first direction; may also be determined by the server based on the included angle between the positive direction of the X axis of the wearable device 100 and the first direction.

**Table 10**

| Included angle between a positive direction of an X axis and a first direction | Distance between a wearable device 100 and a heart in a vertical direction | Blood pressure compensation value |
|---|---|---|
| 180 degrees to 140 degrees | m | M |
| 139 degrees to 90 degrees | n | N |
| 89 degrees to 40 degrees | o | O |
| 39 degrees to 0 degrees | p | P |

Table 10 shows the blood pressure compensation value corresponding to the angle between the positive direction of the X axis and the first direction. As shown in Table 10, when the included angle between the positive direction of the X axis and the first direction is between 180 degrees and 140 degrees, the distance between the wearable device 100 and the heart in the vertical direction is m, and the blood pressure compensation value is M. When the included angle between the positive direction of the X axis and the first direction is between 139 degrees and 90 degrees, the distance between the wearable device 100 and the heart in the vertical direction is n, and the blood pressure compensation value is N. When the included angle between the positive direction of the X axis and the first direction is between 89 degrees and 40 degrees, the distance between the wearable device 100 and the heart in the vertical direction is o, and the blood pressure compensation value is O. When the included angle between the positive direction of the X axis and the first direction is between 39 degrees and 0 degrees, the distance between the wearable device 100 and the heart in the vertical direction is p, and the blood pressure compensation value is P.

The blood pressure compensation value M, the blood pressure compensation value N, the blood pressure compensation value O, and the blood pressure compensation value P are different from each other.

The blood pressure compensation value corresponding to the distance between the wearable device 100 and the heart in the vertical direction in Table 10 may be stored in the wearable device 100, or may be stored in the electronic device 200, or may be stored in the server. This is not limited in this application.

In some embodiments, when the user is in the lying posture, a placement posture of a palm also affects the blood pressure value collected by the wearable device 100. A placement posture of a center of the palm includes but is not limited to the following: the center of the palm faces upward, the center of the palm faces downward, the center of the palm is placed sideward, and the like.

When a placement location of the wrist wearing the wearable device 100 is fixed, the wearable device 100 may further identify a placement posture of the center of the palm, determine the blood pressure compensation value based on different placement postures of the center of the palm, and correct the blood pressure value collected by the wearable device 100. Accuracy of the blood pressure measurement value in the lying posture can be further improved.

How the wearable device 100 identifies the placement posture of the center of the palm when the user is in the lying posture and the arm is horizontally placed on the bed is used as an example for description in this embodiment of this application.

FIG. 10G to FIG. 10I are diagrams of three placement postures of the palm.

FIG. 10G is a diagram of placing a center of a palm downward when a user is in a lying posture and an arm is horizontally placed on a bed.

As shown in FIG. 10G, a placement posture that the center of the palm faces upward may be determined based on an included angle between the positive direction of the Z axis and the positive direction of the gravitational acceleration G.

For example, when it is detected that the user is in the lying posture, and the included angle between the positive direction of the Z axis and the direction of the gravitational acceleration G is the first angle, a placement posture that the center of the palm faces downward may be determined.

For example, the first angle may be 150 degrees to 180 degrees.

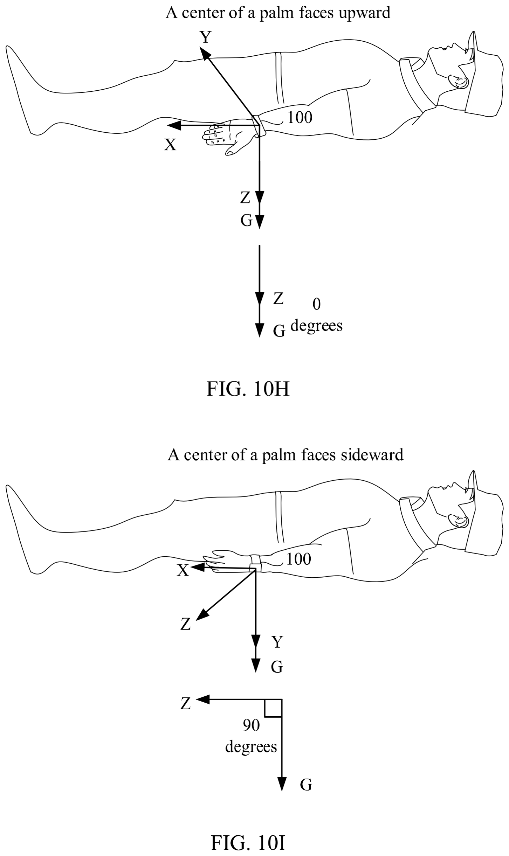

FIG. 10H is a diagram of placing a center of a palm upward when a user is in a lying posture and an arm is horizontally placed on a bed.

As shown in FIG. 10H, a placement posture that the center of the palm faces upward may be determined based on an included angle between the positive direction of the Z axis and the positive direction of the gravitational acceleration G.

For example, when it is detected that the user is in the lying posture, and the included angle between the positive direction of the Z axis and the direction of the gravitational acceleration G is the second angle, a placement posture that the center of the palm faces upward may be determined.

For example, the second angle may be 0 degrees to 30 degrees.

FIG. 10I is a diagram of placing a center of a palm sideward when a user is in a lying posture and an arm is horizontally placed on a bed.

As shown in FIG. 10I, a placement posture that the center of the palm faces upward may be determined based on an included angle between the positive direction of the Z axis and the positive direction of the gravitational acceleration G.

For example, when it is detected that the user is in the lying posture, and the included angle between the positive direction of the Z axis and the direction of the gravitational acceleration G is a third angle, a placement posture that the center of the palm is placed sideward may be determined.

For example, the second angle may be 80 degrees to 100 degrees.

The first angle is greater than the second angle and greater than the third angle. Values of the first angle, the second angle, and the third angle are merely used to explain this application. The values of the first angle, the second angle, and the third angle may alternatively be other values. This is not limited in this application either.

In addition to a manner based on the included angle between the positive direction of the Z axis and the positive direction of the gravitational acceleration G, the placement posture of the center of the palm may be further determined in another manner. This is not limited in this application either.

After determining the placement posture of the center of the palm, the wearable device 100 may determine a blood pressure compensation value corresponding to the placement posture of the center of the palm.

**Table 11**

| Included angle between a positive direction of an X axis and a first direction | Distance between a wearable device 100 and a heart in a vertical direction | | Blood pressure compensation value |
|---|---|---|---|
| 180 degrees to 140 degrees | m | A center of a palm faces upward | M1 |
| | | The center of the palm faces downward | M2 |
| | | The center of the palm faces sideward | M3 |
| 139 degrees to 90 degrees | n | The center of the palm faces upward | N1 |
| | | The center of the palm faces downward | N2 |
| | | The center of the palm faces sideward | N3 |
| 89 degrees to 40 degrees | o | The center of the palm faces upward | O1 |
| | | The center of the palm faces downward | O2 |
| | | The center of the palm faces sideward | O3 |
| 39 degrees to 0 degrees | p | The center of the palm faces upward | P1 |
| | | The center of the palm faces downward | P2 |
| | | The center of the palm faces sideward | P3 |

Table 11 shows the blood pressure compensation value corresponding to the angle between the positive direction of the X axis and the first direction. As shown in Table 11, when the included angle between the positive direction of the X axis and the first direction is between 180 degrees and 140 degrees, the blood pressure compensation value determined by the wearable device 100 is M1 when the center of the palm faces upward. The blood pressure compensation value determined by the wearable device 100 is M2 when the center of the palm faces downward. The blood pressure compensation value determined by the wearable device 100 is M3 when the center of the palm faces sideward. M1, M2, and M3 are different from each other.

When the included angle between the positive direction of the X axis and the first direction is between 139 degrees and 90 degrees, the blood pressure compensation value determined by the wearable device 100 is N1 when the center of the palm faces upward. The blood pressure compensation value determined by the wearable device 100 is N2 when the center of the palm faces downward. The blood pressure compensation value determined by the wearable device 100 is N3 when the center of the palm faces sideward. N1, N2, and N3 are different from each other.

When the included angle between the positive direction of the X axis and the first direction is between 89 degrees and 40 degrees, the blood pressure compensation value determined by the wearable device 100 is O1 when the center of the palm faces upward. The blood pressure compensation value determined by the wearable device 100 is O2 when the center of the palm faces downward. The blood pressure compensation value determined by the wearable device 100 is O3 when the center of the palm faces sideward. O1, O2, and O3 are different from each other.

When the included angle between the positive direction of the X axis and the first direction is between 39 degrees and 0 degrees, the blood pressure compensation value determined by the wearable device 100 is P1 when the center of the palm faces upward. The blood pressure compensation value determined by the wearable device 100 is P2 when the center of the palm faces downward. The blood pressure compensation value determined by the wearable device 100 is P3 when the center of the palm faces sideward. P1, P2, and P3 are different from each other.

The blood pressure compensation value corresponding to the distance between the wearable device 100 and the heart in the vertical direction in Table 11 may be stored in the wearable device 100, or may be stored in the electronic device 200, or may be stored in the server. This is not limited in this application.

### (2) The wearable device 100 is worn on a right-hand wrist of the user.

For a specific implementation of how to identify the lying posture of the user when the wearable device 100 is worn on the right-hand wrist of the user, refer to descriptions of a specific implementation of how to identify the lying posture of the user when the wearable device 100 is worn on the left-hand wrist of the user. Details are not described herein again in this application.

A difference lies in that the positive direction of the X axis, the positive direction of the Y axis, and the positive direction of the Z axis when the wearable device 100 is worn on the right-hand wrist of the user are different from the positive direction of the X axis, the positive direction of the Y axis, and the positive direction of the Z axis when the wearable device 100 is worn on the left-hand wrist of the user.

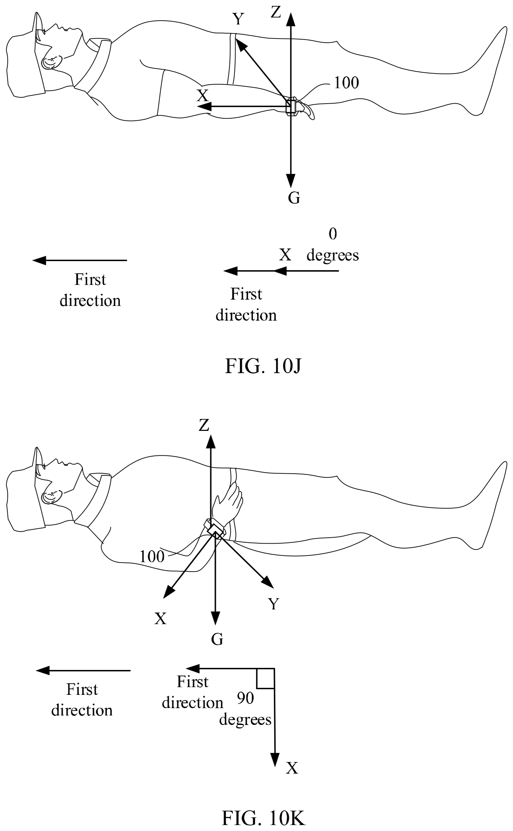

FIG. 10J to FIG. 10L are another group of diagrams in which a wrist wearing a wearable device 100 is located at different placement locations when a user is in a lying posture.

FIG. 10J is a diagram of horizontally placing an arm on a bed when a user is in a lying posture.

As shown in FIG. 10J, when the user is in the lying posture and the arm is horizontally placed, the positive direction of the X axis is opposite to the first direction, and the included angle between the positive direction of the X axis and the first direction is close to 0 degrees.

FIG. 10K is a diagram in which a wrist wearing a wearable device 100 is placed on an abdomen when a user is in a lying posture.

As shown in FIG. 10K, when the wrist wearing the wearable device 100 is placed on the abdomen when the user is in the lying posture, the included angle between the positive direction of the X axis and the first direction is close to 90 degrees.

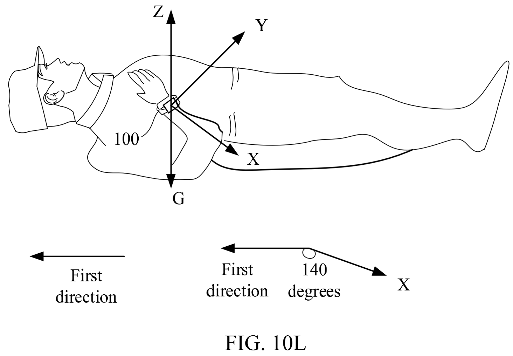

FIG. 10L is a diagram in which a wrist wearing a wearable device 100 is placed at a location of a heart when a user is in a lying posture.

As shown in FIG. 10L, when the wrist wearing the wearable device 100 is placed at the location of the heart when the user is in the lying posture, the included angle between the positive direction of the X axis and the first direction is close to 140 degrees.

**Table 12**

| Included angle between a positive direction of an X axis and a first direction | Blood pressure compensation value |
|---|---|
| 180 degrees to 140 degrees | Q |
| 139 degrees to 90 degrees | R |
| 89 degrees to 40 degrees | S |
| 39 degrees to 0 degrees | T |

Table 12 shows the blood pressure compensation value corresponding to the angle between the positive direction of the X axis and the first direction. As shown in Table 12, when the included angle between the positive direction of the X axis and the first direction is between 180 degrees and 140 degrees, the blood pressure compensation value is Q. When the included angle between the positive direction of the X axis and the first direction is between 139 degrees and 90 degrees, the blood pressure compensation value is R. When the included angle between the positive direction of the X axis and the first direction is between 89 degrees and 40 degrees, the blood pressure compensation value is S. When the included angle between the positive direction of the X axis and the first direction is between 39 degrees and 0 degrees, the blood pressure compensation value is T.

The blood pressure compensation value Q, the blood pressure compensation value R, the blood pressure compensation value S, and the blood pressure compensation value T are different from each other.

For example, when the wearable device 100 determines that the included angle between the positive direction of the X axis and the first direction is 160 degrees, the wearable device 100 may determine that the blood pressure compensation value is Q.

For another example, when the wearable device 100 determines that the included angle between the positive direction of the X axis and the first direction is 40 degrees, the wearable device 100 may determine that the blood pressure compensation value is S.

Table 12 merely describes the blood pressure compensation value corresponding to the angle between the positive direction of the X axis and the first direction as an example. Alternatively, each angle may correspond to one blood pressure compensation value. This is not limited in this application either.

In another possible implementation, the wearable device 100 may send the first angle between the positive direction of the X axis and the first direction in the lying posture of the user to the electronic device 200 that establishes a communication connection to the wearable device 100. The electronic device 200 locally stores blood pressure compensation values corresponding to a plurality of angles between the positive direction of the X axis and the first direction in the lying posture of the user. The electronic device 200 may locally determine the blood pressure compensation value based on the first angle between the positive direction of the X axis and the first direction in the lying posture of the user. Then, the electronic device 200 sends the determined blood pressure compensation value to the wearable device 100.

In another possible implementation, the wearable device 100 may send the first angle between the positive direction of the X axis and the first direction in the lying posture of the user to the server. The server locally stores the blood pressure compensation values corresponding to the plurality of angles between the positive direction of the X axis and the first direction in the lying posture of the user. The server may locally determine the blood pressure compensation value based on the first angle between the positive direction of the X axis and the first direction in the lying posture of the user. Then, the server sends the determined blood pressure compensation value to the wearable device 100.

In another embodiment, the wearable device 100 may determine a distance between the wearable device 100 and the heart in a vertical direction based on the included angle between the X axis of the wearable device 100 and the first direction, and then determine the blood pressure compensation value based on the distance between the wearable device 100 and the heart in the vertical direction.

In a possible implementation, the distance between the wearable device 100 and the heart in the vertical direction may be determined by the wearable device 100 based on the included angle between the positive direction of the X axis of the wearable device 100 and the first direction; or may be determined, by the electronic device 2000 that establishes a communication connection to the wearable device 100, based on the included angle between the positive direction of the X axis of the wearable device 100 and the first direction; may also be determined by the server based on the included angle between the positive direction of the X axis of the wearable device 100 and the first direction.

**Table 13**

| Included angle between a positive direction of an X axis and a first direction | Distance between a wearable device 100 and a heart in a vertical direction | Blood pressure compensation value |
|---|---|---|
| 180 degrees to 140 degrees | q | Q |
| 139 degrees to 90 degrees | r | R |
| 89 degrees to 40 degrees | s | S |
| 39 degrees to 0 degrees | t | T |

Table 13 shows the blood pressure compensation value corresponding to the angle between the positive direction of the X axis and the first direction. As shown in Table 13, when the included angle between the positive direction of the X axis and the first direction is between 180 degrees and 140 degrees, the distance between the wearable device 100 and the heart in the vertical direction is q, and the blood pressure compensation value is Q. When the included angle between the positive direction of the X axis and the first direction is between 139 degrees and 90 degrees, the distance between the wearable device 100 and the heart in the vertical direction is r, and the blood pressure compensation value is R. When the included angle between the positive direction of the X axis and the first direction is between 89 degrees and 40 degrees, the distance between the wearable device 100 and the heart in the vertical direction is s, and the blood pressure compensation value is S. When the included angle between the positive direction of the X axis and the first direction is between 39 degrees and 0 degrees, the distance between the wearable device 100 and the heart in the vertical direction is t, and the blood pressure compensation value is T.

The distance q, the distance r, the distance s, and the distance t are different from each other.

The blood pressure compensation value Q, the blood pressure compensation value R, the blood pressure compensation value S, and the blood pressure compensation value T are different from each other.

The blood pressure compensation value corresponding to the distance between the wearable device 100 and the heart in the vertical direction in Table 13 may be stored in the wearable device 100, or may be stored in the electronic device 200, or may be stored in the server. This is not limited in this application.

In the method, the wearable device 100 may identify the lying posture of the user, determine the blood pressure compensation value based on the lying posture of the user, and correct, based on the blood pressure compensation value, the blood pressure measurement value collected by the wearable device 100.

In some embodiments, after identifying the lying posture of the user, the wearable device 100 may determine the blood pressure compensation value based on the angle between the positive direction of the X axis of the wearable device 100 and the first direction. Because the wearable device 100 is worn at different locations, angles between the positive direction of the X axis and the first direction are different, and blood pressure compensation values are also different. In this way, this can further improve accuracy of the blood pressure measurement value collected by the wearable device 100 in the lying posture of the user.

In some embodiments, the wearable device 100 may further identify whether the wearable device 100 is worn on a left hand or a right hand, and then determine the blood pressure compensation value based on the angle between the positive direction of the X axis of the wearable device 100 and the first direction. Because the wearable device 100 is worn on the left hand or the right hand, when the wearable device 100 is located at a same location, angles between the positive direction of the X axis and the first direction are different. Therefore, whether the wearable device 100 is worn on the left hand or on the right hand needs to be distinguished. After determining whether the wearable device 100 is worn on the left hand or the right hand, the wearable device 100 determines the blood pressure compensation value based on the angle between the positive direction of the X axis of the wearable device 100 and the first direction. In this way, this can further improve accuracy of the blood pressure measurement value collected by the wearable device 100 in the lying posture of the user.

In some embodiments, the wearable device 100 may further identify the placement posture of the center of the palm. The placement posture of the center of the palm includes but is not limited to the following: the center of the palm faces upward, the center of the palm faces downward, the center of the palm is placed sideward, and the like. The wearable device 100 determines the blood pressure compensation value based on the placement posture of the center of the palm. In this way, this can further improve accuracy of the blood pressure measurement value collected by the wearable device 100 in the lying posture of the user.

### III. Determine the blood pressure compensation value based on the user posture, and obtain the blood pressure monitoring value based on the blood pressure compensation value and the blood pressure measurement value.

After determining the blood pressure compensation value, the wearable device 100 may obtain the blood pressure monitoring value based on the blood pressure compensation value and the obtained blood pressure measurement value. The blood pressure monitoring value is a finally obtained blood pressure value of the user.

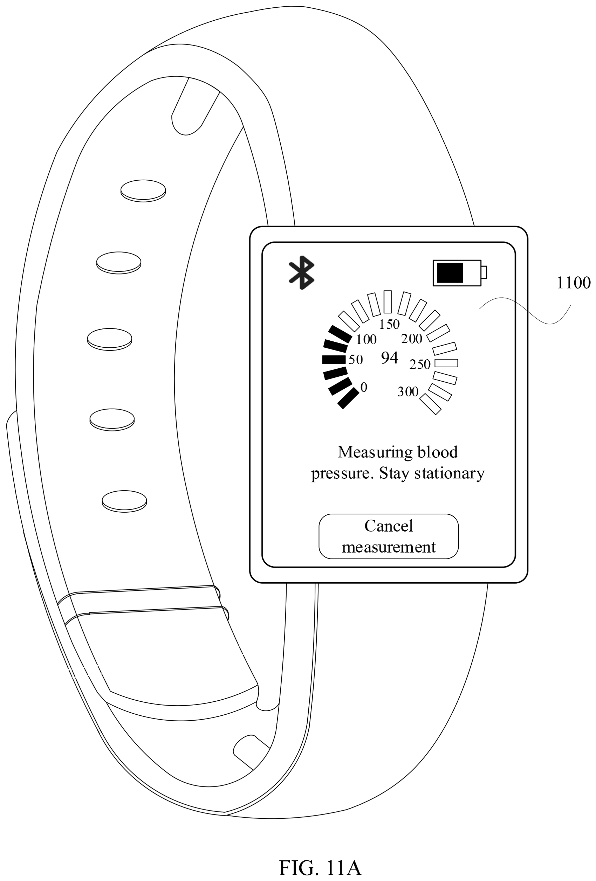

FIG. 11A to FIG. 11C are a group of diagrams in which a wearable device 100 displays a blood pressure measurement result.

Optionally, in some embodiments, before displaying the blood pressure measurement value obtained by the wearable device 100, the wearable device 100 may display a user interface 1100 shown in FIG. 11A. The user interface 1100 includes prompt information "Measuring blood pressure. Stay stationary", to prompt the user to remain stationary during blood pressure measurement, to avoid an inaccurate blood pressure measurement result caused by motion. The user interface 1100 further includes a "Cancel measurement" option. The user may stop the blood pressure measurement by using the "Cancel measurement" option.

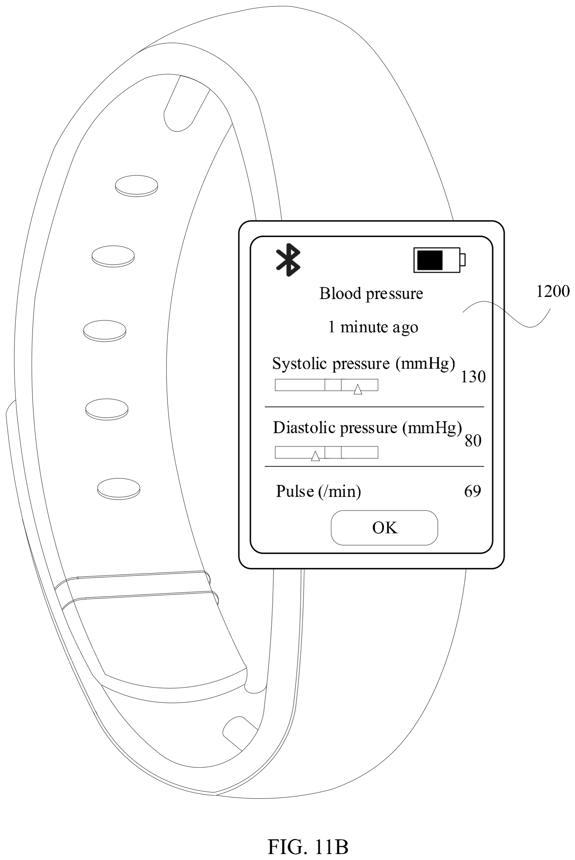

In some embodiments, after the wearable device 100 obtains the blood pressure monitoring value, the wearable device 100 may display a user interface 1200 shown in FIG. 11B. The user interface 1200 includes the blood pressure measurement value. The blood pressure measurement value may include systolic pressure and diastolic pressure. For example, the systolic pressure may be 130 mmHg, and the diastolic pressure may be 80 mmHg. In some embodiments, the user interface 1200 may further include a pulse. For example, the pulse may be 69 times per minute.

In another embodiment, after the wearable device 100 obtains the blood pressure monitoring value, the wearable device 100 may send the blood pressure monitoring value to the electronic device 200, and the electronic device 200 displays the blood pressure monitoring value.

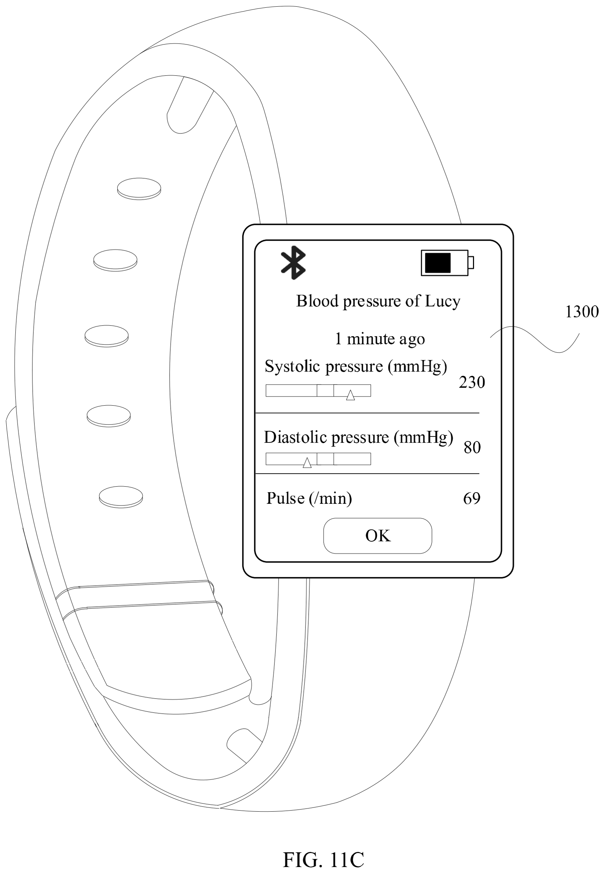

In some embodiments, after the wearable device 100 obtains the blood pressure monitoring value, the wearable device 100 may display a user interface 1300 shown in FIG. 11C. The user interface 1300 is similar to the user interface 1200, and a difference lies in that the user interface 1300 includes a nickname of a detected person, for example, "Blood pressure of Lucy", to notify the user of a user associated with a current blood pressure monitoring value. The wearable device 100 may further store blood pressure monitoring values of different users separately, to view blood pressure monitoring values of a same user in a specific time period.

In some embodiments, the wearable device 100 may store the blood pressure monitoring value within first duration before a current time point. A blood pressure monitoring value at a time point that is the first duration ago is deleted, to save storage space of the wearable device 100.

In some embodiments, the wearable device 100 may alternatively store blood pressure monitoring values with a preset quantity of monitoring times, to save storage space of the wearable device 100. For example, the wearable device 100 may store 10 blood pressure monitoring values. When monitoring is performed at an eleventh time, the wearable device 100 may delete the first blood pressure monitoring value.

Optionally, the wearable device 100 may alternatively receive a user operation and view a blood pressure monitoring value in a specific time period.

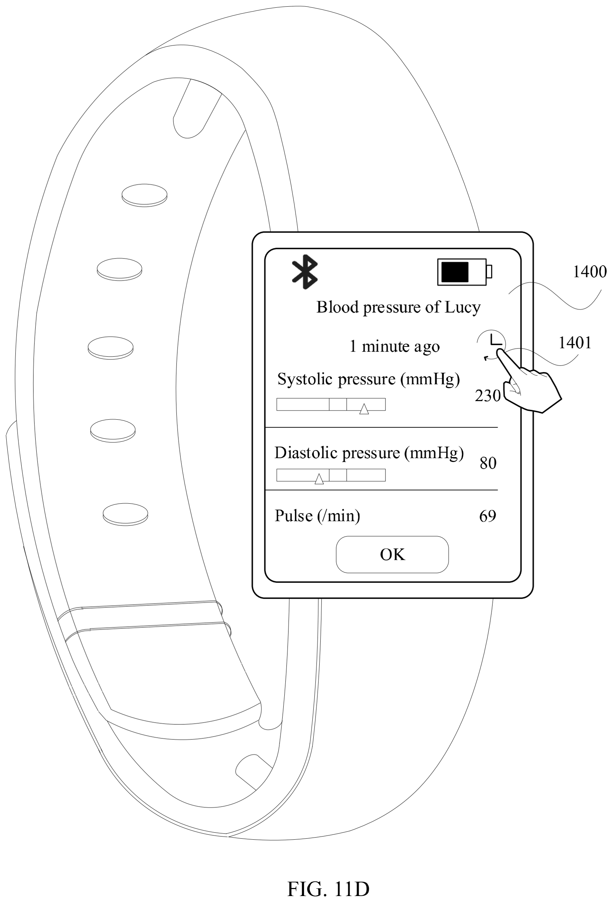

FIG. 11D to FIG. 11G are another group of diagrams in which a wearable device 100 displays blood pressure monitoring values in a specific time period.

For example, the specific time period may be 24 hours.

As shown in FIG. 11D, after the wearable device 100 obtains the blood pressure monitoring value, the wearable device 100 may display a user interface 1400 shown in FIG. 11D. The user interface 1400 is similar to the user interface 1300. A difference lies in that the user interface 1400 further includes an icon 1401, and the icon 1401 is configured to display a blood pressure monitoring value in a specific time period.

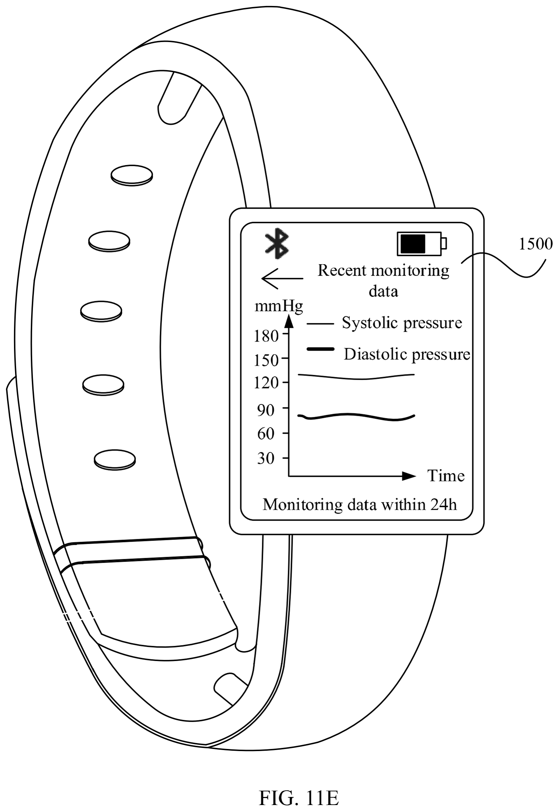

As shown in FIG. 11D, the wearable device 100 may receive an input operation (for example, a tap) performed by the user on the icon 1401 in the user interface 1400, and in response to the input operation performed by the user, the wearable device 100 may obtain a blood pressure monitoring value of the user in a specific time period, and display a user interface 1500 shown in FIG. 11E.

The user interface 1500 includes a chart display area of a blood pressure monitoring value within 24 hours. The chart display area includes a systolic pressure monitoring value curve within 24 hours and a diastolic pressure monitoring value curve within 24 hours. A blood pressure change trend of the user within 24 hours can be intuitively viewed in the chart display area.

In addition to 24 hours, the wearable device 100 may further display a blood pressure monitoring value in another longer or shorter time period. This is not limited in this application either.

In some embodiments, the wearable device 100 may also receive a user operation to view pulmonary function monitoring data of different users.

With reference to the descriptions in FIG. 11D, after the wearable device 100 obtains the blood pressure monitoring value, the wearable device 100 may display the user interface 1400 shown in FIG. 11D.

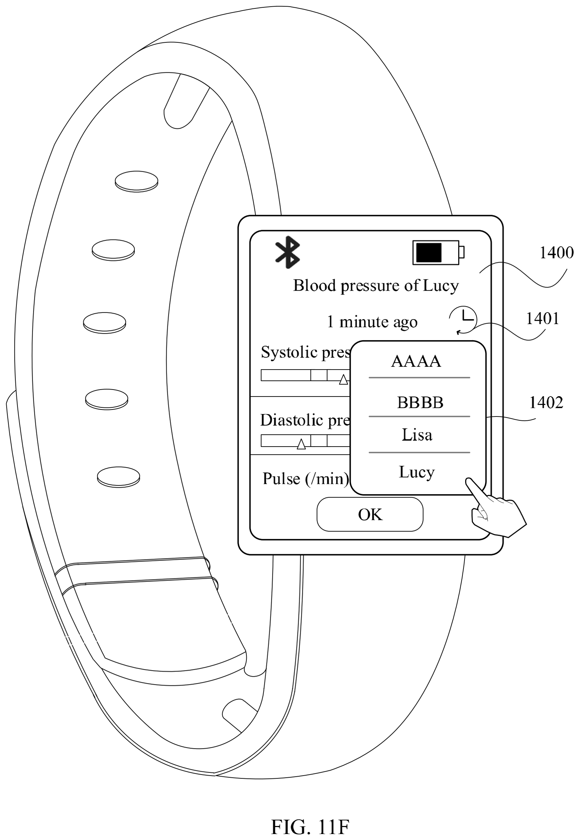

The wearable device 100 may receive an input operation performed by the user on the icon 1401 in the user interface 1400, and in response to the input operation performed by the user, the wearable device 100 may display a selection bar 1402 shown in FIG. 11F. Options of a plurality of different monitoring objects are shown in the selection bar 1402. For example, the plurality of monitoring objects include but are not limited to a monitoring object "AAAA", a monitoring object "BBBB", a monitoring object "Lisa", and a monitoring object "Lucy". The user may select any monitoring object and start to view a blood pressure monitoring value of the monitoring object in a specific time period.

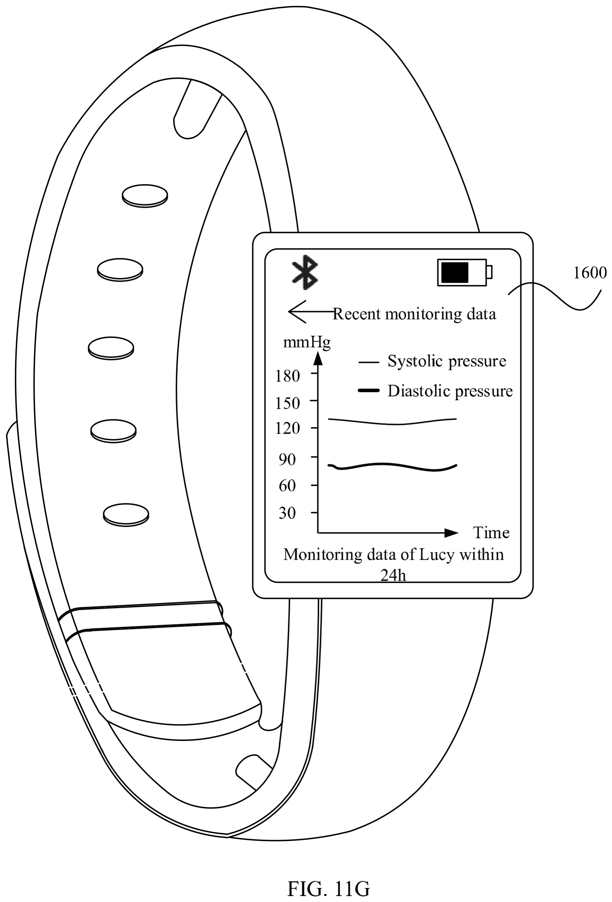

As shown in FIG. 11F, the wearable device 100 may receive an input operation (for example, a tap) performed by the user on the monitoring object "Lucy" option in the selection bar 1402, and in response to the input operation performed by the user, the wearable device 100 may obtain a blood pressure monitoring value of the monitoring object "Lucy" in a specific time period, and display a user interface 1600 shown in FIG. 11G. The user interface 1600 is similar to the user interface 1500, and a difference lies in that the user interface 1600 includes prompt information "Monitoring data of Lucy within 24h", to prompt that the systolic pressure monitoring value curve and the diastolic pressure monitoring value curve that are shown in the user interface 1600 are blood pressure monitoring values of the monitoring object "Lucy" within 24 hours.

In the method, the wearable device 100 may not only display a blood pressure monitoring value of a local user in a specific time period, but also display a blood pressure monitoring value of another user in a specific time period.

In some embodiments, during one-day blood pressure measurement, the wearable device 100 may compare a blood pressure monitoring value obtained through measurement and a blood pressure normal value, and delete a blood pressure monitoring value that is greatly different from the blood pressure normal value, to avoid interference to blood pressure analysis of the user.

In some embodiments, blood pressure monitoring values that are of different users and that are stored in the wearable device 100 in a specific time period may be blood pressure monitoring values that are of different users and that are collected by the wearable device 100 in a specific time period, and that are sent to the wearable device 100, and that are stored in the wearable device 100.

In another embodiment, blood pressure monitoring values that are of different users and are stored in the wearable device 100 in a specific time period may be sent by another electronic device to the wearable device 100 periodically/aperiodically/at a specific time interval. After a user of another electronic device obtains authorization of the user, the another electronic device may send a stored blood pressure monitoring value of the user to the wearable device 100 periodically/aperiodically/at a specific time interval, so that the wearable device 100 may store blood pressure monitoring values of different users in a specific time period, to help a user using the wearable device 100 view blood pressure monitoring values of different users of another authorized user in a specific time period. For example, the another authorized user may be a family member of the user using the wearable device 100. For example, the another authorized user may be a parent, a child, or the like of the user using the wearable device 100. In this case, after the wearable device 100 obtains a blood pressure monitoring value of the parent or the child in a specific time period, the user using the wearable device 100 may view the blood pressure monitoring value of the another authorized user in a specific time period, to facilitate monitoring of the blood pressure of the family member.

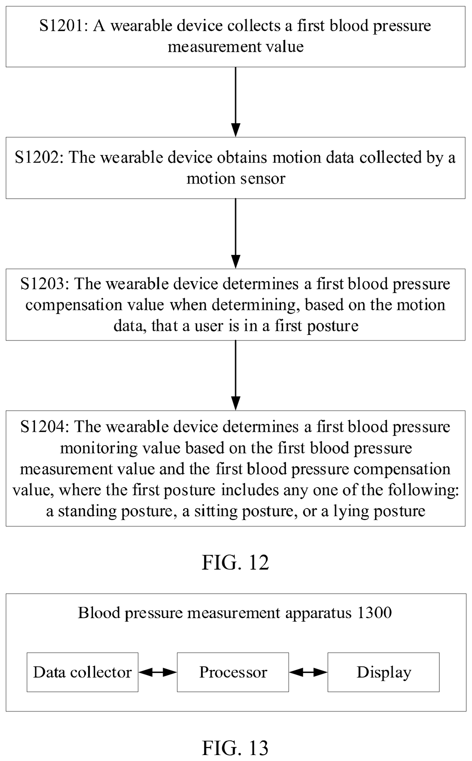

FIG. 12 is a diagram of a method procedure of a blood pressure measurement method according to this application.

S1201: A wearable device collects a first blood pressure measurement value.

S 1202: The wearable device obtains motion data collected by a motion sensor.

S 1203: The wearable device determines a first blood pressure compensation value when determining, based on the motion data, that a user is in a first posture.

S 1204: The wearable device determines a first blood pressure monitoring value based on the first blood pressure measurement value and the first blood pressure compensation value, where the first posture includes any one of the following: a standing posture, a sitting posture, or a lying posture.

In some embodiments, the motion sensor may be an acceleration sensor and/or an angular velocity sensor. The motion data may be one or more types of data such as acceleration data, /or angular velocity data, a motion step count, a heart rate, and a motion trajectory.

In the method, the wearable device may revise a blood pressure measurement result based on different user postures, thereby improving accuracy of measuring blood pressure by the wearable device.

In a possible implementation, that the wearable device determines the first blood pressure compensation value when determining, based on the motion data, that the user is in the first posture specifically includes: The wearable device determines the first blood pressure compensation value when determining, based on the motion data, that the user is in the first posture and the wearable device is worn on a left-hand wrist.

In another possible implementation, the method further includes: The wearable device determines a second blood pressure compensation value when determining, based on the motion data, that the user is in the first posture and the wearable device is worn on a right-hand wrist. The first blood pressure compensation value is different from the second blood pressure compensation value.

In some embodiments, the wearable device may determine a motion trajectory of the wearable device based on the motion data, and determine, based on the motion trajectory of the wearable device, whether the wearable device is worn on a left hand or a right hand.

In this way, when identifying a user posture, the wearable device can further identify whether the wearable device is worn on the left hand or the right hand, and determine different blood pressure compensation values based on whether the wearable device is worn on the left hand or the right hand. This can further improve accuracy of blood pressure measurement.

In a possible implementation, that the wearable device determines the first blood pressure compensation value when determining, based on the motion data, that the user is in the first posture specifically includes: The wearable device determines a first included angle between a positive direction of an X axis and a positive direction of a first direction of the wearable device, where the first included angle is greater than 0 degrees and less than 180 degrees, the first direction is parallel to gravitational acceleration G, the positive direction of the first direction is opposite to a positive direction of the gravitational acceleration G, and when the wearable device is worn on the left-hand wrist, the X axis is parallel to a forearm, and the positive direction of the X axis is a direction pointing to a user finger; and the wearable device determines the first blood pressure compensation value based on the first included angle when determining, based on the motion data, that the user is in the first posture.

When identifying the user posture, the wearable device further identifies an angle between a wrist wearing the wearable device and the positive direction of the first direction, and determines different blood pressure compensation values based on different angles. This can further improve accuracy of blood pressure measurement.

For example, when the wearable device identifies the first posture of the user, the wearable device may determine the first blood pressure compensation value when the wearable device is worn on the left-hand wrist of the user, and there is the first included angle between the positive direction of the X axis of the wearable device and the positive direction of the first direction. The wearable device may determine the second blood pressure compensation value when the wearable device is worn on the right-hand wrist of the user and there is the first included angle between the positive direction of the X axis of the wearable device and the positive direction of the first direction. The first blood pressure compensation value is different from the second blood pressure compensation value.

Specifically, when the first posture is the standing posture, refer to the descriptions in the embodiments in FIG. 8D to FIG. 8I.

When the first posture is the sitting posture, refer to the descriptions in the embodiments in FIG. 9D to FIG. 9I.

When the first posture is the lying posture, refer to the descriptions in the embodiments in FIG. 10D to FIG. 10F and FIG. 10J to FIG. 10L.

In a possible implementation, when the first posture is the lying posture, that the wearable device determines the first blood pressure compensation value when determining, based on the motion data, that the user is in the first posture specifically includes: The wearable device determines the first blood pressure compensation value when determining, based on the motion data, that the user is in the lying posture and a center of a palm corresponding to a wrist wearing the wearable device faces a ground.

In a possible implementation, the method further includes: The wearable device determines a third blood pressure compensation value when determining, based on the motion data, that the user is in the lying posture and the center of the palm corresponding to the wrist wearing the wearable device faces a sky. The third blood pressure compensation value is different from the first blood pressure compensation value.

In a possible implementation, the method further includes: The wearable device determines a fourth blood pressure compensation value when determining, based on the motion data, that the user is in the lying posture and the center of the palm corresponding to the wrist wearing the wearable device faces sideward. The fourth blood pressure compensation value is different from the first blood pressure compensation value and the third blood pressure compensation value.

In this way, when a first user posture is the lying posture, the wearable device may determine different blood pressure compensation values based on a placement posture of the palm corresponding to the wrist wearing the wearable device. This can further improve accuracy of blood pressure measurement.

For details, refer to the descriptions in the embodiments in FIG. 10I to FIG. 10J.

In a possible implementation, when the first posture is the standing posture, the wearable device determines, based on the motion data, that the user is in the standing posture, specifically including: when the motion data meets a first condition, the wearable device determines that the user is in the standing posture. The first condition includes but is not limited to any one or more of the following: a plurality of groups of heart rate values within first duration before blood pressure measurement starts are greater than a first heart rate value; a change value of an included angle between the positive direction of the X axis and the first direction within second duration before the blood pressure measurement starts is greater than a first angle value; and an acceleration component of the gravitational acceleration G on a Z axis is close to a minimum value, and the Z axis is perpendicular to a plane on which a display of the wearable device is located.

This is not limited thereto. The wearable device may further determine the standing posture based on another condition. This is not limited in this application.

For details, refer to the descriptions in the embodiment in FIG. 8A.

In a possible implementation, when the first posture is the sitting posture, the wearable device determines, based on the motion data, that the user is in the sitting posture, specifically including: when the motion data meets a second condition, the wearable device determines that the user is in the sitting posture. The second condition includes but is not limited to any one or more of the following: a plurality of groups of heart rate values within first duration before blood pressure measurement starts are greater than a second heart rate value and less than a first heart rate value, where the second heart rate value is less than the first heart rate value; a change value of an included angle between the positive direction of the X axis and the first direction within second duration before the blood pressure measurement starts is greater than a second angle value and less than a first angle value, and the second angle value is less than the first angle value; and an acceleration component of the gravitational acceleration G on a Z axis is close to a minimum value, and the Z axis is perpendicular to a plane on which a display of the wearable device is located.

This is not limited thereto. The wearable device may further determine the sitting posture based on another condition. This is not limited in this application.

For details, refer to the descriptions in the embodiment in FIG. 9A.

In a possible implementation, when the first posture is the lying posture, the wearable device determines, based on the motion data, that the user is in the lying posture, specifically including: when the motion data meets a third condition, the wearable device determines that the user is in the sitting posture. The third condition includes but is not limited to any one or more of the following: a plurality of groups of heart rate values within first duration before blood pressure measurement starts are less than a second heart rate value; a motion trajectory of the wearable device satisfies a preset motion trajectory, and the preset motion trajectory is an up-down motion trajectory in a vertical direction; and acceleration components of the gravitational acceleration G on the X axis and a Y axis are close to a minimum value, and the Y axis is perpendicular to the X axis.

This is not limited thereto. The wearable device may further determine the lying posture based on another condition. This is not limited in this application.

For details, refer to the descriptions in the embodiment in FIG. 10A.

In a possible implementation, before collecting, by the wearable device, the first blood pressure measurement value, the method further includes: The wearable device displays first prompt information when detecting that the wearable device switches from a non-worn state to a worn state, where the first prompt information is used to prompt the user to confirm whether the wearable device is worn by a local user; and the wearable device receives a first operation performed by the user on a first option in the first prompt information, and in response to the first operation, confirms that the wearable device is worn by the local user; and after determining the first blood pressure monitoring value, the method further includes: The wearable device stores the first blood pressure monitoring value in a first storage area, where the first storage area stores blood pressure measurement data of the local user.

With reference to the first aspect, in a possible implementation, the method further includes: The wearable device receives a second operation performed by the user on a second option in the first prompt information, and in response to the second operation, confirms that the wearable device is worn by a non-local user; and after determining the first blood pressure monitoring value, the method further includes: The wearable device stores the first blood pressure monitoring value in a second storage area, where the second storage area stores blood pressure measurement data of a non-local user, and the first storage area is different from the second storage area.

In this way, before starting to measure the blood pressure, the wearable device may prompt the user to choose whether the wearable device is worn by the local user. Therefore, blood pressure measurement data of different users can be prevented from being stored together, and accuracy of an analysis result of blood pressure measurement data of a single user is affected.

For details, refer to the descriptions in the embodiments in FIG. 6G to FIG. 6L.

This application provides a wearable device. The wearable device includes a motion sensor, a memory, and a processor, the motion sensor, the memory, and the processor are coupled, the memory is configured to store a computer program, and when the processor executes and invokes the computer program, the wearable device is enabled to perform the blood pressure measurement method according to the embodiment in FIG. 12.

This application provides a computer-readable storage medium, including instructions. When the instructions are run on a wearable device, the wearable device is enabled to perform the blood pressure monitoring method according to the embodiment in FIG. 12.

This application provides a chip system. The chip system includes one or more processors, and the processor is configured to invoke computer instructions, to perform the blood pressure monitoring method according to the embodiment in FIG. 12.

This application provides a computer program product including instructions. When the computer program product runs on a wearable device, the wearable device is enabled to perform the blood pressure monitoring method according to the embodiment in FIG. 12.

FIG. 13 is a diagram of a blood pressure measurement apparatus according to this application.

As shown in FIG. 13, a blood pressure measurement apparatus 1300 includes a data collector and a processor.

The data collector is configured to collect a first blood pressure measurement value.

The data collector is further configured to obtain motion data collected by a motion sensor.

The processor is configured to determine, by a wearable device, a first blood pressure compensation value when determining, based on the motion data, that a user is in a first posture.

The processor is further configured to determine a first blood pressure monitoring value based on the first blood pressure measurement value and the first blood pressure compensation value, where the first posture includes any one of the following: a standing posture, a sitting posture, or a lying posture.

In some embodiments, the motion sensor may be an acceleration sensor and/or an angular velocity sensor. The motion data may be one or more types of data such as acceleration data, /or angular velocity data, a motion step count, a heart rate, and a motion trajectory.

According to the method provided in the first aspect, the wearable device may revise a blood pressure measurement result based on different user postures, thereby improving accuracy of measuring blood pressure by the wearable device.

In a possible implementation, the processor is specifically configured to determine, by the wearable device, the first blood pressure compensation value when determining, based on the motion data, that the user is in the first posture and the wearable device is worn on a left-hand wrist.

The processor is further configured to determine, by the wearable device, a second blood pressure compensation value when determining, based on the motion data, that the user is in the first posture and the wearable device is worn on a right-hand wrist. The first blood pressure compensation value is different from the second blood pressure compensation value.

In some embodiments, the wearable device may determine a motion trajectory of the wearable device based on the motion data, and determine, based on the motion trajectory of the wearable device, whether the wearable device is worn on a left hand or a right hand.

In this way, when identifying a user posture, the wearable device can further identify whether the wearable device is worn on the left hand or the right hand, and determine different blood pressure compensation values based on whether the wearable device is worn on the left hand or the right hand. This can further improve accuracy of blood pressure measurement.

In a possible implementation, the processor is specifically configured to: determine a first included angle between a positive direction of an X axis and a positive direction of a first direction of the wearable device, where the first included angle is greater than 0 degrees and less than 180 degrees, the first direction is parallel to gravitational acceleration G, the positive direction of the first direction is opposite to a positive direction of the gravitational acceleration G, and when the wearable device is worn on the left-hand wrist, the X axis is parallel to a forearm, and the positive direction of the X axis is a direction pointing to a user finger; and determine the first blood pressure compensation value based on the first included angle when determining, based on the motion data, that the user is in the first posture.

When identifying the user posture, the wearable device further identifies an angle between a wrist wearing the wearable device and the positive direction of the first direction, and determines different blood pressure compensation values based on different angles. This can further improve accuracy of blood pressure measurement.

In a possible implementation, when the first posture is the lying posture, the processor is specifically configured to determine the first blood pressure compensation value when determining, based on the motion data, that the user is in the lying posture and a center of a palm corresponding to a wrist wearing the wearable device faces a ground.

In a possible implementation, the processor is further configured to determine a third blood pressure compensation value when determining, based on the motion data, that the user is in the lying posture and the center of the palm corresponding to the wrist wearing the wearable device faces a sky. The third blood pressure compensation value is different from the first blood pressure compensation value.

In a possible implementation, the processor is further configured to determine a fourth blood pressure compensation value when determining, based on the motion data, that the user is in the lying posture and the center of the palm corresponding to the wrist wearing the wearable device faces sideward. The fourth blood pressure compensation value is different from the first blood pressure compensation value and the third blood pressure compensation value.

In this way, when a first user posture is the lying posture, the wearable device may determine different blood pressure compensation values based on a placement posture of the palm corresponding to the wrist wearing the wearable device. This can further improve accuracy of blood pressure measurement.

In a possible implementation, when the first posture is the standing posture, the processor is specifically configured to: when the motion data meets a first condition, determines that the user is in the standing posture. The first condition includes but is not limited to any one or more of the following: a plurality of groups of heart rate values within first duration before blood pressure measurement starts are greater than a first heart rate value; a change value of an included angle between the positive direction of the X axis and the first direction within second duration before the blood pressure measurement starts is greater than a first angle value; and an acceleration component of the gravitational acceleration G on a Z axis is close to a minimum value, and the Z axis is perpendicular to a plane on which a display of the wearable device is located.

This is not limited thereto. The wearable device may further determine the standing posture based on another condition. This is not limited in this application.

In a possible implementation, when the first posture is the sitting posture, the processor is specifically configured to: when the motion data meets a second condition, determine that the user is in the sitting posture. The second condition includes but is not limited to any one or more of the following: a plurality of groups of heart rate values within first duration before blood pressure measurement starts are greater than a second heart rate value and less than a first heart rate value, where the second heart rate value is less than the first heart rate value; a change value of an included angle between the positive direction of the X axis and the first direction within second duration before the blood pressure measurement starts is greater than a second angle value and less than a first angle value, and the second angle value is less than the first angle value; and an acceleration component of the gravitational acceleration G on a Z axis is close to a minimum value, and the Z axis is perpendicular to a plane on which a display of the wearable device is located.

This is not limited thereto. The wearable device may further determine the sitting posture based on another condition. This is not limited in this application.

In a possible implementation, when the first posture is the lying posture, the processor is specifically configured to: when the motion data meets a third condition, determine that the user is in the sitting posture. The third condition includes but is not limited to any one or more of the following: a plurality of groups of heart rate values within first duration before blood pressure measurement starts are less than a second heart rate value; a motion trajectory of the wearable device satisfies a preset motion trajectory, and the preset motion trajectory is an up-down motion trajectory in a vertical direction; and acceleration components of the gravitational acceleration G on the X axis and a Y axis are close to a minimum value, and the Y axis is perpendicular to the X axis.

This is not limited thereto. The wearable device may further determine the lying posture based on another condition. This is not limited in this application.

In a possible implementation, the blood pressure measurement apparatus 1300 further includes a display. The display is configured to display first prompt information when detecting that the wearable device switches from a non-worn state to a worn state, where the first prompt information is used to prompt the user to confirm whether the wearable device is worn by a local user; and the display is further configured to: receive a first operation performed by the user on a first option in the first prompt information, and in response to the first operation, confirm that the wearable device is worn by the local user; and after determining the first blood pressure monitoring value, the processor is further configured to store the first blood pressure monitoring value in a first storage area, where the first storage area stores blood pressure measurement data of the local user.

In a possible implementation, the display is further configured to: receive a second operation performed by the user on a second option in the first prompt information, and in response to the second operation, confirm that the wearable device is worn by a non-local user; and after determining the first blood pressure monitoring value, the processor is further configured to store the first blood pressure monitoring value in a second storage area, where the second storage area stores blood pressure measurement data of a non-local user, and the first storage area is different from the second storage area.

In this way, before starting to measure the blood pressure, the wearable device may prompt the user to choose whether the wearable device is worn by the local user. Therefore, blood pressure measurement data of different users can be prevented from being stored together, and accuracy of an analysis result of blood pressure measurement data of a single user is affected.

The foregoing descriptions are merely some embodiments and implementations of this application, but are not intended to limit the protection scope of this application. Any variation or replacement readily figured out by a person skilled in the art within the technical scope disclosed in this application shall fall within the protection scope of this application. Therefore, the protection scope of this application shall be subject to the protection scope of the claims.

It may be understood that each user interface described in embodiments of this application is merely an example interface, and constitutes no limitation on the solutions of this application. In another embodiment, the user interface may use different interface layouts, may include more or fewer controls, and may add or reduce other function options, and provided that the user interface is based on a same inventive idea provided in this application, all fall within the protection scope of this application.

It should be noted that, if no contradiction or conflict occurs, any feature or any part of any feature in any embodiment of this application may be combined, and a combined technical solution also falls within the scope of embodiments of this application.

In conclusion, the foregoing embodiments are merely intended for describing the technical solutions of this application, but not for limiting this application. Although this application is described in detail with reference to the foregoing embodiments, persons of ordinary skill in the art should understand that they may still make modifications to the technical solutions described in the foregoing embodiments or make equivalent replacements to some technical features thereof, without departing from the scope of the technical solutions of embodiments of this application.

## Claims

1. A blood pressure monitoring method, wherein the method is applied to a wearable device, the wearable device comprises a motion sensor, and the method comprises:
collecting, by the wearable device, a first blood pressure measurement value;
obtaining, by the wearable device, motion data collected by the motion sensor;
determining, by the wearable device, a first blood pressure compensation value when determining, based on the motion data, that a user is in a first posture; and
determining, by the wearable device, a first blood pressure monitoring value based on the first blood pressure measurement value and the first blood pressure compensation value, wherein
the first posture comprises any one of the following: a standing posture, a sitting posture, or a lying posture.

2. The method according to claim 1, wherein determining, by the wearable device, the first blood pressure compensation value when determining, based on the motion data, that the user is in the first posture specifically comprises:
determining, by the wearable device, the first blood pressure compensation value when determining, based on the motion data, that the user is in the first posture and the wearable device is worn on a left-hand wrist.

3. The method according to claim 2, wherein the method further comprises:
determining, by the wearable device, a second blood pressure compensation value when determining, based on the motion data, that the user is in the first posture and the wearable device is worn on a right-hand wrist, wherein the first blood pressure compensation value is different from the second blood pressure compensation value.

4. The method according to any one of claims 1 to 3, wherein determining, by the wearable device, the first blood pressure compensation value when determining, based on the motion data, that the user is in the first posture specifically comprises:
determining, by the wearable device, a first included angle between a positive direction of an X axis and a positive direction of a first direction of the wearable device, wherein the first included angle is greater than 0 degrees and less than 180 degrees, the first direction is parallel to gravitational acceleration G, the positive direction of the first direction is opposite to a positive direction of the gravitational acceleration G, and when the wearable device is worn on the left-hand wrist, the X axis is parallel to a forearm, and the positive direction of the X axis is a direction pointing to a user finger; and
determining, by the wearable device, the first blood pressure compensation value based on the first included angle when determining, based on the motion data, that the user is in the first posture.

5. The method according to any one of claims 1 to 4, wherein when the first posture is the lying posture, determining, by the wearable device, the first blood pressure compensation value when determining, based on the motion data, that the user is in the first posture specifically comprises:
determining, by the wearable device, the first blood pressure compensation value when determining, based on the motion data, that the user is in the lying posture and a center of a palm corresponding to a wrist wearing the wearable device faces a ground.

6. The method according to claim 5, wherein the method further comprises:
determining, by the wearable device, a third blood pressure compensation value when determining, based on the motion data, that the user is in the lying posture and the center of the palm corresponding to the wrist wearing the wearable device faces a sky, wherein the third blood pressure compensation value is different from the first blood pressure compensation value.

7. The method according to claim 5 or 6, wherein the method further comprises:
determining, by the wearable device, a fourth blood pressure compensation value when determining, based on the motion data, that the user is in the lying posture and the center of the palm corresponding to the wrist wearing the wearable device faces sideward, wherein the fourth blood pressure compensation value is different from the first blood pressure compensation value and the third blood pressure compensation value.

8. The method according to any one of claims 1 to 7, wherein when the first posture is the standing posture, the wearable device determines, based on the motion data, that the user is in the standing posture, specifically comprising:
when the motion data meets a first condition, determining, by the wearable device, that the user is in the standing posture, wherein
the first condition comprises but is not limited to any one or more of the following:
a plurality of groups of heart rate values within first duration before blood pressure measurement starts are greater than a first heart rate value;
a change value of an included angle between the positive direction of the X axis and the first direction within second duration before the blood pressure measurement starts is greater than a first angle value; and
an acceleration component of the gravitational acceleration G on a Z axis is close to a minimum value, and the Z axis is perpendicular to a plane on which a display of the wearable device is located.

9. The method according to any one of claims 1 to 7, wherein when the first posture is the sitting posture, the wearable device determines, based on the motion data, that the user is in the sitting posture, specifically comprising:
when the motion data meets a second condition, determining, by the wearable device, that the user is in the sitting posture, wherein
the second condition comprises but is not limited to any one or more of the following:
a plurality of groups of heart rate values within first duration before blood pressure measurement starts are greater than a second heart rate value and less than a first heart rate value, and the second heart rate value is less than the first heart rate value;
a change value of an included angle between the positive direction of the X axis and the first direction within second duration before the blood pressure measurement starts is greater than a second angle value and less than a first angle value, wherein the second angle value is less than the first angle value; and
an acceleration component of the gravitational acceleration G on a Z axis is close to a minimum value, and the Z axis is perpendicular to a plane on which a display of the wearable device is located.

10. The method according to any one of claims 1 to 7, wherein when the first posture is the lying posture, the wearable device determines, based on the motion data, that the user is in the lying posture, specifically comprising:
when the motion data meets a third condition, determining, by the wearable device, that the user is in the sitting posture, wherein
the third condition comprises but is not limited to any one or more of the following:
a plurality of groups of heart rate values within first duration before blood pressure measurement starts are less than a second heart rate value;
a motion trajectory of the wearable device satisfies a preset motion trajectory, and the preset motion trajectory is an up-down motion trajectory in a vertical direction; and
acceleration components of the gravitational acceleration G on the X axis and a Y axis are close to a minimum value, and the Y axis is perpendicular to the X axis.

11. The method according to any one of claims 1 to 10, wherein before collecting, by the wearable device, the first blood pressure measurement value, the method further comprises:
displaying, by the wearable device, first prompt information when detecting that the wearable device switches from a non-worn state to a worn state, wherein the first prompt information is used to prompt the user to confirm whether the wearable device is worn by a local user; and
receiving, by the wearable device, a first operation performed by the user on a first option in the first prompt information, and in response to the first operation, confirming that the wearable device is worn by the local user; and
after determining the first blood pressure monitoring value, the method further comprises:
storing, by the wearable device, the first blood pressure monitoring value in a first storage area, wherein the first storage area stores blood pressure measurement data of the local user.

12. The method according to claim 11, wherein the method further comprises:
receiving, by the wearable device, a second operation performed by the user on a second option in the first prompt information, and in response to the second operation, confirming that the wearable device is worn by a non-local user; and
after determining the first blood pressure monitoring value, the method further comprises:
storing, by the wearable device, the first blood pressure monitoring value in a second storage area, wherein the second storage area stores blood pressure measurement data of the non-local user, and the first storage area is different from the second storage area.

13. A wearable device, wherein the wearable device comprises a motion sensor, a memory, and a processor, the motion sensor, the memory, and the processor are coupled, the memory is configured to store a computer program, and when the processor invokes the computer program, the wearable device is enabled to perform the method according to any one of claims 1 to 12.

14. A computer-readable storage medium, comprising instructions, wherein when the instructions are run on a wearable device, the wearable device is enabled to perform the method according to any one of claims 1 to 12.
